# EUROPEAN PATENT APPLICATION

(11) **EP 4 596 584 A1**
(43) Date of publication of application: **06.08.2025**
(21) Application number: 23870705.3
(22) Date of filing: 25.09.2023
(51) Int. Cl.: C07K 16/46, A61K 39/395, C07K 16/18

(54) **TRISPECIFIC ANTIBODY TARGETING PD-1, CTLA-4, AND VEGF AND USE THEREOF**

(30) Priority: 29.09.2022 CN 202211204407
(71) Applicant: Shanghai Hongcheng Biopharmaceutical Co., Ltd., Shanghai 201203 (CN)
(72) Inventor: SU, Yunpeng, Shanghai 201203 (CN); LI, Yong, Shanghai 201203 (CN); YIN, Qiaoshan, Shanghai 201203 (CN); ZHUANG, Weiliang, Shanghai 201203 (CN); SONG, Fei, Shanghai 201203 (CN); SUI, Ruirui, Shanghai 201203 (CN); WU, Feng, Shanghai 201203 (CN); JIANG, Diandong, Shanghai 201203 (CN); XI, Yue, Shanghai 201203 (CN); XU, Jinling, Shanghai 201203 (CN); YANG, Linchuan, Shanghai 201203 (CN); LIANG, Shaoqin, Shanghai 201203 (CN)
(74) Representative: Latscha Schöllhorn Partner AG
(86) International application number: PCT/CN2023/121074
(87) International publication number: WO 2024/067474

(57) **Abstract**

The present invention relates to a trispecific antibody that specifically binds to PD-1, CTLA-4 and VEGF. The present invention also relates to a composition comprising the trispecific antibody and its use in the treatment of tumors, autoimmune diseases, infectious diseases or angiogenesis-related diseases.

## Description

### FIELD OF THE INVENTION

The present invention belongs to the field of biomedicine, and specifically relates to trispecific antibodies targeting PD-1, CTLA-4 and VEGF and their use for the treatment of tumor.

### BACKGROUND OF THE INVENTION

In clinical studies, it has been found that many patients cannot respond to a mono-specific therapy adequately due to the complexity of the disease, although immunotherapy has significantly enhanced the ability to treat diseases (such as cancer). Tumor microenvironment is composed of cells, such as tumor cells, immune cells and vascular cells, and non-cellular components in the extracellular matrix. The interactions between the components limit the host's anti-tumor immunity and control of tumor growth. Researchers hope to achieve more favorable therapeutic effects by developing combination therapies targeting different cells and different targets. For example, the use of Nivolumab, an antibody that blocks the immune checkpoint PD-1, in combination with Ipilimumab, an antibody that blocks CTLA-4, can stimulate a stronger immune response in preclinical and clinical trials, resulting in an improved therapeutic effect relative to blocking a single immune checkpoint (The efficacy and safety of combined immune checkpoint inhibitors (nivolumab plus ipilimumab): a systematic review and meta-analysis, Jingjie Chen etal., World J Surg Oncol, 2020 Jul 3;18(1):150). However, the above combination therapy also increases the cost of medication, enhances multiple side effects, and has higher toxicity, which may be related to the clearance of regulatory T cells (Treg) by Ipilimumab (Safety profiles of anti-CTLA-4 and anti-PD-1 antibodies alone and in combination,Celine Boutros et.al., Nat Rev Clin Oncol, 2016 Aug;13(8):473-86).

Immune checkpoints PD-1 and CTLA-4 have different expression patterns on different immune cells, and the signal interaction mediated by the two inhibits anti-tumor immunity. Therefore, simultaneous blocking of PD-1 and CTLA-4 can promote anti-tumor immunity through multiple mechanisms (PD-L1:CD80 Cis-Heterodimer Triggers the Co-stimulatory Receptor CD28 While Repressing the Inhibitory PD-1 and CTLA-4 Pathways, Yunlong Zhao et.al., Immunity. 2019 Dec 17; 51(6): 1059-1073.e9). Bispecific antibodies targeting both PD-1 and CTLA-4 can not only block the signaling pathways of PD-1 and CTLA-4 at the same time, but also induce endocytosis of PD-1, leading to its degradation. On cells co-expressing PD-1 and CTLA-4 (such as exhausted T cells in the tumor microenvironment), they can enhance anti-tumor immunity by blocking the interaction between CTLA-4 and CD80, and clinically demonstrate efficacy in patients who are refractory to immune checkpoint inhibitors (Development and Preliminary Clinical Activity of PD-1-Guided CTLA-4 Blocking Bispecific DART Molecule, Alexey Berezhnoy et al., Cell Rep Med. 2020 Dec 22; 1(9): 100163, Design and Efficacy of a Monovalent Bispecific PD-1/CTLA4 Antibody That Enhances CTLA-4 Blockade on PD-1+ Activated T Cells, Dovedi SJ et al., Cancer Discovery, 08 Jan 2021, 11(5): 1100-1117).

Angiogenic factors can drive immunosuppression by directly inhibiting the function of antigen-presenting cells and immune effector cells and by enhancing the function of immunosuppressive cells such as regulatory T cells (Tregs), while immunosuppressive cells promote angiogenesis by secreting cytokines, etc. The immune response and angiogenesis influence each other and both play an important role in the occurrence and development of tumors. Studies have found that immune checkpoint inhibitors can promote the normalization of blood vessels in tumor tissues (The Intersection between Tumor Angiogenesis and Immune Suppression, Rahma OE et al., Clin Cancer Res (2019) 25 (18): 5449-5457), antiangiogenesis can also improve antitumor immunity in the tumor microenvironment (Antiangiogenic therapy reverses the immunosuppressive breast cancer microenvironment, Wuzhen Chen et al., Biomark Res. 2021 Jul 22;9(1):59). The combination of anti-PD-L1 antibody atezolizumab and bevacizumab has shown significant efficacy in unresectable liver cancer (Atezolizumab plus Bevacizumab in Unresectable Hepatocellular Carcinoma, Richard S Finn et al., N Engl J Med., 2020 May 14;382(20):1894-1905), and approved by the FDA for the treatment of unresectable liver cancer. The combined use of other immune checkpoint inhibitors (including anti-PD-1/PD-L1 antibodies) and anti-angiogenic inhibitors (including anti-VEGF antibodies and other small molecule inhibitors that block VEGF signals) has also achieved positive results in clinical practice (Combination of Anti-Angiogenics and Checkpoint Inhibitors for Renal Cell Carcinoma: Is the Whole Greater Than the Sum of Its Parts? Eric Jonasch et al., Cancers (Basel). 2022 Jan 27;14(3):644). Bispecific antibodies that simultaneously block both immune checkpoints and angiogenesis have shown better efficacy in preclinical animal models than the combined use (A Novel Bispecific Antibody Targeting PD-L1 and VEGF With Combined Anti-Tumor Activities, Xiaopei Cui, Front Immunol. 2021 Dec 2;12:778978). Recent studies have found that the combined administration of the anti-PD-L1 antibody atezolizumab and bevacizumab is less effective in treating liver cancer in patients with a higher ratio of Tregs to effector T cells (Molecular correlates of clinical response and resistance to atezolizumab in combination with bevacizumab in advanced hepatocellular carcinoma. Andrew X Zhu et al. Nat Med. 2022 Aug;28(8): 1599-1611). This suggests that simultaneously targeting T cells, Treg cells and angiogenesis may provide new therapeutic ideas in clinic.

Therefore, multispecific antibodies that simultaneously target multiple targets, such as bispecific and trispecific antibodies, offer great promise for the clinical treatment of complex diseases. However, it has been recognized in the art that simply linking two or more antibodies or proteins together usually does not induce a synergistic effect and may even have adverse effects. Therefore, bispecific or trispecific antibodies, especially trispecific antibodies, face huge challenges in design and need to consider a large number of variable factors, including molecular compatibility, antibody affinity, stability and pharmaceutical properties.

The present invention solves the above problems and meets the market demand to a certain extent by providing trispecific antibodies that can simultaneously block two major immune checkpoints and one major angiogenesis pathway. The trispecific antibody provided by the present invention can simultaneously target PD-1, CTLA-4 and VEGF in the tumor microenvironment, simultaneously block immunosuppression and angiogenesis, and regulate the immune response and angiogenesis in the tumor microenvironment, thereby achieving the therapeutic effect of the combined use of the three antibodies.

### SUMMARY OF THE INVENTION

The present invention provides novel trispecific antibodies that simultaneously target PD-1, CTLA-4 and VEGF, wherein the trispecific antibodies simultaneously maintain the good antigen binding specificity and selectivity of each antigen binding site and thus have good biological activity. In efficacy studies *in vitro,* the trispecific antibodies of the present invention have similar immune activation activity as the combined use of anti-PD-1 antibody and anti-CTLA-4 antibody, and have similar VEGF blocking activity as bevacizumab. The trispecific antibodies of the present invention have anti-tumor activity comparable to or even superior to that of anti-PD-1 antibody, anti-CTLA-4 antibody and bevacizumab when used in combination in the treatment of various tumors *in vivo* (such as melanoma, lung cancer, colon cancer, liver cancer, *etc*.). Compared with monotherapy and combination therapy, trispecific antibodies targeting PD-1, CTLA-4 and VEGF have the following advantages:
1. synergistically inhibiting tumor cell proliferation and have better therapeutic effects than any single monoclonal antibody;
2. having stronger affinity than multispecific antibodies with 1 valence antigen binding site due to 2 valence antigen binding sites for each target;
3. improving the low immune response of subjects to monoclonal antibodies;
4. reducing or lowering the toxic side effects of anti-CTLA-4 monoclonal antibodies caused by Treg clearance by using FC without or with weak ADCC function;
5. increasing the ratio of trispecific antibodies distributed within- and peri-tumor, and reducing the toxicity of targeting CTLA-4 and VEGF via PD-1-mediated direction;
6. degrading the PD-1 antigen on the cell surface through CTLA-4-mediated endocytosis;
7. reduceing dosing frequency and discomfort caused by combination administration;
8. having increased stability; and
9. prolonging the effective period of tumor suppression.

In general, the trispecific antibody molecules provided by the present invention can recognize three targets, and can significantly reduce costs compared with the combined use of three antibodies; the two identical antigen binding sites of the trispecific antibody molecules for each target essentially maintain the binding ability of the corresponding natural bivalent antibody to the target, and can exert a synergistic effect locally in the tumor. In addition, the trispecific antibodies provided by the present invention have good purity and thermal stability, which are very favorable for downstream development and large-scale production.

Therefore, the present invention mainly relates to the following aspects:
In a first aspect, the present invention provides a trispecific antibody that simultaneously targets PD-1, CTLA-4 and VEGF, wherein the antibody comprises a first, a second, and a third antigen binding site, wherein the first, the second, and the third antigen binding site bind to a first, a second, and a third antigen that are different from each other and independently selected from PD-1, CTLA-4 and VEGF.

In one embodiment, the trispecific antibody provided by the present invention comprises a dimerized Fc region, and one or more of the first, second and third antigen binding sites are connected to the N-terminus and/or C-terminus of the dimerized Fc region.

In another embodiment of the trispecific antibody provided by the present invention, the first, second and third antigen binding sites can be in the form of Fab, scFv, or VHH. In a specific embodiment, the antigen binding site that recognizes the antigen CTLA-4 is in the form of scFv or VHH.

In a specific embodiment of the trispecific antibody provided by the present invention, the antigen binding site that binds to PD-1 comprises heavy chain CDRs (HCDRs) and/or light chain CDRs (LCDRs) as follows: HCDR1 comprising or consisting of the sequence as shown in SEQ ID NO: 1, HCDR2 comprising or consisting of the sequence as shown in SEQ ID NO: 2, and HCDR3 comprising or consisting of the sequence as shown in SEQ ID NO: 3; and/or LCDR1 comprising or consisting of the sequence as shown in SEQ ID NO: 4, LCDR2 comprising or consisting of the sequence shown in as SEQ ID NO: 5, and LCDR3 comprising or consisting of the sequence as shown in SEQ ID NO: 6.

In a specific embodiment of the trispecific antibody provided by the present invention, the antigen binding site that binds to PD-1 comprises a heavy chain variable region and/or a light chain variable region, wherein the heavy chain variable region comprises the sequence as shown in SEQ ID NO:7, or comprises an amino acid sequence having at least 90%, 91%, 92%, 93%, 94%, 95 %, 96%, 97%, 98%, 99% or higher identity to SEQ ID NO:7, or consists of the sequence as shown in SEQ ID NO:7, and the light chain variable region comprises the sequence as shown in SEQ ID NO:8, or comprises an amino acid sequence having at least 90%, 91%, 92%, 93%, 94%, 95%, 96%, 97%, 98%, 99% or higher identity to SEQ ID NO:8, or consists of the sequence as shown in SEQ ID NO:8.

In a specific embodiment of the trispecific antibody provided by the present invention, the antigen binding site that binds to CTLA-4 comprises HCDRs and/or LCDRs as follows: HCDR1 comprising or consisting of the sequence as shown in SEQ ID NO:17, HCDR2 comprising or consisting of the sequence as shown in SEQ ID NO:18, and HCDR3 comprising or consisting of the sequence as shown in SEQ ID NO:19; and/or LCDR1 comprising or consisting of the sequence as shown in SEQ ID NO:20, LCDR2 comprising or consisting of the sequence as shown in SEQ ID NO:21, and LCDR3 comprising or consisting of the sequence as shown in SEQ ID NO:22.

In another specific embodiment of the trispecific antibody provided by the present invention, the antigen binding site that binds to CTLA-4 is VHH and comprises HCDRs as follows: HCDR1 comprising or consisting of the sequence as shown in SEQ ID NO: 12, HCDR2 comprising or consisting of the sequence as shown in SEQ ID NO: 13, and HCDR3 comprising or consisting of the sequence as shown in SEQ ID NO: 14.

In a specific embodiment of the trispecific antibody provided by the present invention, the antigen binding site that binds to CTLA-4 comprises a heavy chain variable region comprising the sequence as shown in SEQ ID NO:15, or comprising an amino acid sequence having at least 90%, 91%, 92%, 93%, 94%, 95%, 96%, 97%, 98%, 99% or higher identity to SEQ ID NO:15, or consisting of the sequence as shown in SEQ ID NO:15, and a light chain variable region comprising the sequence as shown in SEQ ID NO:16, or comprising an amino acid sequence having at least 90%, 91%, 92%, 93%, 94%, 95 %, 96%, 97%, 98%, 99% or higher identity to SEQ ID NO:16, or consisting of the sequence as shown in SEQ ID NO:16.

In a specific embodiment of the trispecific antibodies provided by the present invention, the antigen binding site that binds to CTLA-4 comprises a single VH domain (VHH) comprising the sequence as shown in SEQ ID NO:11, or comprising an amino acid sequence having at least 90%, 91%, 92%, 93%, 94%, 95 %, 96%, 97%, 98%, 99% or higher identity to SEQ ID NO:11, or consisting of the sequence as shown in SEQ ID NO:11.

In a specific embodiment of the trispecific antibody provided by the present invention, the antigen binding site that binds to VEGF comprises HCDRs and/or LCDRs as follows: HCDR1 comprising or consisting of the sequence as shown in SEQ ID NO:26, HCDR2 comprising or consisting of the sequence as shown in SEQ ID NO:27, and HCDR3 comprising or consisting of the sequence as shown in SEQ ID NO:28; and/or LCDR1 comprising or consisting of the sequence as shown in SEQ ID NO:29, LCDR2 comprising or consisting of the sequence as shown in SEQ ID NO:30, and LCDR3 comprising or consisting of the sequence as shown in SEQ ID NO:31.

In a specific embodiment of the trispecific antibody provided by the present invention, the antigen binding site that binds to VEGF comprises a heavy chain variable region and/or a light chain variable region, wherein the heavy chain variable region comprises the sequence as shown in SEQ ID NO:24, or comprises an amino acid sequence having at least 90%, 91%, 92%, 93%, 94%, 95%, 96%, 97%, 98%, 99% or higher identity to SEQ ID NO:24, or consists of the sequence as shown in SEQ ID NO:24, and the light chain variable region comprises the sequence as shown in SEQ ID NO:25, or comprises an amino acid sequence having at least 90%, 91%, 92%, 93%, 94%, 95 %, 96%, 97%, 98%, 99% or higher identity to SEQ ID NO:25, or consists of the sequence as shown in SEQ ID NO:25.

In one embodiment of the trispecific antibody provided by the present invention, the first, second and third antigen binding sites comprise:
1) an antigen binding site that binds to PD-1, comprising HCDR1 comprising or consisting of the sequence as shown in SEQ ID NO:1, HCDR2 comprising or consisting of the sequence as shown in SEQ ID NO:2, and HCDR3 comprising or consisting of the sequence as shown in SEQ ID NO:3; and LCDR1 comprising or consisting of the sequence as shown in SEQ ID NO:4, LCDR2 comprising or consisting of the sequence as shown in SEQ ID NO:5, and LCDR3 comprising or consisting of the sequence as shown in SEQ ID NO:6;
2) an antigen binding site that binds to CTLA-4, comprising
   i) HCDR1 comprising or consisting of the sequence as shown in SEQ ID NO: 17, HCDR2 comprising or consisting of the sequence as shown in SEQ ID NO: 18, and HCDR3 comprising or consisting of the sequence as shown in SEQ ID NO: 19; and LCDR1 comprising or consisting of the sequence as shown in SEQ ID NO: 20, LCDR2 comprising or consisting of the sequence as shown in SEQ ID NO: 21, and LCDR3 comprising or consisting of the sequence as shown in SEQ ID NO: 22; or
   ii) HCDR1 comprising or consisting of the sequence as shown in SEQ ID NO: 12, HCDR2 comprising or consisting of the sequence as shown in SEQ ID NO: 13, and HCDR3 comprising or consisting of the sequence as shown in SEQ ID NO: 14; and
3) an antigen binding site that binds to VEGF, comprising HCDR1 comprising or consisting of the sequence as shown in SEQ ID NO:26, HCDR2 comprising or consisting of the sequence as shown in SEQ ID NO:27, and HCDR3 comprising or consisting of the sequence as shown in SEQ ID NO:28; and LCDR1 comprising or consisting of the sequence as shown in SEQ ID NO:29, LCDR2 comprising or consisting of the sequence as shown in SEQ ID NO:30, and LCDR3 comprising or consisting of the sequence as shown in SEQ ID NO:31.

In one embodiment of the trispecific antibody provided by the present invention, the first, second and third antigen binding sites comprise:
1) an antigen binding site that binds to PD-1, comprising a heavy chain variable region and/or a light chain variable region, wherein the heavy chain variable region comprises the sequence as shown in SEQ ID NO:7, or comprises an amino acid sequence having at least 90%, 91%, 92%, 93%, 94%, 95%, 96%, 97%, 98%, 99% or higher identity to SEQ ID NO:7, or consists of the sequence as shown in SEQ ID NO:7, and the light chain variable region comprises the sequence as shown in SEQ ID NO:8, or comprises an amino acid sequence having at least 90%, 91%, 92%, 93%, 94%, 95%, 96%, 97%, 98%, 99% or higher identity to SEQ ID NO:8, or consists of the sequence as shown in SEQ ID NO:8;
2) an antigen binding site that binds to CTLA-4, comprising
   i) a heavy chain variable region and a light chain variable region, wherein the heavy chain variable region comprises the sequence as shown in SEQ ID NO:15, or comprises an amino acid sequence having at least 90%, 91%, 92%, 93%, 94%, 95%, 96%, 97%, 98%, 99% or higher identity to SEQ ID NO:15, or consists of the sequence as shown in SEQ ID NO:15, and the light chain variable region comprises the sequence as shown in SEQ ID NO:16, or comprises an amino acid sequence having at least 90%, 91%, 92%, 93%, 94%, 95%, 96%, 97%, 98%, 99% or higher identity to SEQ ID NO:16, or consists of the sequence as shown in SEQ ID NO:16;
   ii) comprises the sequence as shown in SEQ ID NO: 11, or comprises an amino acid sequence having at least 90%, 91%, 92%, 93%, 94%, 95%, 96%, 97%, 98%, 99% or higher identity to SEQ ID NO: 11, or consists of the sequence as shown in SEQ ID NO: 11; and
3) an antigen binding site that binds to VEGF, comprising a heavy chain variable region and/or a light chain variable region, wherein the heavy chain variable region comprises the sequence as shown in SEQ ID NO:24, or comprises an amino acid sequence having at least 90%, 91%, 92%, 93%, 94%, 95%, 96%, 97%, 98%, 99% or higher identity to SEQ ID NO:24, or consists of the sequence as shown in SEQ ID NO:24, and the light chain variable region comprises the sequence as shown in SEQ ID NO:25, or comprises an amino acid sequence having at least 90%, 91%, 92%, 93%, 94%, 95%, 96%, 97%, 98%, 99% or higher identity to SEQ ID NO:25, or consists of the sequence as shown in SEQ ID NO:25;

In one embodiment of the trispecific antibody provided by the present invention, the antigen binding site comprises one or more amino acid substitutions, deletions or additions or any combination thereof. In a preferred embodiment, the substitution occurs in the framework region (FR region). In a more preferred embodiment, the substitution is heavy chain variable region G44C, light chain variable region Q100C or G100C (according to Kabat numbering).

In one embodiment of the trispecific antibody provided by the present invention, it comprises a homodimeric or heterodimeric Fc region, which may be the one of immunoglobulin IgG1, IgG2, IgG3, or IgG4 having a native sequence or a variant sequence. In a preferred embodiment, the Fc region comprises modification(s), for example, the Fc region comprises knob-into-hole structure. In a more preferred embodiment, the Fc region comprises substitution selected from the group consisting of S228P, S354C, T366W, T366S, L368A, Y394C, Y407V, H435R, Y436F and K447A (according to the EU numbering system). In one embodiment, the Fc region is derived from the heavy chain constant region sequence as shown in SEQ ID NO:33, 34 or 35.

In one embodiment of the trispecific antibody provided by the present invention, the first, second and third antigen binding sites are connected to each other by a linker/hinge region, or the first, second and third antigen binding sites are connected to the Fc region by a linker/hinge region. In a preferred embodiment, the linker comprises the amino acid sequence (G₄S)ₙ, wherein n is an integer equal to or greater than 1. In a preferred embodiment, the linker consists of the amino acid sequence (G₄S)₃ or (G₄S)₄. In a preferred embodiment, the linker has the sequence as shown in SEQ ID NO:9.

In one embodiment, the present invention provides a trispecific antibody comprising a first, a second, and a third antigen binding site, and consisting of two identical heavy chains and two identical light chains, wherein the heavy chain and the light chain have a structure selected from the following:
1) the heavy chain having the structure of VH-CH1-Fc-VHH-ScFv from N-terminus to C-terminus,
   the light chain having the structure of VL-CL from N-terminus to C-terminus;
2) the heavy chain having the structure of ScFv-VH-CH1-Fc-VHH from N-terminus to C-terminus,
   the light chain having the structure of VL-CL from N-terminus to C-terminus;
3) the heavy chain having the structure of VHH-VH-CH1-Fc-ScFv from N-terminus to C-terminus,
   the light chain having the structure of VL-CL from N-terminus to C-terminus;
4) the heavy chain having the structure of VH-CH1-Fc-ScFv-VHH from N-terminus to C-terminus,
   the light chain having the structure of VL-CL from N-terminus to C-terminus;
5) the heavy chain having the structure of VH-CH1-Fc-ScFv from N-terminus to C-terminus, the light chain having the structure of VL-CL-VHH from N-terminus to C-terminus;
6) the heavy chain having the structure of ScFv-VH-CH1-Fc-ScFv from N-terminus to C-terminus,
   the light chain having the structure of VL-CL from N-terminus to C-terminus;
7) the heavy chain having the structure of VH-CH1-Fc-ScFv from N-terminus to C-terminus, the light chain having the structure of VHH-VL-CL from N-terminus to C-terminus; or
8) the heavy chain having the structure of ScFv-VHH-VH-CH1-Fc from N-terminus to C-terminus,
   the light chain having the structure of VL-CL from N-terminus to C-terminus;
   wherein Fc represents Fc region of immunoglobulin heavy chain, and the two heavy chains comprising the Fc region homodimerize through the Fc region,
   wherein CH1 represents immunoglobulin heavy chain CH1 domain, and CL represents immunoglobulin light chain CL domain,
   wherein VH-CH1 and VL-CL pair with each other to form Fab.
   wherein the first, second and third antigen binding sites are in the form of Fab, VHH and/or ScFv, and each binds to different antigen independently selected from PD-1, CTLA-4 and VEGF.

In a preferred embodiment, adjacent antigen binding sites are connected via a linker, preferably, the antigen binding site is connected to Fc via a linker/hinge region.

In another embodiment, the present invention provides a trispecific antibody comprising a first, second, and third antigen binding site, and consisting of three chains having the following structure:
1) a first heavy chain comprising the structure of VH-CH1-Fc-ScFv from N-terminus to C-terminus,
2) a second heavy chain comprising the structure of VHH-Fc-ScFv from N-terminus to C-terminus, and
3) a light chain comprising the structure of VL-CL from N-terminus to C-terminus,

wherein Fc is the Fc region of an immunoglobulin heavy chain and comprises a knob-into-hole structure, wherein the two heavy chains comprising the Fc region heterodimerize via the Fc region,
wherein CH1 represents the immunoglobulin heavy chain CH1 domain, and CL represents the immunoglobulin light chain CL domain,
wherein VH-CH1 and VL-CL pair with each other to form Fab.
wherein the first, second and third antigen binding sites are in the form of Fab, VHH and/or ScFv, and bind to different antigens independently selected from PD-1, CTLA-4 and VEGF.

In a preferred embodiment, adjacent antigen binding sites are connected via a linker, preferably, the antigen binding site is connected to Fc via a linker/hinge region.

In another embodiment, the present invention provides a trispecific antibody comprising a first, second, and third antigen binding site, and consisting of three chains having the following structure:
1) a first heavy chain comprising the structure of VH-CH1-Fc-ScFv2 from N-terminus to C-terminus,
2) a second heavy chain comprising the structure of ScFv1-Fc-ScFv2 from N-terminus to C-terminus,
3) a light chain comprising the structure of VL-CL from N-terminus to C-terminus,

wherein Fc is the Fc region of an immunoglobulin heavy chain and comprises a knob-into-hole structure, wherein the two heavy chains comprising the Fc region heterodimerize via the Fc region,
wherein CH1 represents the immunoglobulin heavy chain CH1 domain, and CL represents the immunoglobulin light chain CL domain,
wherein VH-CH1 and VL-CL pair with each other to form Fab,
wherein the first, second and third antigen binding sites are in the form of Fab, ScFv1 and/or ScFv2, and each binds to different antigen independently selected from PD-1, CTLA-4 and VEGF.

In a preferred embodiment, adjacent antigen binding sites are connected via a linker, preferably, the antigen binding site is connected to Fc via a linker/hinge region.

In another embodiment, the present invention provides a trispecific antibody comprising a first, second, and third antigen binding site, and consisting of two chains having the following structure:
1) a first heavy chain comprising the structure of ScFv1-Fc-ScFv2 from N-terminus to C-terminus,
2) a second heavy chain comprising the structure of VHH-Fc-ScFv2 from N-terminus to C-terminus,

wherein Fc is the Fc region of an immunoglobulin heavy chain and comprises a knob-into-hole structure, wherein the two heavy chains comprising the Fc region heterodimerize via the Fc region,
wherein the first, second and third antigen binding sites are in the form of VHH, ScFv1 and/or ScFv2, and each binds to different antigen independently selected from PD-1, CTLA-4 and VEGF.

In a preferred embodiment, adjacent antigen binding sites are connected via a linker, preferably, the antigen binding site is connected to Fc via a linker/hinge region.

In a preferred embodiment, the first, second and third antigen binding sites of the above trispecific antibody comprise:
1) an antigen binding site that binds to PD-1, comprising HCDR1 comprising or consisting of the sequence as shown in SEQ ID NO:1, HCDR2 comprising or consisting of the sequence as shown in SEQ ID NO:2, and HCDR3 comprising or consisting of the sequence as shown in SEQ ID NO:3; and LCDR1 comprising or consisting of the sequence as shown in SEQ ID NO:4, LCDR2 comprising or consisting of the sequence as shown in SEQ ID NO:5, and LCDR3 comprising or consisting of the sequence as shown in SEQ ID NO:6;
2) an antigen binding site that binds to CTLA-4, comprising
   i) HCDR1 comprising or consisting of the sequence as shown in SEQ ID NO: 17, HCDR2 comprising or consisting of the sequence as shown in SEQ ID NO: 18, and HCDR3 comprising or consisting of the sequence as shown in SEQ ID NO: 19; and LCDR1 comprising or consisting of the sequence as shown in SEQ ID NO: 20, LCDR2 comprising or consisting of the sequence as shown in SEQ ID NO: 21, and LCDR3 comprising or consisting of the sequence as shown in SEQ ID NO: 22; or
   ii) HCDR1 comprising or consisting of the sequence as shown in SEQ ID NO: 12, HCDR2 comprising or consisting of the sequence as shown in SEQ ID NO: 13, and HCDR3 comprising or consisting of the sequence as shown in SEQ ID NO: 14; and
3) an antigen binding site that binds to VEGF, comprising HCDR1 comprising or consisting of the sequence as shown in SEQ ID NO:26, HCDR2 comprising or consisting of the sequence as shown in SEQ ID NO:27, and HCDR3 comprising or consisting of the sequence as shown in SEQ ID NO:28; and LCDR1 comprising or consisting of the sequence as shown in SEQ ID NO:29, LCDR2 comprising or consisting of the sequence as shown in SEQ ID NO:30, and LCDR3 comprising or consisting of the sequence as shown in SEQ ID NO:31.

In a preferred embodiment, the first, second and third antigen binding sites of the above trispecific antibody comprise:
1) an antigen binding site that binds to PD-1, comprising a heavy chain variable region and a light chain variable region, wherein the heavy chain variable region comprises the sequence as shown in SEQ ID NO:7, or comprises an amino acid sequence having at least 90%, 91%, 92%, 93%, 94%, 95%, 96%, 97%, 98%, 99% or higher identity to SEQ ID NO:7, or consists of the sequence as shown in SEQ ID NO:7, and the light chain variable region comprises the sequence as shown in SEQ ID NO:8, or comprises an amino acid sequence having at least 90%, 91%, 92%, 93%, 94%, 95%, 96%, 97%, 98%, 99% or higher identity to SEQ ID NO:8, or consists of the sequence as shown in SEQ ID NO:8;
2) an antigen binding site that binds to CTLA-4, comprising
   i) a heavy chain variable region and a light chain variable region, wherein the heavy chain variable region comprises the sequence as shown in SEQ ID NO:15, or comprises an amino acid sequence having at least 90%, 91%, 92%, 93%, 94%, 95%, 96%, 97%, 98%, 99% or higher identity to SEQ ID NO:15, or consists of the sequence as shown in SEQ ID NO:15, and the light chain variable region comprises the sequence as shown in SEQ ID NO:16, or comprises an amino acid sequence having at least 90%, 91%, 92%, 93%, 94%, 95%, 96%, 97%, 98%, 99% or higher identity to SEQ ID NO:16, or consists of the sequence as shown in SEQ ID NO:16;
   ii) comprises the sequence as shown in SEQ ID NO: 11, or comprises an amino acid sequence having at least 90%, 91%, 92%, 93%, 94%, 95%, 96%, 97%, 98%, 99% or higher identity to SEQ ID NO: 11, or consists of the sequence as shown in SEQ ID NO: 11; and
3) an antigen binding site that binds to VEGF, comprising a heavy chain variable region and a light chain variable region, wherein the heavy chain variable region comprises the sequence as shown in SEQ ID NO:24, or comprises an amino acid sequence having at least 90%, 91%, 92%, 93%, 94%, 95%, 96%, 97%, 98%, 99% or higher identity to SEQ ID NO:24, or consists of the sequence as shown in SEQ ID NO:24, and the light chain variable region comprises the sequence as shown in SEQ ID NO:25, or comprises an amino acid sequence having at least 90%, 91%, 92%, 93%, 94%, 95%, 96%, 97%, 98%, 99% or higher identity to SEQ ID NO:25, or consists of the sequence as shown in SEQ ID NO:25.

In a preferred embodiment, the first, second, and third antigen binding sites described above comprise substitution(s) in the framework regions (FR regions). In a more preferred embodiment, the substitution is heavy chain variable region G44C, light chain variable region Q100C or G100C (according to Kabat numbering).

In a preferred embodiment, the Fc region of the above trispecific antibody is derived from the Fc region of IgG1 or IgG4. In a preferred embodiment, the Fc region comprises modification(s), for example, the Fc region comprises a knob-into-hole structure. In a more preferred embodiment, the Fc region comprises substitution selected from the group consisting of S228P, S354C, T366W, T366S, L368A, Y394C, Y407V, H435R, Y436F and K447A (according to the EU numbering system). In a preferred embodiment, the Fc region is derived from the heavy chain constant region sequence as shown in SEQ ID NO:33, 34 or 35.

In a preferred embodiment, the linker comprises the amino acid sequence (G₄S)ₙ, wherein n is an integer equal to or greater than 1. In a preferred embodiment, the linker consists of the amino acid sequence (G₄S)₃ or (G₄S)₄. In a preferred embodiment, the linker has the sequence as shown in SEQ ID NO:9.

In a preferred embodiment, the present invention provides a trispecific antibody comprising:
1) a heavy chain comprising SEQ ID NO: 37 or an amino acid sequence having at least 90%, 91%, 92%, 93%, 94%, 95%, 96%, 97%, 98% or 99% identity thereto, and a light chain comprising SEQ ID NO: 38 or an amino acid sequence having at least 90%, 91%, 92%, 93%, 94%, 95%, 96%, 97%, 98% or 99% identity thereto; or
2) a heavy chain comprising SEQ ID NO: 39 or an amino acid sequence having at least 90%, 91%, 92%, 93%, 94%, 95%, 96%, 97%, 98% or 99% identity thereto, and a light chain comprising SEQ ID NO: 40 or an amino acid sequence having at least 90%, 91%, 92%, 93%, 94%, 95%, 96%, 97%, 98% or 99% identity thereto; or
3) a heavy chain comprising SEQ ID NO: 41 or an amino acid sequence having at least 90%, 91%, 92%, 93%, 94%, 95%, 96%, 97%, 98% or 99% identity thereto, and a light chain comprising SEQ ID NO: 42 or an amino acid sequence having at least 90%, 91%, 92%, 93%, 94%, 95%, 96%, 97%, 98% or 99% identity thereto; or
4) a heavy chain comprising SEQ ID NO: 43 or an amino acid sequence having at least 90%, 91%, 92%, 93%, 94%, 95%, 96%, 97%, 98% or 99% identity thereto, and a light chain comprising SEQ ID NO: 44 or an amino acid sequence having at least 90%, 91%, 92%, 93%, 94%, 95%, 96%, 97%, 98% or 99% identity thereto; or
5) a heavy chain comprising SEQ ID NO: 45 or an amino acid sequence having at least 90%, 91%, 92%, 93%, 94%, 95%, 96%, 97%, 98% or 99% identity thereto, and a light chain comprising SEQ ID NO: 46 or an amino acid sequence having at least 90%, 91%, 92%, 93%, 94%, 95%, 96%, 97%, 98% or 99% identity thereto; or
6) a heavy chain comprising SEQ ID NO: 47 or an amino acid sequence having at least 90%, 91%, 92%, 93%, 94%, 95%, 96%, 97%, 98% or 99% identity thereto, and a light chain comprising SEQ ID NO: 48 or an amino acid sequence having at least 90%, 91%, 92%, 93%, 94%, 95%, 96%, 97%, 98% or 99% identity thereto; or
7) a heavy chain comprising SEQ ID NO: 49 or an amino acid sequence having at least 90%, 91%, 92%, 93%, 94%, 95%, 96%, 97%, 98% or 99% identity thereto, and a light chain comprising SEQ ID NO: 50 or an amino acid sequence having at least 90%, 91%, 92%, 93%, 94%, 95%, 96%, 97%, 98% or 99% identity thereto; or
8) a heavy chain comprising SEQ ID NO: 59 or an amino acid sequence having at least 90%, 91%, 92%, 93%, 94%, 95%, 96%, 97%, 98% or 99% identity thereto, and a light chain comprising SEQ ID NO: 60 or an amino acid sequence having at least 90%, 91%, 92%, 93%, 94%, 95%, 96%, 97%, 98% or 99% identity thereto; or
9) a heavy chain comprising SEQ ID NO: 61 or an amino acid sequence having at least 90%, 91%, 92%, 93%, 94%, 95%, 96%, 97%, 98% or 99% identity thereto, and a light chain comprising SEQ ID NO: 62 or an amino acid sequence having at least 90%, 91%, 92%, 93%, 94%, 95%, 96%, 97%, 98% or 99% identity thereto; or
10) a heavy chain comprising SEQ ID NO:63 or an amino acid sequence having at least 90%, 91%, 92%, 93%, 94%, 95%, 96%, 97%, 98% or 99% identity thereto, and a light chain comprising SEQ ID NO:64 or an amino acid sequence having at least 90%, 91%, 92%, 93%, 94%, 95%, 96%, 97%, 98% or 99% identity thereto; or
11) a heavy chain comprising SEQ ID NO: 65 or an amino acid sequence having at least 90%, 91%, 92%, 93%, 94%, 95%, 96%, 97%, 98% or 99% identity thereto, and a light chain comprising SEQ ID NO: 66 or an amino acid sequence having at least 90%, 91%, 92%, 93%, 94%, 95%, 96%, 97%, 98% or 99% identity thereto; or
12) a heavy chain comprising SEQ ID NO: 67 or an amino acid sequence having at least 90%, 91%, 92%, 93%, 94%, 95%, 96%, 97%, 98% or 99% identity thereto, and a light chain comprising SEQ ID NO: 68 or an amino acid sequence having at least 90%, 91%, 92%, 93%, 94%, 95%, 96%, 97%, 98% or 99% identity thereto; or
13) a heavy chain comprising SEQ ID NO: 69 or an amino acid sequence having at least 90%, 91%, 92%, 93%, 94%, 95%, 96%, 97%, 98% or 99% identity thereto, and a light chain comprising SEQ ID NO: 70 or an amino acid sequence having at least 90%, 91%, 92%, 93%, 94%, 95%, 96%, 97%, 98% or 99% identity thereto; or
14) a heavy chain comprising SEQ ID NO: 71 or an amino acid sequence having at least 90%, 91%, 92%, 93%, 94%, 95%, 96%, 97%, 98% or 99% identity thereto, and a light chain comprising SEQ ID NO: 72 or an amino acid sequence having at least 90%, 91%, 92%, 93%, 94%, 95%, 96%, 97%, 98% or 99% identity thereto; or
15) a heavy chain comprising SEQ ID NO: 73 or an amino acid sequence having at least 90%, 91%, 92%, 93%, 94%, 95%, 96%, 97%, 98% or 99% identity thereto, and a light chain comprising SEQ ID NO: 74 or an amino acid sequence having at least 90%, 91%, 92%, 93%, 94%, 95%, 96%, 97%, 98% or 99% identity thereto; or
16) a heavy chain comprising SEQ ID NO: 75 or an amino acid sequence having at least 90%, 91%, 92%, 93%, 94%, 95%, 96%, 97%, 98% or 99% identity thereto, and a light chain comprising SEQ ID NO: 76 or an amino acid sequence having at least 90%, 91%, 92%, 93%, 94%, 95%, 96%, 97%, 98% or 99% identity thereto.

In a preferred embodiment, the present invention provides a trispecific antibody comprising:
1) a first heavy chain comprising SEQ ID NO: 51 or an amino acid sequence having at least 90%, 91%, 92%, 93%, 94%, 95%, 96%, 97%, 98% or 99% identity thereto, a second heavy chain comprising SEQ ID NO: 53 or an amino acid sequence having at least 90%, 91%, 92%, 93%, 94%, 95%, 96%, 97%, 98% or 99% identity thereto, and a light chain comprising SEQ ID NO: 52 or an amino acid sequence having at least 90%, 91%, 92%, 93%, 94%, 95%, 96%, 97%, 98% or 99% identity thereto; or
2) a first heavy chain comprising SEQ ID NO: 56 or an amino acid sequence having at least 90%, 91%, 92%, 93%, 94%, 95%, 96%, 97%, 98% or 99% identity thereto, a second heavy chain comprising SEQ ID NO: 58 or an amino acid sequence having at least 90%, 91%, 92%, 93%, 94%, 95%, 96%, 97%, 98% or 99% identity thereto, and a light chain comprising SEQ ID NO: 57 or an amino acid sequence having at least 90%, 91%, 92%, 93%, 94%, 95%, 96%, 97%, 98% or 99% identity thereto.

In a preferred embodiment, the present invention provides a trispecific antibody comprising:
a first heavy chain comprising SEQ ID NO: 54 or an amino acid sequence having at least 90%, 91%, 92%, 93%, 94%, 95%, 96%, 97%, 98% or 99% identity thereto, and a second heavy chain comprising SEQ ID NO: 55 or an amino acid sequence having at least 90%, 91%, 92%, 93%, 94%, 95%, 96%, 97%, 98% or 99% identity thereto.

In a preferred embodiment, the present invention provides a trispecific antibody comprising:
1) a heavy chain comprising or consisting of SEQ ID NO: 37, and a light chain comprising or consisting of SEQ ID NO: 38; or
2) a heavy chain comprising or consisting of SEQ ID NO: 39, and a light chain comprising or consisting of SEQ ID NO: 40; or
3) a heavy chain comprising or consisting of SEQ ID NO: 41, and a light chain comprising or consisting of SEQ ID NO: 42; or
4) a heavy chain comprising or consisting of SEQ ID NO: 43, and a light chain comprising or consisting of SEQ ID NO: 44; or
5) a heavy chain comprising or consisting of SEQ ID NO: 45, and a light chain comprising or consisting of SEQ ID NO: 46; or
6) a heavy chain comprising or consisting of SEQ ID NO: 47, and a light chain comprising or consisting of SEQ ID NO: 48; or
7) a heavy chain comprising or consisting of SEQ ID NO: 49, and a light chain comprising or consisting of SEQ ID NO: 50; or
8) a heavy chain comprising or consisting of SEQ ID NO: 59, and a light chain comprising or consisting of SEQ ID NO: 60; or
9) a heavy chain comprising or consisting of SEQ ID NO: 61, and a light chain comprising or consisting of SEQ ID NO: 62; or
10) a heavy chain comprising or consisting of SEQ ID NO: 63, and a light chain comprising or consisting of SEQ ID NO: 64; or
11) a heavy chain comprising or consisting of SEQ ID NO: 65, and a light chain comprising or consisting of SEQ ID NO: 66; or
12) a heavy chain comprising or consisting of SEQ ID NO: 67, and a light chain comprising or consisting of SEQ ID NO: 68; or
13) a heavy chain comprising or consisting of SEQ ID NO: 69, and a light chain comprising or consisting of SEQ ID NO: 70; or
14) a heavy chain comprising or consisting of SEQ ID NO: 71, and a light chain comprising or consisting of SEQ ID NO: 72; or
15) a heavy chain comprising or consisting of SEQ ID NO: 73, and a light chain comprising or consisting of SEQ ID NO: 74; or
16) a heavy chain comprising or consisting of SEQ ID NO: 75, and a light chain comprising or consisting of SEQ ID NO: 76.

In a preferred embodiment, the present invention provides a trispecific antibody comprising:
1) a first heavy chain comprising or consisting of SEQ ID NO: 51, a second heavy chain comprising or consisting of SEQ ID NO: 53, and a light chain comprising or consisting of SEQ ID NO: 52; or
2) a first heavy chain comprising or consisting of SEQ ID NO: 56, a second heavy chain comprising or consisting of SEQ ID NO: 58, and a light chain comprising or consisting of SEQ ID NO: 57.

In a preferred embodiment, the present invention provides a trispecific antibody comprising:
a first heavy chain comprising or consisting of SEQ ID NO: 54, and a second heavy chain comprising or consisting of SEQ ID NO: 55.

In a second aspect, the present invention provides a polynucleotide encoding the trispecific antibody molecule of the present invention; a vector comprising the polynucleotide; and a host cell comprising the polynucleotide or the vector of the present invention.

In one embodiment, the vector is preferably an expression vector.

In one embodiment, the host cell may be a prokaryotic cell or a eukaryotic cell commonly used in the art.

In one embodiment, the present invention provides a host cell comprising one or more polynucleotides of the invention. In some embodiments, a host cell comprising the vector of the invention is provided. Suitable host cells include prokaryotic microorganisms, such as Escherichia coli, eukaryotic microorganisms, such as filamentous fungi or yeast, or various eukaryotic cells, such as Chinese hamster ovary cells (CHOs), insect cells, and the like. Mammalian cell lines suitable for suspension culture can be used. Examples of useful mammalian host cell lines include SV40 transformed monkey kidney CV1 line (COS-7), human embryonic kidney line (HEK293 or 293F cells), baby hamster kidney cells (BHK), monkey kidney cells (CV1), African green monkey kidney cells (VERO-76), human cervical carcinoma cells (HELA), canine kidney cells (MDCK), Buffalo rat liver cells (BRL 3A), human lung cells (W138), human liver cells (HepG2), CHO cells, NSO cells, myeloma cell lines such as Y0, NS0, P3X63 and Sp2/0, etc. In a preferred embodiment, the host cell is CHO, HEK293 or NSO cell.

In a third aspect, the present invention provides a method for producing the trispecific antibody of the present invention, including the steps of (i) culturing the host cell disclosed in the second aspect of the present invention under conditions suitable for expressing the trispecific antibody disclosed in the first aspect of the present invention, and optionally, (ii) recovering the trispecific antibody of the present invention.

In a fourth aspect, the present invention provides a pharmaceutical composition comprising the trispecific antibody molecule of the present invention.

In one embodiment, the pharmaceutical composition provided by the present invention further comprises other therapeutic agents, and optional pharmaceutical excipients; preferably, the other therapeutic agents are selected from chemotherapeutic agents, cytotoxic agents, and the like.

In a fifth aspect, the present invention provides use of the trispecific antibody and pharmaceutical composition of the present invention for treating, preventing and/or diagnosing cancer, autoimmune diseases, infectious diseases or angiogenesis-related diseases.

In one embodiment, the present invention provides the use of any antibody described in the first aspect, any polynucleotide or vector or host cell described in the second aspect, and the pharmaceutical composition described in the fourth aspect in the preparation of a medicament for the treatment, prevention and/or diagnosis of cancer, autoimmune diseases, infectious diseases or angiogenesis-related diseases.

In one embodiment, the present invention provides any antibody of the first aspect, any polynucleotide or vector or host cell of the second aspect, and the pharmaceutical composition of the fourth aspect for use in the treatment, prevention and/or diagnosis.

In one embodiment, the present invention provides any antibody of the first aspect, any polynucleotide or vector or host cell of the second aspect, and the pharmaceutical composition of the fourth aspect for use in the treatment, prevention and/or diagnosis of cancers, autoimmune diseases, infectious diseases or angiogenesis-related diseases.

In one embodiment, the cancer is, for example, solid tumor such as lung cancer (e.g., small cell lung cancer, non-small cell lung cancer), breast cancer, liver cancer, bladder cancer, breast cancer, melanoma, colon cancer, rectal cancer, ovarian cancer, cervical cancer, prostate cancer, pancreatic adenocarcinoma, basal cell carcinoma, esophageal cancer, bile duct cancer, head and neck squamous cell carcinoma, thyroid cancer, brain cancer, gastric cancer, head and neck cancer, head and neck squamous cell carcinoma, kidney cancer, testicular cancer, multiple myeloma, glioblastoma, glioma, and hematologic tumor such as leukemia, lymphoma (e.g., Hodgkin's lymphoma, non-Hodgkin's lymphoma, diffuse large B-cell lymphoma, acute B-cell lymphoma, follicular lymphoma),etc.

In a sixth aspect, the present invention provides a method for treating, preventing and/or diagnosing cancers, autoimmune diseases, infectious diseases or angiogenesis-related diseases, including administering an effective amount of the trispecific antibody of the present invention, or the pharmaceutical composition of the present invention to a patient in need thereof.

In one embodiment, the cancer is, for example, solid tumor such as lung cancer (e.g., small cell lung cancer, non-small cell lung cancer), breast cancer, liver cancer, bladder cancer, breast cancer, melanoma, colon cancer, rectal cancer, ovarian cancer, cervical cancer, prostate cancer, pancreatic adenocarcinoma, basal cell carcinoma, esophageal cancer, bile duct cancer, head and neck squamous cell carcinoma, thyroid cancer, brain cancer, gastric cancer, head and neck cancer, head and neck squamous cell carcinoma, kidney cancer, testicular cancer, multiple myeloma, glioblastoma, glioma, and hematologic tumor such as leukemia, lymphoma (e.g., Hodgkin's lymphoma, non-Hodgkin's lymphoma, diffuse large B-cell lymphoma, acute B-cell lymphoma, follicular lymphoma), etc.

### DESCRIPTION OF THE DRAWINGS

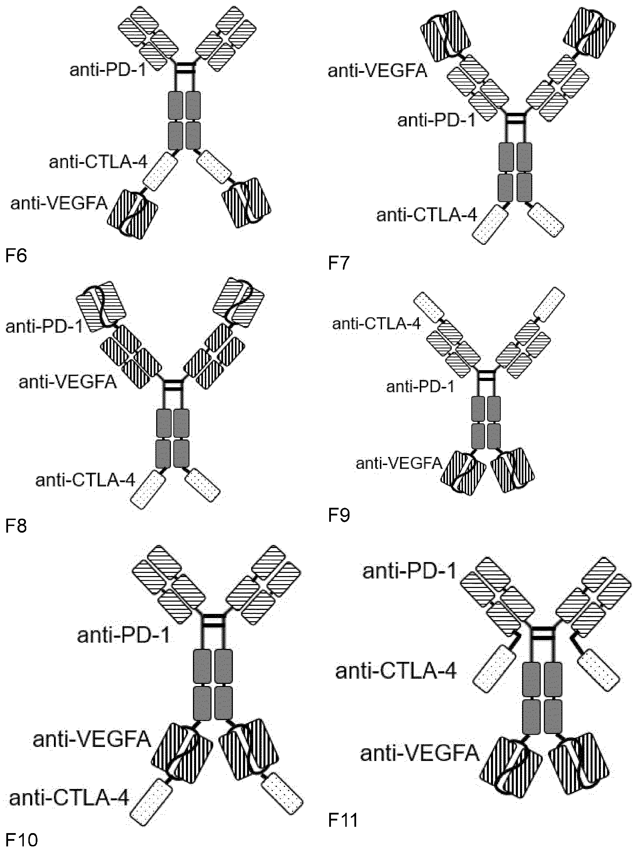

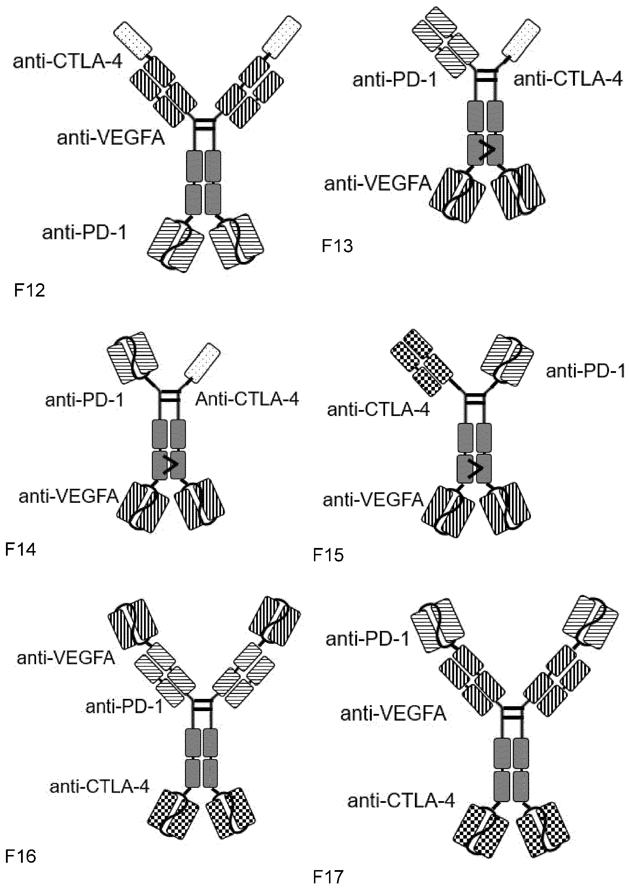

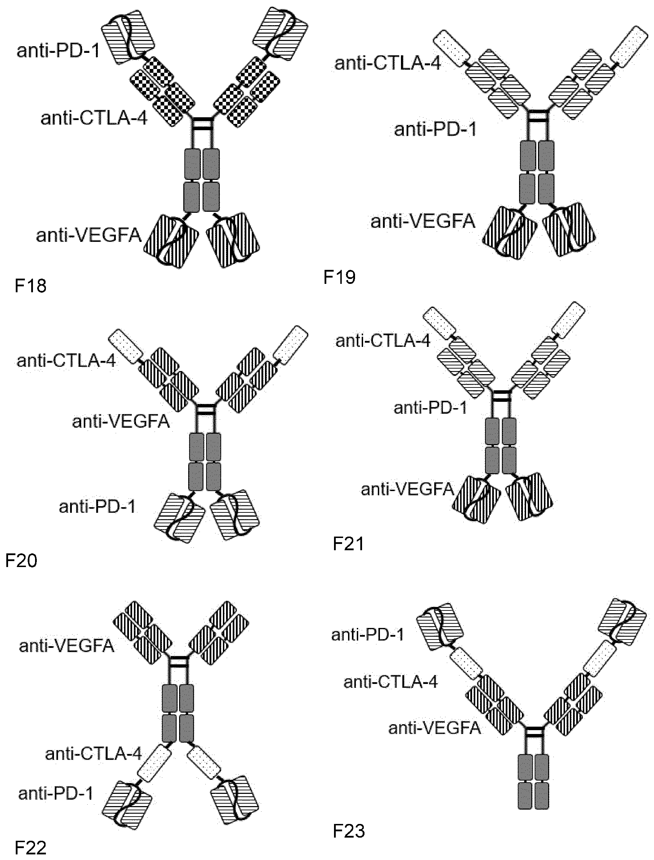

Figure 1 shows schematic diagram of the structure of the trispecific antibodies constructed in the present application.
Figure 2 shows the activity of the trispecific antibodies binding to cell surface PD-1. The activity of the trispecific antibodies binding to 293T-hPD-1 cells was detected with FACS. Figure A and Figure B are assays FACS#1 and FACS#2, respectively.
Figure 3 shows the activity of the trispecific antibodies binding to cell surface CTLA-4. The activity of the trispecific antibodies binding to CHO-hCTLA-4 cells was detected with FACS. Figure A and Figure B are assays FACS#3 and FACS#4, respectively.
Figure 4 shows the activity of the trispecific antibodies binding to the activated T cells. Figure A shows the activity of the trispecific antibodies and anti-PD-1 antibodies binding to T cells; Figure B shows the activity of the anti-CTLA-4 antibodies binding to T cells.
Figure 5 shows the activity of the trispecific antibodies binding to free VEGFA after binding to 293T-hPD-1-hCTLA-4 cells (co-expressing PD-1 and CTLA-4).
Figure 6 shows the activity of the trispecific antibodies binding to free VEGFA after binding to the activated T cells.
Figure 7 shows the ability of the trispecific antibodies to enhance SEB-stimulated IL-2 secretion from PBMCs. Panels A and B show the results of IL-2 secretion by PBMCs from donors Lot#A10Z707023 and Lot#A10Z647018, respectively, after treatment with SEB and antibodies for 3 days.
Figure 8 shows that the trispecific antibodies promote IL-2 secretion in MLR. Trispecific antibodies or control antibodies were added to the MLR, and IL-2 secretion was measured 4 days later. Figure A: DC donor: Lot#Z0160, PBMC donor: Lot#Z0177, IL-2 secretion was detected 4 days later; Figure B: DC donor: Lot#Z0160, PBMC donor Lot#Z0182.
Figure 9 shows that different concentrations of trispecific antibodies increase IL-2 secretion in MLRs containing different ratios of Tregs. The concentrations of the test antibodies in A-C were 500, 50, and 5 nM, respectively, and IL-2 secretion was measured 4 days after treating MLR.
Figure 10 shows that the trispecific antibodies inhibit VEGFA-induced proliferation of human umbilical vein endothelial cells.
Figure 11 shows that the trispecific antibody HC010-F8 inhibits the growth of human melanoma A375 in human PBMC humanized mice.
Figure 12 shows that the trispecific antibody HC010-F8 does not affect the body weight of mice in the human PBMC humanized human melanoma A375 mouse model.
Figure 13 shows the binding of trispecific antibodies to VEGFA (Panel A) and CTLA-4 (Panel B) after binding to human PD-1.
Figure 14 shows the binding of trispecific antibodies to VEGFA (Panel A) and CTLA-4 (Panel B) after binding to cell surface human PD-1.
Figure 15 shows the binding of trispecific antibodies to PD-1 (Panel A) and VEGF (Panel B) after binding to cell surface human CTLA-4.
Figure 16 shows that the trispecific antibodies HC010-F8 and HC010-F23 inhibit the growth of human melanoma A375 in human PBMC humanized mice.
Figure 17 shows that the trispecific antibodies HC010-F8 and HC010-F23 does not affect the body weight of mice in the human PBMC humanized human melanoma A375 mouse model.
Figure 18 shows the activity of trispecific antibodies in inhibiting human non-small cell lung cancer A549 cells in mice.
Figure19 shows the activity of trispecific antibodies in inhibiting human non-small cell lung cancer H1299 cells in mice.
Figure 20 shows the activity of trispecific antibodies in inhibiting human liver cancer Huh7 cells in mice.

### DETAILED DESCRIPTION OF THE INVENTION

Unless defined otherwise, all technical and scientific terms used herein have the same meaning as commonly understood by one of ordinary skill in the art to which this invention belongs. All publications, patent applications, patents, and other references mentioned herein are incorporated by reference in their entirety. In addition, the materials, methods, and examples described herein are illustrative only and not intended to be limiting. Other features, objects, and advantages of the invention will be apparent from the description and drawings, and from the appended claims.

### I. Definitions

The term "about" when used in connection with a numerical value is meant to encompass numerical values within the range between the lower limit of 5% less than the specified numerical value and the upper limit of 5% greater than the specified numerical value.

As used herein, the term "comprising" or "including" means to include the stated elements, integers or steps, but does not exclude any other elements, integers or steps.

The term "antibody" is used herein in the broadest sense to refer to a protein that contains an antigen binding site.

The terms "antigen binding site" and "antigen binding domain" are used interchangeably to refer to the region of an antibody molecule that actually binds to an antigen. Antigen binding sites include, but are not limited to, Fv, Fab fragment, Fab', Fab'-SH, F(ab')2, single-chain antibody molecule (*e.g.,* scFv), VHH and the like.

The term "immunoglobulin" refers to a protein having the structure of a naturally occurring antibody and is often used interchangeably with the term "antibody" in this application. IgG class immunoglobulins are heterotetrameric glycoproteins composed of two light chains and two heavy chains bonded by disulfide bonds. From N-terminus to C-terminus, each immunoglobulin heavy chain has a heavy chain variable region (VH), also called a heavy chain variable domain, followed by three heavy chain constant domains (CH1, CH2 and CH3). Similarly, from N-terminus to C-terminus, each immunoglobulin light chain has a light chain variable region (VL), also called a light chain variable domain, followed by a light chain constant domain (CL). In IgG molecules, the VH-CH1 of the heavy chain usually pairs with the VL-CL of the light chain to form a Fab fragment that specifically binds to the antigen. Thus, one IgG immunoglobulin essentially consists of two Fab molecules connected by the immunoglobulin hinge region and two dimerized Fc regions. The heavy chains of immunoglobulins can be assigned to one of five classes, called α (IgA), δ (IgD), ε (IgE), γ (IgG) or µ (IgM), based on the type of their constant region, some of which can be further divided into subclasses, such as γ1 (IgG1), γ2 (IgG2), γ3 (IgG3), γ4 (IgG4), α1 (IgA1) and α2 (IgA2). The light chains of immunoglobulins can also be assigned to one of two types, called kappa and lambda, based on the amino acid sequences of their constant domains.

The term "variable region" or "variable domain" of an antibody refers to the domain of an antibody heavy or light chain that is involved in binding the antibody to antigen. The variable regions of an antibody can be further subdivided into regions of hypervariability, *i.e.,* complementarity determining regions (CDRs), and regions that are more conserved, *i.e*., framework regions (FRs), intervening between the hypervariable regions. In the case of IgG class immunoglobulins, the heavy chain variable region or the light chain variable region comprises FR1-CDR1-FR2-CDR2-FR3-CDR3-FR4 in order, respectively, from N-terminus to C-terminus. In the case of heavy chain antibodies (also referred to herein as nanobodies), e.g. from Camelidae, the antigen binding site consists of a single VH domain (*i.e.,* a "VHH" domain). The VHH of a natural heavy chain antibody has a similar structure to the heavy chain variable region of a natural IgG antibody, namely, it contains four conserved framework regions (FRs) and three complementarity determining regions (CDRs).

The term "multispecific antibody" refers to an antibody that has at least two antigen binding sites, each of which binds to a different epitope of the same antigen or to different epitopes of different antigens.

The expression "valency" in relation to an antibody refers to the total number of antigen binding sites in the antibody molecule, or the number of antigen binding sites with the same antigen binding specificity. For example, a hexavalent antibody means that regardless of whether the bound epitopes are the same, the antibody molecule contains a total of 6 antigen binding sites. Preferably, in the present invention, the hexavalent antibody has three different antigen binding specificities, wherein there are 2 identical antigen binding sites for each antigen binding specificity.

"Heavy chain constant region domain" or "heavy chain constant region" refers to a constant region domain from, obtained from or derived from an immunoglobulin heavy chain, including heavy chain constant regions CH1, CH2, CH3, and optionally heavy chain constant region CH4 covalently linked sequentially from N-terminus to C-terminus. In most cases, the heavy chain constant regions CH1 and CH2 are connected by a heavy chain hinge region, but may also be connected by a flexible linker when appropriate. In some embodiments of the present invention, the heavy chain constant region of the antibody molecule of the present invention comprises CH1- Hinge -CH2-CH3. The heavy chain constant region domain can be selected according to the intended function of the antibody molecule. For example, the constant domain may be an IgA, IgD, IgE, IgG or IgM domain, in particular an immunoglobulin constant domain of human IgG, such as a constant domain of human IgG1, IgG2, IgG3 or IgG4.

In the multispecific antibody of the present invention, the chain containing the Fc domain is a heavy chain, and the chain without the Fc domain is a light chain. In some embodiments of the invention, the antibody molecules of the invention are composed of two identical heavy chains and two identical light chains. In other embodiments of the present invention, the antibody molecules of the present invention comprise two different heavy chains.

The term "Fc domain" or "Fc region" refers to the C-terminal region of an immunoglobulin heavy chain that contains at least a portion of the constant region. The term includes naturally occurring Fc region, or variant Fc region. The Fc domain that can be used in the antibodies of the present invention includes, but is not limited to, the Fc domain of IgG1, IgG2, IgG3, or IgG4 having a naturally occuring sequence or a variant sequence. The lysine residue at position 447 at the C-terminus of the Fc domain (according to the EU numbering system) may be present or absent. Unless otherwise indicated herein, the numbering of amino acid residues in the Fc region or the heavy chain constant region is according to the EU numbering system as described in Kabat et al., Sequences of Proteins of Immunological Interes, 5th edition, Public Health Service, National Institutes of Health, Bethesda, MD, 1991 (also called the EU index).

The Fc domain, fusion proteins comprising the Fc domain (e.g., antibodies) can be modified using a variety of known methods, such as modifications to reduce immunogenicity, improve stability, solubility, function and clinical benefit. Such modifications include, but are not limited to, the following modifications: modification of the amino acid residue K447 to increase the stability of IgG, preferably the amino acid substitution K447A; modification of the amino acid residue S228 to address the issue of the IgG4 heterogeneity, preferably the amino acid substitution S228P; the knobs-into-holes structure that increases the stability of the antibody, wherein the knob can occur at the amino acid residues S354 and T366, and the hole can occur at the amino acid residues Y394, T366, L368, and Y407, preferably the knob can be the following amino acid substitution: S354C, T366W, and the hole can be the following amino acid substitution: Y394C, T366S, L368A, Y407V; the modification of the hole structure that reduces the homodimer can occur at the amino acid residues H435 and Y436, preferably the amino acid substitutions H435R and Y436F.

"Complementarity determining regions" or "CDR regions" or "CDRs" or "hypervariable regions" are regions of an antibody variable domain that are highly variable in sequence and form structurally defined loops ("hypervariable loops") and/or contain antigen contact residues ("antigen contact points"). The CDRs are mainly responsible for binding to antigenic epitopes.

The term "immune checkpoint" refers to a class of inhibitory signaling molecules present in the immune system, which prevent tissue damage by regulating the persistence and intensity of immune responses in peripheral tissues and participate in maintaining tolerance to self-antigens (Pardoll DM., The blockade of immune checkpoints in cancer immunotherapy. Nat Rev Cancer, 2012, 12(4): 252-264). Studies have found that one of the reasons that tumor cells are able to evade the body's immune system and proliferate uncontrollably is that they utilize the inhibitory signaling pathway of immune checkpoints, thereby inhibiting the activity of T lymphocytes and making it impossible for T lymphocytes to effectively kill tumors. Immune checkpoint molecules include but are not limited to programmed death 1 (PD-1), programmed cell death-ligand 1 (PD-L1), PD-L2, cytotoxic T lymphocyte antigen 4 (CTLA-4), LAG-3 and TIM-3.

The term "PD-1" refers to programmed cell death protein 1 expressed on the surface of T cells, its ligandPD-L1 or PD-L2 is expressed on the surface of a variety of cell types, including many cancer cells. When PD-1 binds to its ligand (PD-L1 or PD-L2), it inhibits T cell activation by recruiting SHP-2, thereby inhibiting T cell proliferation and effector functions, such as IFN-γ production and cytotoxic activity. Cancer cells also produce immune evasion by inhibiting T-cells from attacking them via ligands or PD-L2 on their surface by this mechanism. The term "PD-1" as used herein includes human PD-1, human PD-1 variants, isoforms and species homologues.

The term "human PD-1" refers to the human PD-1 protein encoded by the wild-type human PD-1 gene, such as human PD-1 disclosed in GenBank Accession No. NM_005018.2.

The term "CTLA-4", *i.e.,* cytotoxic T-lymphocyte-associated protein 4, inhibits immune responses by binding to the ligands CD80 (also known as B7-1) and CD86 (also known as B7-2). CTLA-4 inhibits immune responses in multiple ways: for example, 1) competing with the T cell co-stimulatory receptor CD28 for its ligands CD80 and CD86, thereby blocking co-stimulation; and 2) sending negative signals that inhibit T cell activation. CTLA-4 inhibitors can cause T cells to proliferate in large numbers and to attack tumor cells by inhibiting CTLA-4 molecules. As used herein, the term "CTLA-4" includes human "CTLA-4", human CTLA-4 variants, isoforms and species homologues. Human CTLA-4 is disclosed, for example, in GenBank Accession No. AAB59385.

Both PD-1 and CTLA-4 are immune checkpoints belonging to the CD28 family and are expressed on activated T cells, but the two differ somewhat from each other in different stages of the immune response and in the expression of different immune cells. PD-1 inhibitors and CTLA-4 inhibitors have different activation effects on different immune cells. In addition to activating exhausted T cells, PD-1 inhibitors mainly activate cytotoxic T cells and Treg, while CTLA-4 inhibitors mainly activate Th1 effector T cells and Tfh cells (PMID: 35241833). Similar to the phenotypes of PD-1 knockout and CTLA-4 knockout mice, PD-1 inhibitors are less toxic than CTLA-4 inhibitors, while CTLA-4 inhibitors tend to have greater toxicity, which may be related to their clearance of peripheral Tregs. For example, the widespread clinical use of the anti-CTLA-4 antibody Ipilimumab for the treatment of advanced melanoma has been limited due to its severe toxic side effects.

The term "VEGF" refers to vascular endothelial growth factor, also known as vascular permeability factor (VPF), which is a highly specific vascular endothelial cell growth factor that promotes vascular permeability, extracellular matrix degeneration, vascular endothelial cell migration, proliferation and angiogenesis, etc. VEGF is a family that includes VEGFA, VEGFB, VEGFC, VEGFD, VEGFE and placental growth factor (PGF).

The term "VEGFA (vascular endothelial growth factor A)" is a highly conserved 27 kDa dimeric glycoprotein secreted by a variety of cells including endothelial cells and tumors, etc. Common isoforms of VEGFA produced by alternative splicing include VEGFA121, VEGFA165, VEGFA189 and VEGFA206, which contain 121, 165, 189 and 206 amino acids, respectively. VEGFA includes human VEGFA, for example, the human VEGFA protein under accession number UniProt NO.: P15692. VEGFA is a key regulator of angiogenesis during the growth of solid tumors. Therefore, treatment targeting VEGFA is one of the current focuses of oncology. Bevacizumab is the first approved VEGFA inhibitor. Throughout this application, VEGF generally refers to VEGFA, for example, VEGF165 generally refers to VEGFA165 .

The term "EC₅₀", also known as "half effective concentration", refers to the concentration of a medicament, antibody or toxic agent that induces a response that is 50% between baseline and maximum after a specified exposure time. In the context of this application, the unit of EC₅₀ is "nM".

The term "IC50", also known as "half inhibitory concentration", refers to the concentration of a medicament or substance (inhibitor) that inhibits a biological process (or certain substances involved in the process, such as enzymes, cell receptors or microorganisms) by 50%.

The terms "flexible linker" or "linker" or "connector peptide" are used interchangeably and refer to a short amino acid sequence consisting of amino acids, such as glycine (G) and/or serine (S) and/or threonine residues (T), used alone or in combination, or from the hinge region of an immunoglobulin.

Linkers that can be used in the present invention can be readily determined by one skilled in the art. For example, the linker comprises the amino acid sequence (G₄S)ₙ, wherein n is an integer equal to or greater than 1. In a preferred embodiment, the linker consists of the amino acid sequence (G₄S)₃ or (G₄S)₄. The linker that can be used in the antibody molecule of the present invention can also be, for example but not limited to, the following amino acid sequence:(G₃S)₂, (G₄S)₂, (G₃S)₃, (G₄S)₃, (G₃S)₄, (G₄S)₄, (G₃S)₅, (G₄S)₅, (G₃S)₆, (G₄S)₆, GGG, DGGGS, TGEKP, GGRR, EGKSSGSGSESKVD, KESGSVSSEQLAQFRSLD, GGRRGGGS, LRQRDGERP, LRQKDGGGSERP and GSTSGSGKPGSGEGSTKG

As used herein, the term "binding" or "specific binding" means that the binding is selective for the antigen and can be distinguished from unwanted or non-specific interactions. The ability of an antigen binding site to bind a specific antigen can be determined by enzyme-linked immunosorbent assay (ELISA) or conventional binding assays known in the art.

"Percentage (%) identity" of an amino acid sequence refers to the percentage of amino acid residues in the candidate sequence that are identical to the amino acid residues in the specific amino acid sequence shown in this specification, after aligning the candidate sequence with the specific amino acid sequence shown in this specification and introducing gaps, if necessary, to achieve the maximum percentage of sequence identity, and not considering any conservative substitutions as part of the sequence identity. In some embodiments, the present invention contemplates variants of the antibody molecules of the invention having a substantial degree of identity, such as at least 80%, 85%, 90%, 95%, 97%, 98% or 99% or more, relative to the antibody molecules and sequences thereof specifically disclosed herein. The variant may contain conservative modifications.

For polypeptide sequences, "conservative modifications" include substitutions, deletions or additions to the polypeptide sequence which result in the substitution of an amino acid with a chemically similar amino acid. Conservative substitution tables providing functionally similar amino acids are well known in the art. Such conservatively modified variants are additional to and do not exclude the polymorphic variants, interspecies homologues, and alleles of the invention. The following eight groups contain amino acids that are conservative substitutions for each other: 1) alanine (A), glycine (G); 2) aspartic acid (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M) (see, e.g., Creighton, Proteins (1984)). In some embodiments, the term "conservative sequence modifications" is used to refer to amino acid modifications that do not significantly affect or alter the binding characteristics of the antibody containing the amino acid sequence.

The term "host cell" refers to a cell into which an exogenous polynucleotide has been introduced, including the progeny of such cells. Host cells include "transformants" and "transformed cells," which include the primary transformed cell and progeny derived therefrom. Host cells are any type of cell system that can be used to produce the antibody molecules of the invention, including eukaryotic cells, such as mammalian cells, insect cells, yeast cells; and prokaryotic cells, such as *E*. *coli* cells. Host cells include cultured cells, as well as cells within transgenic animals, transgenic plants, or cultured plant tissues or animal tissues.

The term "expression vector" refers to a vector comprising a recombinant polynucleotide comprising expression control sequences operably linked to a nucleotide sequence to be expressed. The expression vector contains sufficient cis-acting elements for expression; other elements for expression can be provided by the host cell or by *in vitro* expression system. Expression vectors include all those known in the art, including cosmids, plasmids (*e.g*., naked or contained in liposomes), and viruses (*e.g*., lentiviruses, retroviruses, adenoviruses, and adeno-associated viruses) into which the recombinant polynucleotide is incorporated.

The terms "individual" or "subject" are used interchangeably to refer to a mammal. Mammals include, but are not limited to, domesticated animals (*e.g.*, cows, sheep, cats, dogs, and horses), primates (*e.g.,* humans and non-human primates such as monkeys), rabbits, and rodents (*e.g.,* mice and rats). In particular, the individual is a human being.

The term "anti-tumor effect" refers to a biological effect that can be exhibited by a variety of means, including but not limited to, for example, a decrease in tumor volume, a decrease in the number of tumor cells, a decrease in tumor cell proliferation, or a decrease in tumor cell survival. The terms "tumor" and "cancer" are used interchangeably herein and encompass both solid tumors and liquid tumors.

The term "cancer" refers to the physiological disorder in mammals that is typically characterized by unregulated cell growth. Examples of cancer include, but are not limited to, carcinoma, lymphoma, blastoma, sarcoma, and leukemia or lymphoid malignancy. The cancer is, for example, solid tumor such as lung cancer (*e.g.*, small cell lung cancer, non-small cell lung cancer), breast cancer, liver cancer, bladder cancer, breast cancer, melanoma, colon cancer, rectal cancer, ovarian cancer, cervical cancer, prostate cancer, pancreatic adenocarcinoma, basal cell carcinoma, esophageal cancer, bile duct cancer, head and neck squamous cell carcinoma, thyroid cancer, brain cancer, gastric cancer, head and neck cancer, head and neck squamous cell carcinoma, kidney cancer, testicular cancer, multiple myeloma, glioblastoma, glioma, and hematologic tumor such as leukemia, lymphoma (*e.g.*, Hodgkin's lymphoma, non-Hodgkin's lymphoma, diffuse large B-cell lymphoma, acute B-cell lymphoma, follicular lymphoma),etc. In certain embodiments, cancers suitable for treatment by the antibodies of the invention include breast cancer, gastric cancer, ovarian cancer, gastroesophageal junction cancer, bladder cancer, small intestine cancer and ampullary cancer, esophageal cancer, lung cancer, and cervical cancer. The metastatic forms of those cancers are also included. In some embodiments, the present invention provides, inter alia, multispecific antibodies useful for the treatment of tumor/cancer, and the therapeutic use thereof in the treatment of said tumor/cancer.

The term "treatment" refers to clinical intervention intended to alter the natural course of a disease in the individual being treated. Desired therapeutic effects include, but are not limited to, preventing disease appearance or recurrence, alleviating symptoms, reducing any direct or indirect pathological consequences of the disease, preventing metastasis, reducing the rate of disease progression, ameliorating or palliating the disease state, and alleviating or improving prognosis. In some embodiments, the antibody molecules of the invention are used to delay the development of a disease or to slow the progression of a disease.

The term "prevention" includes the inhibition of the occurrence or progression of a disease or disorder or symptoms of a particular disease or disorder. In some embodiments, subjects with a family history of cancer are candidates for prophylactic regimens. Generally, in the context of cancer, the term "prevention" refers to the administration of a medicament prior to the onset of signs or symptoms of cancer, particularly in subjects at risk of cancer.

The term "effective amount" refers to an amount or dose of an antibody or composition of the invention to produce the desired effect in a patient in need of treatment or prevention after being administered to the patient in single or multiple doses. The effective amount can be readily determined by the attending physician skilled in the art, by taking into account various factors such as the species; body weight; age and general health condition of the mammals; the specific disease involved; the degree or severity of the disease; the response of the individual patient; the specific antibody to be administered; the mode of administration; the bioavailability profile of the preparation to be administered; the selected dosing regimen; and the use of any concomitant therapy.

The term "therapeutically effective amount" refers to an amount effective to achieve the desired therapeutic result, at necessary dosages and for periods of time necessary. A therapeutically effective amount of the antibody or antibody fragment or composition may vary according to factors such as the disease state, age, sex, and body weight of the individual, and the ability of the antibody or antibody portion to elicit a desired response in the individual. A therapeutically effective amount is also the one in which any toxic or detrimental effects of the antibody or antibody fragment or composition are outweighed by the therapeutically beneficial effects. The "therapeutically effective amount" preferably inhibits a measurable parameter (*e.g.,* tumor growth rate, tumor volume, *etc.*) by at least about 20%, more preferably at least about 40%, even more preferably at least about 50%, 60% or 70%, and still more preferably at least about 80% or 90% relative to untreated subjects. The ability of a compound to inhibit a measurable parameter (*e*.*g*., cancer) can be evaluated in an animal model system in which the efficacy in human tumors is predictive.

The term "prophylactically effective amount" refers to an amount effective to achieve the desired prophylactic result, at necessary dosages and for periods of time necessary. Typically, since a prophylactic dose is used in subjects prior to or at an earlier stage of disease, the prophylactically effective amount will be less than the therapeutically effective amount.

The term "pharmaceutical composition" refers to a composition that is in a form that allows the biological activity of the active ingredients contained therein to be effective and does not contain additional ingredients unacceptably toxic to a subject who will be administered with the composition.

### II. Production and purification of antibodies of the invention

To produce the trispecific antibodies of the present invention, each polypeptide chain of the antibodies of the present invention can be obtained, for example, by solid-state peptide synthesis (*e.g*. Merrifield solid phase synthesis) or recombinant production methods, and assembled under appropriate conditions.

In the case of recombinant production, the polynucleotide(s) encoding any one and/or more than one polypeptide chains of the antibody can be isolated and inserted into one or more vectors for subsequent cloning and/or expression in a host cell. The polynucleotide(s) can be easily isolated and verified using conventional methods, for example by sequencing. In one embodiment, polynucleotide(s) encoding one or more polypeptide chains of the trispecific antibody of the invention is(are) provided. In yet another embodiment, the present invention provides a vector, preferably an expression vector, comprising one or more polynucleotides of the present invention. Therefore, in one embodiment, the present invention provides a method for producing the trispecific antibody of the present invention, including: culturing the host cell comprising the polynucleotides encoding the polypeptide chains of the trispecific antibody under conditions suitable for expressing the polypeptide chains; and assembling the polypeptide chains to produce the antibody under conditions suitable for assembly of the polypeptide chains into the trispecific antibody.

Expression vectors can be constructed using methods well known to those skilled in the art. Expression vectors include, but are not limited to, viruses, plasmids, cosmids, lambda phages, or yeast artificial chromosomes (YACs).

The antibodies prepared by the methods described herein can be purified by known existing techniques such as high performance liquid chromatography, ion exchange chromatography, gel electrophoresis, affinity chromatography, size exclusion chromatography, and the like. Following purification, the purity of the antibodies of the invention can be determined by any of a number of well-known analytical methods, including size exclusion chromatography, gel electrophoresis, high performance liquid chromatography, and the like. The physical/chemical properties and/or biological activities of the antibodies provided herein can be identified, screened or characterized by a variety of assays known in the art.

### III. Pharmaceutical Compositions, Pharmaceutical Combinations, and Kits

In one aspect, the invention provides a composition, e.g., a pharmaceutical composition, comprising an antibody described herein formulated together with a pharmaceutically acceptable carrier. As used herein, "pharmaceutically acceptable carrier" includes any and all physiologically compatible solvents, dispersion media, isotonic agents and absorption delaying agents, and the like. The pharmaceutical compositions of the present invention are suitable for intravenous, intramuscular, subcutaneous, parenteral, rectal, spinal or epidermal administration (*e.g.*, by injection or infusion). In some embodiments, the antibody of the invention is the sole active ingredient in a pharmaceutical composition. In other embodiments, pharmaceutical compositions may comprise the antibody described herein and more than one therapeutic agent.

In another aspect, the invention also provides a pharmaceutical combination comprising the antibody described herein and one or more therapeutic agents.

The compositions of the present invention may be in a variety of forms. These forms include, for example, liquid, semisolid, and solid dosage forms, such as liquid solutions (*e.g.*, injectable solutions and infusible solutions), dispersions or suspensions, liposomes, and suppositories. The preferred form depends on the intended mode of administration and therapeutic use. Typical preferred compositions are in the form of injectable or infusible solutions.

The pharmaceutical composition of the present invention may contain a "therapeutically effective amount" or a "prophylactically effective amount" of the antibody of the present invention. "Therapeutically effective amount" refers to an amount effective to achieve the desired therapeutic result, at necessary dosages and for periods of time necessary. The therapeutically effective amount may vary according to various factors such as the disease state, age, sex, and weight of the individual, and the like. The therapeutically effective amount is the one in which any toxic or detrimental effects are outweighed by therapeutically beneficial effects. The "therapeutically effective amount" preferably inhibits a measurable parameter (*e.g.,* tumor growth rate, *etc*.) by at least about 20%, more preferably at least about 40%, even more preferably at least about 60%, and still more preferably at least about 80% relative to untreated subjects. The ability of antibodies of the invention to inhibit a measurable parameter (*e.g.*, tumor volume) can be evaluated in an animal model system predictive of efficacy in human tumors. A "prophylactically effective amount" refers to an amount effective, at dosages and for periods of time necessary, to achieve the desired prophylactic result. Typically, because a prophylactic dose is used in subjects prior to or at an earlier stage of disease, the prophylactically effective amount will be less than the therapeutically effective amount.

In another aspect, the invention provides a kit comprising the antibody or composition described herein. The kit may also include one or more other elements, including, for example: instructions for use; other reagents, such as labels or reagents for coupling; pharmaceutically acceptable carriers; and devices or other materials for administration to a subject.

### IV. Uses and treatment methods of the antibodies and compositions of the invention

The trispecific antibodies binding to PD-1/CTLA-4/VEGF of the present invention are suitable for use as anti-tumor, anti-angiogenesis, anti-autoimmune disease, and anti-infection agents. In some embodiments, the trispecific antibodies according to the invention are used for the treatment of cancers, e.g.Melanoma, Non-small cell lung cancer, Renal cell carcinoma, Bladder cancer, Hodgkin's lymphoma, head and neck cancer, ovarian cancer, and brain cancer.

### EXAMPLES

The following examples are intended to illustrate the present invention only and therefore should not be construed as limiting the present invention in any way.

### Example 1. Construction, expression and purification of anti -PD-1/CTLA-4/VEGF trispecific antibodies

### 1.1 Sequence and structure of trispecific antibodies

Trispecific antibodies that can recognize PD-1, CTLA-4 and VEGF were constructed in the application based on anti-PD-1 antibody, anti-CTLA-4 antibody and anti-VEGFA antibody, wherein the anti-PD-1 antibody was the self-developed antibody CQ1-3/1-11; the anti-CTLA-4 antibody was the disclosed nanoantibody 202F1 (Application No. CN202111229808.3, SEQ ID NO: 9) or Ipilimumab, which blocks the binding of CD80 to CTLA-4; the anti-VEGFA antibody was Bevacizumab, which blocks the binding of VEGF to VEGFR-2 (KDR). The variable region sequences of each monoclonal antibody are shown in Table 1. In the trispecific antibody, the anti-PD-1 antibody and the anti-VEGFA antibody can be in the form of Fab or scFv, and the corresponding sequences are shown in Table 1. The antibody fragments can be connected using a linker sequence commonly used in the art, for example, (G₄S)₄ (GGGGSGGGGSGGGGSGGGGS (SEQ ID NO: 9)) linker. The constant region sequence of human hIgG1 or hIgG4 disclosed in the prior art can be used to construct the trispecific antibodies of the present application. For example, the constant region sequence of the trispecific antibodies is the hIgG1 or hIgG4 (S228P) sequence shown in Table 2. The structures and sequences of the constructed trispecific antibodies are shown in Table 3.

**Table 1. Variable region sequences of anti-PD-1 antibody CQ1-3/1-11, anti-CTLA-4 antibody 202F1 and anti-VEGFA antibody Bevacizumab and the sequences of the corresponding ScFv structures (bold indicates CDR sequences determined according to Kabat assignment scheme, and underlines indicate substitutions of corresponding amino acid residues)**

| Antibody Name | Heavy chain variable region | Light chain variable region |
|---|---|---|
| CQ1-3/1-11 | EVQLVESGGGLVQPGGSLRLSCAASG FTFSSYGMSWVRQAPGKGLEWVSTIS GGGRDTYYPDSVKGRFTISRDNSKN TLYLQMNSLRAEDTAVYYCARQNGIT WFADWGQGTLVTVSS (SEQ ID NO:7) | DIQMTQSPSSLSASVGDRVTITC RASESVDNYGISFMNWYQQKP GKAPKLLIYAASNQGSGVPSRFS GSGSGTDFTLTISSLQPEDFATYY CQQSKEVPWTFGGGTKVEIK |
| | CDR1: **SYGMS** (SEQ ID NO:1) | (SEQ ID NO:8) |
| | CDR2: **TISGGGRDTYYPDSVKG** (SEQ ID NO:2) | CDR1: **RASESVDNYGISFM** (SEQ ID NO:4) |
| | CDR3: **QNGITWFAD** (SEQ ID NO:3) | CDR2: **AASNQGS** (SEQ ID NO:5) |
| | | CDR3: **QQSKEVPWT** (SEQ ID NO:6) |
| Description of CQ1-3/1-11-ScFv with modification s: | EVQLVESGGGLVQPGGSLRLSCAASG FTFSSYGMSWVRQAPGKCLEWVSTIS GGGRDTYYPDSVKGRFTISRDNSKN TLYLQMNSLRAEDTAVYYCARQNGIT WFADWGQGTLVTVSSGGGGSGGGGS GGGGSGGGGSDIQMTQSPSSLSASVG DRVTITCRASESVDNYGISFMNWYQ QKPGKAPKLLIYAASNQGSGVPSRFS GSGSGTDFTLTISSLQPEDFATYYCQQ SKEVPWTFGCGTKVEIK (SEQ ID | |
| CQ1-3/1-11-HV(G44C)_( G₄S)₄_CQ1-3/1-11-LV(G 100C) | | |
| | NO:10) | |
| Anti-CTLA-4 Nanobody (202F1) | EVQLLESGGGLVQPGGSLRLSCAASG SIFSINHMAWYRQAPGKQRELVAGVN SRGTTNYVDSVKGRFTISRDNSKNTL YLQMNSLRAEDTAVYYCRALGGAVA AWGQGTLVTVSS (SEQ ID NO:11) | |
| | CDR1: **INHMA** (SEQ ID NO: 12) | |
| | CDR2: **GVNSRGTTNYVDSVKG** (SEQ ID NO:13) | |
| | CDR3: **LGGAVAA** (SEQ ID NO:14) | |
| Anti-CTLA-4 antibody (Ipilimumab) | QVQLVESGGGVVQPGRSLRLSCAASG FTFSSYTMHWVRQAPGKGLEWVTFI SYDGNNKYYADSVKGRFTISRDNSKN TLYLQMNSLRAEDTAIYYCARTGWL GPFDYWGQGTLVTVSS (SEQ ID NO:15) | EIVLTQSPGTLSLSPGERATLSCR ASQSVGSSYLAWYQQKPGQAP RLLIYGAFSRATGIPDRFSGSGSG TDFTLTISRLEPEDFAVYYCQQY GSSPWTFGQGTKVEIK (SEQ ID NO:16) |
| | CDR1: **SYTMH** (SEQ ID NO: 17) | CDR1: **RASQSVGSSYLA** (SEQ ID NO:20) |
| | CDR2: **FISYDGNNKYYADSVK** (SEQ ID NO:18) | CDR2: **GAFSRAT** (SEQ ID NO:21) |
| | CDR3: **TGWLGPFDY** (SEQ ID NO: 19) | CDR3: **QQYGSSP** (SEQ ID NO:22) |
| Description of Ipilimumab -ScFv with modification s | QVQLVESGGGVVQPGRSLRLSCAASG FTFSSYTMHWVRQAPGKCLEWVTFI SYDGNNKYYADSVKGRFTISRDNSKN TLYLQMNSLRAEDTAIYYCARTGWL GPFDYWGQGTLVTVSSGGGGSGGGG SGGGGSGGGGSEIVLTQSPGTLSLSPG ERATLSCRASQSVGSSYLAWYQQKP GQAPRLLIYGAFSRATGIPDRFSGSGS GTDFTLTISRLEPEDFAVYYCQQYGSS PWTFGCGTKVEIK (SEQ ID NO:23) | |
| Ipilimumab-HV (G44C)_(G₄S)₄ _Ipilimumab -LV(Q100C) | | |
| Bevacizumab |  (SEQ ID NO:24) |  (SEQ ID NO:25) |
| | CDR1: **NYGMN** (SEQ ID NO:26) | CDR1: **SASQDISNYLN** (SEQ ID NO:29) |
| | CDR2: **WINTYTGEPTYAADFK** (SEQ ID NO:27) | |
| | | CDR2: **FTSSLHS** (SEQ ID NO:30) |
| | CDR3: **YPHYYGSSHWYFDV** (SEQ ID NO:28) | CDR3: **QQYSTVP** (SEQ ID NO:31) |
| Description of Bevacizumab -ScFv with modification s | EVQLVESGGGLVQPGGSLRLSCAASG YTFTNYGMNWVRQAPGKCLEWVG WINTYTGEPTYAADFKRRFTFSLDTS KSTAYLQMNSLRAEDTAVYYCAKYP HYYGSSHWYFDVWGQGTLVTVSSG GGGSGGGGSGGGGSGGGGSDIQMTQ SPSSLSASVGDRVTITCSASQDISNYL NWYQQKPGKAPKVLIYFTSSLHSGVP SRFSGSGSGTDFTLTISSLQPEDFATYY CQQYSTVPWTFGCGTKVEIK (SEQ ID NO:32) | |
| Bevacizumab -HV(G44C)_ (G₄S)₄ _Bevacizuma b- LV (Q100C) | | |

**Table 2. hIgG1 and hIgG4 constant regions**

| |
|---|
| The heavy chain constant region of hIgG1 antibody |
| |
|  (SEQ ID NO:33) |
| Heavy chain constant region of hIgG4 with modification S228P (named hIgG4-1, underline indicates the substitution of the corresponding amino acid residue) |
| ASTKGPSVFPLAPCSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPAPEFLGGPSVFLFP PKPKDTLMISRTPEVTCVVVDVSQEDPEVQFNWYVDGVEVHNAKTKPREEQFNSTYRVV SVLTVLHQDWLNGKEYKCKVSNKGLPSSIEKTISKAKGQPREPQVYTLPPSQEEMTKNQV SLTCLVKGFYPSDIAVEWESNGQPENNYKTTPPVLDSDGSFFLYSRLTVDKSRWQEGNVFS CSVMHEALHNHYTQKSLSLSLGK (SEQ ID NO:34) |
| Heavy chain constant region of hIgG4 with modifications S228P, K447A (named hIgG4-2, underline indicates the substitution of the corresponding amino acid residues) |
| ASTKGPSVFPLAPCSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPAPEFLGGPSVFLFP PKPKDTLMISRTPEVTCVVVDVSQEDPEVQFNWYVDGVEVHNAKTKPREEQFNSTYRVV SVLTVLHQDWLNGKEYKCKVSNKGLPSSIEKTISKAKGQPREPQVYTLPPSQEEMTKNQV SLTCLVKGFYPSDIAVEWESNGQPENNYKTTPPVLDSDGSFFLYSRLTVDKSRWQEGNVFS CSVMHEALHNHYTQKSLSLSLGA (SEQ ID NO:35) |
| Light chain constant region (kappa chain) |
|  (SEQ ID NO:36) |

**Table 3. Structure and sequence of trispecific antibodies (underline indicates substitution of corresponding amino acid residues)**

| Antibody name | Heavy chain sequence | Light chain sequence |
|---|---|---|
| F6 | EVQLVESGGGLVQPGGSLRLSCAASGFTFSSYGM SWVRQAPGKGLEWVSTISGGGRDTYYPDSVKG RFTISRDNSKNTLYLQMNSLRAEDTAVYYCARQN GITWFADWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV | DIQMTQSPSSLSASVGD RVTITCRASESVDNYGI SFMNWYQQKPGKAPK LLIYAASNQGSGVPSRF SGSGSGTDFTLTISSLQP EDFATYYCQQSKEVP WTFGGGTKVEIKRTVA |
| | FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI AVEWESNGQPENNYKTTPPVLDSDGSFFLYSRLT VDKSRWQEGNVFSCSVMHEALHNHYTQKSLSLS LGA GGGGSGGGGSGGGGSGGGGS EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS GGGGSGGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:37) | APSVFIFPPSDEQLKSG TASVVCLLNNFYPREA KVQWKVDNALQSGNS QESVTEQDSKDSTYSLS STLTLSKADYEKHKVY ACEVTHQGLSSPVTKS FNRGEC (SEQ ID NO:38) |
| F7 | EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK GGGGS GGGGSGGGGSGGGGS EVQLVESGGGLVQPGGSLRLSCAASGFTFSSYGM SWVRQAPGKGLEWVSTISGGGRDTYYPDSVKG RFTISRDNSKNTLYLQMNSLRAEDTAVYYCARQN GITWFADWGQGTLVTVSSASTKGPSVFPLAPCSR | DIQMTQSPSSLSASVGD RVTITCRASESVDNYGI SFMNWYQQKPGKAPK LLIYAASNQGSGVPSRF SGSGSGTDFTLTISSLQP EDFATYYCQQSKEVP WTFGGGTKVEIKRTVA APSVFIFPPSDEQLKSGT ASVVCLLNNFYPREAK VQWKVDNALQSGNSQ ESVTEQDSKDSTYSLSS TLTLSKADYEKHKVYA CEVTHQGLSSPVTKSF |
| | STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI AVEWESNGQPENNYKTTPPVLDSDGSFFLYSRLT VDKSRWQEGNVFSCSVMHEALHNHYTQKSLSLS LGA GGGGS GGGGSGGGGSGGGGS EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS (SEQ ID NO:39) | NRGEC (SEQ ID NO:40) |
| F8 | EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV PWTFGCGTKVEIK GGGGS GGGGSGGGGSGGGGS EVQLVESGGGLVQPGGSLRLSCAASGYTFTNYG MNWVRQAPGKGLEWVGWINTYTGEPTYAADF KRRFTFSLDTSKSTAYLQMNSLRAEDTAVYYCAK YPHYYGSSHWYFDVWGQGTLVTVSSASTKGPSV FPLAPCSRSTSESTAALGCLVKDYFPEPVTVSWNS GALTSGVHTFPAVLQSSGLYSLSSVVTVPSSSLGT KTYTCNVDHKPSNTKVDKRVESKYGPPCPPCPAP EFLGGPSVFLFPPKPKDTLMISRTPEVTCVVVDVS QEDPEVQFNWYVDGVEVHNAKTKPREEQFNSTY RVVSVLTVLHQDWLNGKEYKCKVSNKGLPSSIEK | DIQMTQSPSSLSASVGD RVTITCSASQDISNYLN WYQQKPGKAPKVLIYF TSSLHSGVPSRFSGSGS GTDFTLTISSLQPEDFA TYYCQQYSTVPWTFG QGTKVEIKRTVAAPSV FIFPPSDEQLKSGTASV VCLLNNFYPREAKVQ WKVDNALQSGNSQES VTEQDSKDSTYSLSSTL TLSKADYEKHKVYACE VTHQGLSSPVTKSFNR GEC (SEQ ID NO:42) |
| | TISKAKGQPREPQVYTLPPSQEEMTKNQVSLTCLV KGFYPSDIAVEWESNGQPENNYKTTPPVLDSDGS FFLYSRLTVDKSRWQEGNVFSCSVMHEALHNHY TQKSLSLSLGA GGGGS GGGGSGGGGSGGGGS EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS (SEQ ID NO:41) | |
| F9 | EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS GGGGS GGGGSGGGGSGGGGS EVQLVESGGGLVQPGGSLRLSCAASGFTFSSYGM SWVRQAPGKGLEWVSTISGGGRDTYYPDSVKG RFTISRDNSKNTLYLQMNSLRAEDTAVYYCARQN GITWFADWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI AVEWESNGQPENNYKTTPPVLDSDGSFFLYSRLT VDKSRWQEGNVFSCSVMHEALHNHYTQKSLSLS LGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT | DIQMTQSPSSLSASVGD RVTITCRASESVDNYGI SFMNWYQQKPGKAPK LLIYAASNQGSGVPSRF SGSGSGTDFTLTISSLQP EDFATYYCQQSKEVP WTFGGGTKVEIKRTVA APSVFIFPPSDEQLKSGT ASVVCLLNNFYPREAK VQWKVDNALQSGNSQ ESVTEQDSKDSTYSLSS TLTLSKADYEKHKVYA CEVTHQGLSSPVTKSF NRGEC (SEQ ID NO:44) |
| | CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:43) | |
| F10 | EVQLVESGGGLVQPGGSLRLSCAASGFTFSSYGM SWVRQAPGKGLEWVSTISGGGRDTYYPDSVKG RFTISRDNSKNTLYLQMNSLRAEDTAVYYCARQN GITWFADWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI AVEWESNGQPENNYKTTPPVLDSDGSFFLYSRLT VDKSRWQEGNVFSCSVMHEALHNHYTQKSLSLS LGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK GGGGS GGGGSGGGGSGGGGS EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS (SEQ ID NO:45) | DIQMTQSPSSLSASVGD RVTITCRASESVDNYGI SFMNWYQQKPGKAPK LLIYAASNQGSGVPSRF SGSGSGTDFTLTISSLQP EDFATYYCQQSKEVP WTFGGGTKVEIKRTVA APSVFIFPPSDEQLKSGT ASVVCLLNNFYPREAK VQWKVDNALQSGNSQ ESVTEQDSKDSTYSLSS TLTLSKADYEKHKVYA CEVTHQGLSSPVTKSF NRGEC (SEQ ID NO:46) |
| F11 | EVQLVESGGGLVQPGGSLRLSCAASGFTFSSYGM SWVRQAPGKGLEWVSTISGGGRDTYYPDSVKG RFTISRDNSKNTLYLQMNSLRAEDTAVYYCARQN | DIQMTQSPSSLSASVGD RVTITCRASESVDNYGI SFMNWYQQKPGKAPK |
| | GITWFADWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI AVEWESNGQPENNYKTTPPVLDSDGSFFLYSRLT VDKSRWQEGNVFSCSVMHEALHNHYTQKSLSLS LGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:47) | LLIYAASNQGSGVPSRF SGSGSGTDFTLTISSLQP EDFATYYCQQSKEVP WTFGGGTKVEIKRTVA APSVFIFPPSDEQLKSGT ASVVCLLNNFYPREAK VQWKVDNALQSGNSQ ESVTEQDSKDSTYSLSS TLTLSKADYEKHKVYA CEVTHQGLSSPVTKSF NRGECGGGGS GGGGSGGGGSGGGGS EVQLLESGGGLVQPGG SLRLSCAASGSIFSINH MAWYRQAPGKQRELV AGVNSRGTTNYVDSV KGRFTISRDNSKNTLY LQMNSLRAEDTAVYY CRALGGAVAAWGQGT LVTVSS (SEQ ID NO:48) |
| F12 | EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS GGGGS GGGGSGGGGSGGGGS EVQLVESGGGLVQPGGSLRLSCAASGYTFTNYG MNWVRQAPGKGLEWVGWINTYTGEPTYAADF KRRFTFSLDTSKSTAYLQMNSLRAEDTAVYYCAK YPHYYGSSHWYFDVWGQGTLVTVSSASTKGPSV FPLAPCSRSTSESTAALGCLVKDYFPEPVTVSWNS GALTSGVHTFPAVLQSSGLYSLSSVVTVPSSSLGT KTYTCNVDHKPSNTKVDKRVESKYGPPCPPCPAP | DIQMTQSPSSLSASVGD RVTITCSASQDISNYLN WYQQKPGKAPKVLIYF TSSLHSGVPSRFSGSGS GTDFTLTISSLQPEDFA TYYCQQYSTVPWTFG QGTKVEIKRTVAAPSV FIFPPSDEQLKSGTASV VCLLNNFYPREAKVQ WKVDNALQSGNSQES VTEQDSKDSTYSLSSTL TLSKADYEKHKVYACE |
| | EFLGGPSVFLFPPKPKDTLMISRTPEVTCVVVDVS QEDPEVQFNWYVDGVEVHNAKTKPREEQFNSTY RVVSVLTVLHQDWLNGKEYKCKVSNKGLPSSIEK TISKAKGQPREPQVYTLPPSQEEMTKNQVSLTCLV KGFYPSDIAVEWESNGQPENNYKTTPPVLDSDGS FFLYSRLTVDKSRWQEGNVFSCSVMHEALHNHY TQKSLSLSLGA GGGGS GGGGSGGGGSGGGGS EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV PWTFGCGTKVEIK (SEQ ID NO:49) | VTHQGLSSPVTKSFNR GEC (SEQ ID NO:50) |
| F13 | EVQLVESGGGLVQPGGSLRLSCAASGFTFSSYGM SWVRQAPGKGLEWVSTISGGGRDTYYPDSVKG RFTISRDNSKNTLYLQMNSLRAEDTAVYYCARQN GITWFADWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPCQEEMTKNQVSLWCLVKGFYPSD IAVEWESNGQPENNYKTTPPVLDSDGSFFLYSRLT VDKSRWQEGNVFSCSVMHEALHNHYTQKSLSLS LGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK | DIQMTQSPSSLSASVGD RVTITCRASESVDNYGI SFMNWYQQKPGKAPK LLIYAASNQGSGVPSRF SGSGSGTDFTLTISSLQP EDFATYYCQQSKEVP WTFGGGTKVEIKRTVA APSVFIFPPSDEQLKSGT ASVVCLLNNFYPREAK VQWKVDNALQSGNSQ ESVTEQDSKDSTYSLSS TLTLSKADYEKHKVYA CEVTHQGLSSPVTKSF NRGEC (SEQ ID NO:52) |
| | YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:51) | |
| | EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS GGGGS GGGGSGGGGSGGGGS ESKYGPPCPPCPAPEFLGGPSVFLFPPKPKDTLMIS RTPEVTCVVVDVSQEDPEVQFNWYVDGVEVHNA KTKPREEQFNSTYRVVSVLTVLHQDWLNGKEYK CKVSNKGLPSSIEKTISKAKGQPREPQVCTLPPSQE EMTKNQVSLSCAVKGFYPSDIAVEWESNGQPENN YKTTPPVLDSDGSFFLVSRLTVDKSRWQEGNVFS CSVMHEALHNRFTQKSLSLSLGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:53) | |
| F14 | EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV PWTFGCGTKVEIK | None |
| | GGGGS GGGGSGGGGSGGGGS ESKYGPPCPPCPAPEFLGGPSVFLFPPKPKDTLMIS RTPEVTCVVVDVSQEDPEVQFNWYVDGVEVHNA KTKPREEQFNSTYRVVSVLTVLHQDWLNGKEYK CKVSNKGLPSSIEKTISKAKGQPREPQVYTLPPCQ EEMTKNQVSLWCLVKGFYPSDIAVEWESNGQPE NNYKTTPPVLDSDGSFFLYSRLTVDKSRWQEGNV FSCSVMHEALHNHYTQKSLSLSLGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:54) | |
| | EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS GGGGS GGGGSGGGGSGGGGS ESKYGPPCPPCPAPEFLGGPSVFLFPPKPKDTLMIS RTPEVTCVVVDVSQEDPEVQFNWYVDGVEVHNA KTKPREEQFNSTYRVVSVLTVLHQDWLNGKEYK CKVSNKGLPSSIEKTISKAKGQPREPQVCTLPPSQE EMTKNQVSLSCAVKGFYPSDIAVEWESNGQPENN YKTTPPVLDSDGSFFLVSRLTVDKSRWQEGNVFS CSVMHEALHNRFTQKSLSLSLGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG | |
| | GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:55) | |
| F15 | QVQLVESGGGVVQPGRSLRLSCAASGFTFSSYTM HWVRQAPGKGLEWVTFISYDGNNKYYADSVKG RFTISRDNSKNTLYLQMNSLRAEDTAIYYCARTG WLGPFDYWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPCQEEMTKNQVSLWCLVKGFYPSD IAVEWESNGQPENNYKTTPPVLDSDGSFFLYSRLT VDKSRWQEGNVFSCSVMHEALHNHYTQKSLSLS LGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:56) | EIVLTQSPGTLSLSPGE RATLSCRASQSVGSSY LAWYQQKPGQAPRLLI YGAFSRATGIPDRFSGS GSGTDFTLTISRLEPED FAVYYCQQYGSSPWT FGQGTKVEIKRTVAAP SVFIFPPSDEQLKSGTA SVVCLLNNFYPREAKV QWKVDNALQSGNSQE SVTEQDSKDSTYSLSST LTLSKADYEKHKVYAC EVTHQGLSSPVTKSFN RGEC (SEQ ID NO:57) |
| | EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV | |
| | PWTFGCGTKVEIK GGGGS GGGGSGGGGSGGGGS ESKYGPPCPPCPAPEFLGGPSVFLFPPKPKDTLMIS RTPEVTCVVVDVSQEDPEVQFNWYVDGVEVHNA KTKPREEQFNSTYRVVSVLTVLHQDWLNGKEYK CKVSNKGLPSSIEKTISKAKGQPREPQVCTLPPSQE EMTKNQVSLSCAVKGFYPSDIAVEWESNGQPENN YKTTPPVLDSDGSFFLVSRLTVDKSRWQEGNVFS CSVMHEALHNRFTQKSLSLSLGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:58) | |
| F16 | EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK GGGGS GGGGSGGGGSGGGGS EVQLVESGGGLVQPGGSLRLSCAASGFTFSSYGM SWVRQAPGKGLEWVSTISGGGRDTYYPDSVKG RFTISRDNSKNTLYLQMNSLRAEDTAVYYCARQN GITWFADWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV | DIQMTQSPSSLSASVGD RVTITCRASESVDNYGI SFMNWYQQKPGKAPK LLIYAASNQGSGVPSRF SGSGSGTDFTLTISSLQP EDFATYYCQQSKEVP WTFGGGTKVEIKRTVA APSVFIFPPSDEQLKSGT ASVVCLLNNFYPREAK VQWKVDNALQSGNSQ ESVTEQDSKDSTYSLSS TLTLSKADYEKHKVYA CEVTHQGLSSPVTKSF NRGEC (SEQ ID NO:60) |
| | FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI AVEWESNGQPENNYKTTPPVLDSDGSFFLYSRLT VDKSRWQEGNVFSCSVMHEALHNHYTQKSLSLS LGA GGGGS GGGGSGGGGSGGGGS QVQLVESGGGVVQPGRSLRLSCAASGFTFSSYTM HWVRQAPGKCLEWVTFISYDGNNKYYADSVKG RFTISRDNSKNTLYLQMNSLRAEDTAIYYCARTG WLGPFDYWGQGTLVTVSSGGGGSGGGGSGGGG SGGGGSEIVLTQSPGTLSLSPGERATLSCRASQSV GSSYLAWYQQKPGQAPRLLIYGAFSRATGIPDRF SGSGSGTDFTLTISRLEPEDFAVYYCQQYGSSPW TFGCGTKVEIK (SEQ ID NO:59) | |
| F17 | EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV PWTFGCGTKVEIK GGGGS GGGGSGGGGSGGGGS EVQLVESGGGLVQPGGSLRLSCAASGYTFTNYG MNWVRQAPGKGLEWVGWINTYTGEPTYAADF KRRFTFSLDTSKSTAYLQMNSLRAEDTAVYYCAK YPHYYGSSHWYFDVWGQGTLVTVSSASTKGPSV FPLAPCSRSTSESTAALGCLVKDYFPEPVTVSWNS GALTSGVHTFPAVLQSSGLYSLSSVVTVPSSSLGT KTYTCNVDHKPSNTKVDKRVESKYGPPCPPCPAP EFLGGPSVFLFPPKPKDTLMISRTPEVTCVVVDVS QEDPEVQFNWYVDGVEVHNAKTKPREEQFNSTY | DIQMTQSPSSLSASVGD RVTITCSASQDISNYLN WYQQKPGKAPKVLIYF TSSLHSGVPSRFSGSGS GTDFTLTISSLQPEDFA TYYCQQYSTVPWTFG QGTKVEIKRTVAAPSV FIFPPSDEQLKSGTASV VCLLNNFYPREAKVQ WKVDNALQSGNSQES VTEQDSKDSTYSLSSTL TLSKADYEKHKVYACE VTHQGLSSPVTKSFNR GEC (SEQ ID NO:62) |
| | RVVSVLTVLHQDWLNGKEYKCKVSNKGLPSSIEK TISKAKGQPREPQVYTLPPSQEEMTKNQVSLTCLV KGFYPSDIAVEWESNGQPENNYKTTPPVLDSDGS FFLYSRLTVDKSRWQEGNVFSCSVMHEALHNHY TQKSLSLSLGA GGGGS GGGGSGGGGSGGGGS QVQLVESGGGVVQPGRSLRLSCAASGFTFSSYTM HWVRQAPGKCLEWVTFISYDGNNKYYADSVKG RFTISRDNSKNTLYLQMNSLRAEDTAIYYCARTG WLGPFDYWGQGTLVTVSSGGGGSGGGGSGGGG SGGGGSEIVLTQSPGTLSLSPGERATLSCRASQSV GSSYLAWYQQKPGQAPRLLIYGAFSRATGIPDRF SGSGSGTDFTLTISRLEPEDFAVYYCQQYGSSPW TFGCGTKVEIK (SEQ ID NO:61) | |
| F18 | EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV PWTFGCGTKVEIK GGGGS GGGGSGGGGSGGGGS QVQLVESGGGVVQPGRSLRLSCAASGFTFSSYTM HWVRQAPGKGLEWVTFISYDGNNKYYADSVKG RFTISRDNSKNTLYLQMNSLRAEDTAIYYCARTG WLGPFDYWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI | EIVLTQSPGTLSLSPGE RATLSCRASQSVGSSY LAWYQQKPGQAPRLLI YGAFSRATGIPDRFSGS GSGTDFTLTISRLEPED FAVYYCQQYGSSPWT FGQGTKVEIKRTVAAP SVFIFPPSDEQLKSGTA SVVCLLNNFYPREAKV QWKVDNALQSGNSQE SVTEQDSKDSTYSLSST LTLSKADYEKHKVYAC EVTHQGLSSPVTKSFN RGEC (SEQ ID NO:64) |
| | AVEWESNGQPENNYKTTPPVLDSDGSFFLYSRLT VDKSRWQEGNVFSCSVMHEALHNHYTQKSLSLS LGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGCGTKVEIK (SEQ ID NO:63) | |
| F19 | EVQLVESGGGLVQPGGSLRLSCAASGFTFSSYGM SWVRQAPGKGLEWVSTISGGGRDTYYPDSVKG RFTISRDNSKNTLYLQMNSLRAEDTAVYYCARQN GITWFADWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI AVEWESNGQPENNYKTTPPVLDSDGSFFLYSRLT VDKSRWQEGNVFSCSVMHEALHNHYTQKSLSLS LGA GGGGS GGGGSGGGGSGGGGS EVQLVESGGGLVQPGGSLRLSCAASGYTFTNYG MNWVRQAPGKCLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY | EVQLLESGGGLVQPGG SLRLSCAASGSIFSINH MAWYRQAPGKQRELV AGVNSRGTTNYVDSV KGRFTISRDNSKNTLY LQMNSLRAEDTAVYY CRALGGAVAAWGQGT LVTVSS GGGGS GGGGSGGGGSGGGGS DIQMTQSPSSLSASVGD RVTITCRASESVDNYGI SFMNWYQQKPGKAPK LLIYAASNQGSGVPSRF SGSGSGTDFTLTISSLQP EDFATYYCQQSKEVP WTFGGGTKVEIKRTVA APSVFIFPPSDEQLKSGT ASVVCLLNNFYPREAK VQWKVDNALQSGNSQ ESVTEQDSKDSTYSLSS TLTLSKADYEKHKVYA CEVTHQGLSSPVTKSF |
| | STVPWTFGCGTKVEIK (SEQ ID NO:65) | NRGEC (SEQ ID NO:66) |
| F20 | EVQLVESGGGLVQPGGSLRLSCAASGYTFTNYG MNWVRQAPGKGLEWVGWINTYTGEPTYAADF KRRFTFSLDTSKSTAYLQMNSLRAEDTAVYYCAK YPHYYGSSHWYFDVWGQGTLVTVSSASTKGPSV FPLAPCSRSTSESTAALGCLVKDYFPEPVTVSWNS GALTSGVHTFPAVLQSSGLYSLSSVVTVPSSSLGT KTYTCNVDHKPSNTKVDKRVESKYGPPCPPCPAP EFLGGPSVFLFPPKPKDTLMISRTPEVTCVVVDVS QEDPEVQFNWYVDGVEVHNAKTKPREEQFNSTY RVVSVLTVLHQDWLNGKEYKCKVSNKGLPSSIEK TISKAKGQPREPQVYTLPPSQEEMTKNQVSLTCLV KGFYPSDIAVEWESNGQPENNYKTTPPVLDSDGS FFLYSRLTVDKSRWQEGNVFSCSVMHEALHNHY TQKSLSLSLGA GGGGS GGGGSGGGGSGGGGS EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV PWTFGCGTKVEIK (SEQ ID NO:67) | EVQLLESGGGLVQPGG SLRLSCAASGSIFSINH MAWYRQAPGKQRELV AGVNSRGTTNYVDSV KGRFTISRDNSKNTLY LQMNSLRAEDTAVYY CRALGGAVAAWGQGT LVTVSS GGGGS GGGGSGGGGSGGGGS DIQMTQSPSSLSASVGD RVTITCSASQDISNYLN WYQQKPGKAPKVLIYF TSSLHSGVPSRFSGSGS GTDFTLTISSLQPEDFA TYYCQQYSTVPWTFG QGTKVEIKRTVAAPSV FIFPPSDEQLKSGTASV VCLLNNFYPREAKVQ WKVDNALQSGNSQES VTEQDSKDSTYSLSSTL TLSKADYEKHKVYACE VTHQGLSSPVTKSFNR GEC (SEQ ID NO:68) |
| F21 | EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS GGGGS GGGGSGGGGSGGGGS EVQLVESGGGLVQPGGSLRLSCAASGFTFSSYGM SWVRQAPGKGLEWVSTISGGGRDTYYPDSVKG | DIQMTQSPSSLSASVGD RVTITCRASESVDNYGI SFMNWYQQKPGKAPK LLIYAASNQGSGVPSRF SGSGSGTDFTLTISSLQP EDFATYYCQQSKEVP WTFGGGTKVEIKRTVA |
| | RFTISRDNSKNTLYLQMNSLRAEDTAVYYCARQN GITWFADWGQGTLVTVSSASTKGPSVFPLAPCSR STSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI AVEWESNGQPENNYKTTPPVLDSDGSFFLYSRLT VDKSRWQEGNVFSCSVMHEALHNHYTQKSLSLS LGA GGGGS GGGGSGGGGSGGGGS EVQLVESGGGLVQPGGSLRLSCAASGYTFTNYG MNWVRQAPGKGLEWVGWINTYTGEPTYAADF KRRFTFSLDTSKSTAYLQMNSLRAEDTAVYYCAK YPHYYGSSHWYFDVWGQGTLVTVSSGGGGSGG GGSGGGGSGGGGSDIQMTQSPSSLSASVGDRVTIT CSASQDISNYLNWYQQKPGKAPKVLIYFTSSLHS GVPSRFSGSGSGTDFTLTISSLQPEDFATYYCQQY STVPWTFGQGTKVEIK (SEQ ID NO:69) | APSVFIFPPSDEQLKSGT ASVVCLLNNFYPREAK VQWKVDNALQSGNSQ ESVTEQDSKDSTYSLSS TLTLSKADYEKHKVYA CEVTHQGLSSPVTKSF NRGEC (SEQ ID NO:70) |
| F22 | EVQLVESGGGLVQPGGSLRLSCAASGYTFTNYG MNWVRQAPGKGLEWVGWINTYTGEPTYAADF KRRFTFSLDTSKSTAYLQMNSLRAEDTAVYYCAK YPHYYGSSHWYFDVWGQGTLVTVSSASTKGPSV FPLAPCSRSTSESTAALGCLVKDYFPEPVTVSWNS GALTSGVHTFPAVLQSSGLYSLSSVVTVPSSSLGT KTYTCNVDHKPSNTKVDKRVESKYGPPCPPCPAP EFLGGPSVFLFPPKPKDTLMISRTPEVTCVVVDVS QEDPEVQFNWYVDGVEVHNAKTKPREEQFNSTY RVVSVLTVLHQDWLNGKEYKCKVSNKGLPSSIEK TISKAKGQPREPQVYTLPPSQEEMTKNQVSLTCLV KGFYPSDIAVEWESNGQPENNYKTTPPVLDSDGS FFLYSRLTVDKSRWQEGNVFSCSVMHEALHNHY | DIQMTQSPSSLSASVGD RVTITCSASQDISNYLN WYQQKPGKAPKVLIYF TSSLHSGVPSRFSGSGS GTDFTLTISSLQPEDFA TYYCQQYSTVPWTFG QGTKVEIKRTVAAPSV FIFPPSDEQLKSGTASV VCLLNNFYPREAKVQ WKVDNALQSGNSQES VTEQDSKDSTYSLSSTL TLSKADYEKHKVYACE VTHQGLSSPVTKSFNR |
| | TQKSLSLSLGA GGGGS GGGGSGGGGSGGGGS EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS GGGGS GGGGSGGGGSGGGGS EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV PWTFGCGTKVEIK (SEQ ID NO:71) | GEC (SEQ ID NO:72) |
| F23 | EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV PWTFGCGTKVEIK GGGGS GGGGSGGGGSGGGGS EVQLLESGGGLVQPGGSLRLSCAASGSIFSINHMA WYRQAPGKQRELVAGVNSRGTTNYVDSVKGRF TISRDNSKNTLYLQMNSLRAEDTAVYYCRALGG AVAAWGQGTLVTVSS GGGGS GGGGSGGGGSGGGGS EVQLVESGGGLVQPGGSLRLSCAASGYTFTNYG MNWVRQAPGKGLEWVGWINTYTGEPTYAADF KRRFTFSLDTSKSTAYLQMNSLRAEDTAVYYCAK YPHYYGSSHWYFDVWGQGTLVTVSSASTKGPSV FPLAPCSRSTSESTAALGCLVKDYFPEPVTVSWNS | DIQMTQSPSSLSASVGD RVTITCSASQDISNYLN WYQQKPGKAPKVLIYF TSSLHSGVPSRFSGSGS GTDFTLTISSLQPEDFA TYYCQQYSTVPWTFG QGTKVEIKRTVAAPSV FIFPPSDEQLKSGTASV VCLLNNFYPREAKVQ WKVDNALQSGNSQES VTEQDSKDSTYSLSSTL TLSKADYEKHKVYACE VTHQGLSSPVTKSFNR GEC (SEQ ID NO:74) |
| | GALTSGVHTFPAVLQSSGLYSLSSVVTVPSSSLGT KTYTCNVDHKPSNTKVDKRVESKYGPPCPPCPAP EFLGGPSVFLFPPKPKDTLMISRTPEVTCVVVDVS QEDPEVQFNWYVDGVEVHNAKTKPREEQFNSTY RVVSVLTVLHQDWLNGKEYKCKVSNKGLPSSIEK TISKAKGQPREPQVYTLPPSQEEMTKNQVSLTCLV KGFYPSDIAVEWESNGQPENNYKTTPPVLDSDGS FFLYSRLTVDKSRWQEGNVFSCSVMHEALHNHY TQKSLSLSLGA (SEQ ID NO:73) | |
| F24 | EVQLVESGGGLVQPGGSLRLSCAASGYTFTNYG MNWVRQAPGKGLEWVGWINTYTGEPTYAADF KRRFTFSLDTSKSTAYLQMNSLRAEDTAVYYCAK YPHYYGSSHWYFDVWGQGTLVTVSSASTKGPSV FPLAPCSRSTSESTAALGCLVKDYFPEPVTVSWNS GALTSGVHTFPAVLQSSGLYSLSSVVTVPSSSLGT KTYTCNVDHKPSNTKVDKRVESKYGPPCPPCPAP EFLGGPSVFLFPPKPKDTLMISRTPEVTCVVVDVS QEDPEVQFNWYVDGVEVHNAKTKPREEQFNSTY RVVSVLTVLHQDWLNGKEYKCKVSNKGLPSSIEK TISKAKGQPREPQVYTLPPSQEEMTKNQVSLTCLV KGFYPSDIAVEWESNGQPENNYKTTPPVLDSDGS FFLYSRLTVDKSRWQEGNVFSCSVMHEALHNHY TQKSLSLSLGA GGGGS GGGGSGGGGSGGGGS EVQLVESGGGLVQPGGSLRLSCAASGFTFSSYGM SWVRQAPGKCLEWVSTISGGGRDTYYPDSVKG RFTISRDNSKNTLYLQMNSLRAEDTAVYYCARQN GITWFADWGQGTLVTVSSGGGGSGGGGSGGGGS GGGGSDIQMTQSPSSLSASVGDRVTITCRASESVD NYGISFMNWYQQKPGKAPKLLIYAASNQGSGVP SRFSGSGSGTDFTLTISSLQPEDFATYYCQQSKEV PWTFGCGTKVEIK GGGGS GGGGSGGGGSGGGGS EVQLLESGGGLVQPGGSLRLSCAASGSIFSINHMA | DIQMTQSPSSLSASVGD RVTITCSASQDISNYLN WYQQKPGKAPKVLIYF TSSLHSGVPSRFSGSGS GTDFTLTISSLQPEDFA TYYCQQYSTVPWTFG QGTKVEIKRTVAAPSV FIFPPSDEQLKSGTASV VCLLNNFYPREAKVQ WKVDNALQSGNSQES VTEQDSKDSTYSLSSTL TLSKADYEKHKVYACE VTHQGLSSPVTKSFNR GEC (SEQ ID NO:76) |
| | WYRQAPGKQRELVAGVNSRGTTNYVDSVKGRF TISRDNSKNTLYLQMNSLRAEDTAVYYCRALGG AVAAWGQGTLVTVSS (SEQ ID NO:75) | |

### 1.2 Expression and purification of trispecific antibodies

According to the sequences of the trispecific antibodies in Table 3, polynucleotides with the corresponding sequences were synthesized and inserted into the expression vector pCDNA3.1. After confirmed correct by sequencing, expression was performed. The specific protocol was as follows: 200 mL of expiCHO cells (Gibco, Catalog No.: A29129) with a cell density of 3×10⁶ cells/mL were co-transfected with 100 µg of each of the light chain and heavy chain expression plasmids encoding the corresponding trispecific antibodies shown in Table 3, and cultured at 37°C in a carbon dioxide incubator for 7 days. The supernatant was collected by centrifugation and filtered with a 0.22 µM filter membrane. The filtered supernatant was transferred to a protein A chromatography column containing 2 mL of protein A chromatography packing MabSelect PrismA (cytiva, Catalog No.: 17549802) for affinity chromatography, the column is pre-equilibrated with 5 column volumes of PBS solution. The sample retention time was at least 2 minutes, and then the column was washed with 10-15 column volumes of PBS solution to remove non-specifically adsorbed impurities, and the target protein was eluted with 4 mL of 20 mM Na-Citrate, pH 3.2 eluting solution, and finally the pH of the eluent was adjusted to 5.0-6.0 with 1M Tris. After centrifugal filtration, the protein concentration was detected by UV spectrophotometer, the relative expression level was calculated according to the concentration, and the protein purity was detected by size exclusion chromatography combined with high performance liquid chromatography (Dionex, Ultimate 3000). The corresponding expression levels and purities of each trispecific antibody detected are shown in Table 4.

The control antibodies used in this application are constructed as follows and expressed and purified accordingly based on the above method. Adjusting the corresponding parameters of specific expression and purification for specific antibodies belongs to the routine knowledge of those skilled in the art:
The heavy chain variable region of the anti-PD-1 antibody (CQ1-3/1-11) shown in Table 1 was fused with the hIgG4-1 constant region sequence shown in Table 2 to obtain a full-length heavy chain; and the light chain variable region of CQ1-3/1-11 was fused with the light chain constant region sequence shown in Table 2 to obtain a full-length light chain. The full-length antibody thus obtained was named CQ1-3/1-11-hIgG4 antibody, or CQ1-3/1-11 for short.

The heavy chain variable region of the anti-CTLA-4 antibody (202F1) shown in Table 1 was fused with the hIgG4-1 constant region sequence shown in Table 2 to obtain a full-length heavy chain; and the light chain variable region of 202F1 was fused with the light chain constant region sequence shown in Table 2 to obtain a full-length light chain. The full-length antibody thus obtained was named 202F1-hIgG4 antibody.

The heavy chain variable region of antibody 202F1 shown in Table 1 was fused with the hIgG1 constant region sequence shown in Table 2, and assembled with the corresponding full-length light chain described above to obtain a full-length antibody named 202F1-hIgG1.

The variable region of Ipilimumab shown in Table 1 was fused with the hIgG1 constant region sequence shown in Table 2 and assembled with the corresponding full-length light chain to obtain Ipilimumab.

The variable region of Bevacizumab shown in Table 1 was fused with the hIgG1 constant region sequence shown in Table 2 and assembled with the corresponding full-length light chain to obtain Bevacizumab.

Regarding the corresponding scFv of each antibody used in the present application, the corresponding expression and purification were carried out according to the above method according to the sequence disclosed in Table 1. It is within the scope of routine knowledge of those skilled in the art to adjust the corresponding parameters of specific expression and purification for specific antibodies.

**Table 4 Expression level and purity of trispecific antibodies**

| Antibody Name | Expression level, mg/100mL | Purity of main peak detected by SEC, % |
|---|---|---|
| H010-F6 | 1.28 | 91.77 |
| H010-F7 | 1.74 | 87.53 |
| H010-F8 | 3.42 | 88.71 |
| H010-F9 | 2.28 | 92.99 |
| H010-F10 | 1.62 | 86.02 |
| H010-F11 | 2.14 | 83.59 |
| H010-F12 | 3.10 | 80.76 |
| H010-F13 | 1.24 | 82.48 |
| H010-F14 | 0.90 | 85.17 |
| H010-F15 | 0.94 | 96.04 |
| H010-F16 | 0.82 | 62.96 |
| H010-F17 | 1.66 | 65.61 |
| H010-F18 | 0.56 | 80.80 |
| H010-F19 | 3.28 | 39.91 |
| H010-F20 | 4.34 | 55.50 |
| H010-F21 | 0.26 | 57.60 |
| H010-F22 | 2.62 | 82.06 |
| H010-F23 | 3.50 | 96.93 |
| H010-F24 | 2.88 | 95.42 |

### Example 2. Analysis of binding activity and physicochemical properties of anti-PD-1/CTLA-4/VEGF trispecific antibodies

### 2.1 Activity of trispecific antibodies binding to PD-1

Enzyme-linked immunosorbent assay (ELISA) and flow cytometry (FACS) were used to detect the activity of the trispecific antibodies binding to PD-1 protein and to PD-1 antigen on the cell surface, respectively.

ELISA was used to determine the activity of the trispecific antibodies binding to the PD-1 protein: 1 µg/mL human PD-1 protein (Acro, Catalog No.: PD1-H5221) was used to coat a high-adsorption 96-well ELISA plate and incubated at 4°C overnight. On the next day, the plate was washed three times with PBST solution and blocked with 1% BSA-PBS solution at 37°C for 1 hour. At the same time, 3-fold gradient dilutions of the antibody were prepared with 1% BSA-PBS solution, with a starting concentration of 10 nM. The antibodies of each gradient concentration were added to the blocked 96-well ELISA plate at 100 µL/well in triplicate and incubated at 37°C for 1 hour. The plate was then washed three times with 0.05% PBST solution, and 100 µL/well of HRP-labeled secondary antibody (Sigma, Catalog No.: A0170) diluted at 1:5000 was added to the ELISA plate and incubated at 37°C for 1 hour. The plate was washed three times with 0.05% PBST solution and developed with TMB. The absorbance of the 96-well ELISA plate at 450 nm was detected by an ELISA reader. The OD₄₅₀ value was fitted using a four-parameter model to calculate the EC₅₀ value of each antibody binding to PD-1. The results are shown in Table 5.

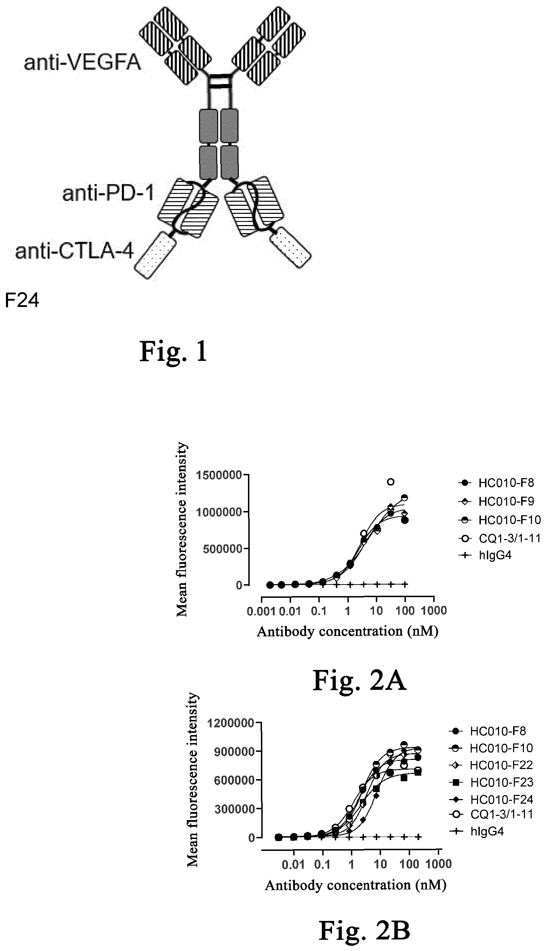

FACS was used to detect the activity of the trispecific antibodies binding to the cell surface PD-1 antigen: 293T cells overexpressing PD-1 (293T-hPD-1, Kangyuan Bochuang, KC-0204) were digested with trypsin (Gibco, 25200072), the cells were washed twice with PBS (FACS buffer) containing 2% fetal bovine serum (FBS), resuspended in FACS buffer, and added to a 96-well plate at approximately 1x10⁵ cells/well. After centrifugation at 300 g for 5 minutes, the supernatant was discarded. The antibodies were serially diluted 3-fold with FACS buffer to obtain two gradient series solution FACS#1 and FACS#2, with the starting concentration of 200 nM (Table 5, FACS#1) or 100 nM (Table 5, FACS#2, Figure 2), respectively. Then 100 µL/well was added to a 96-well plate in triplicate, mixed, and incubated at 4°C for 60 min. The plate was centrifuged and washed three times with FACS buffer. 100 µL/well of 1:800 diluted AF647-anti-hIgG(H+L) (Jackson, Catalog No.: 109-605-003) was added and incubated at 4°C for 50 minutes. After washing the plate three times with FACS buffer, 100 µL/well of FACS buffer was added to resuspend the cells, and the fluorescence signal was detected on a flow cytometer (BECKMAN COULTER cytoFLEX). The binding activity was fitted using a four-parameter model to calculate the EC₅₀ value of each antibody binding to PD-1. The results are shown in Table 5 and Figure 2.

In order to compare the performance of the self-developed antibody CQ1-3/1-11 with the corresponding commercial antibody, the applicant used the same method to detect the binding activity of antibody CQ1-3/1-11-hIgG4 and antibody pembrolizumab to the cell surface PD-1 antigen. The results showed that the self-developed antibody CQ1-3/1-11 and pembrolizumab have comparable ability to bind to cell surface PD-1, with EC₅₀ values of 4.34 nM and 5.05 nM, respectively.

**Table 5 Activity of trispecific antibodies binding to PD-1, CTLA-4 and VEGFA (EC₅₀, nM)**

| | PD-1 binding activity | | | CTLA-4 binding activity | | | VEGFA binding activity |
|---|---|---|---|---|---|---|---|
| Experimental methods | ELISA | FACS#1 | FACS#2 | ELISA | FACS#3 | FACS#4 | ELISA |
| CQ1-3/1-11 | 0.041 | 2.800 | 1.119 | N/A | N/A | N/A | N/A |
| 202F1-hIgG1 | N/A | N/A | N/A | 0.078 | 1.713 | 1.046 | N/A |
| 202F1-hIgG4 | N/A | N/A | N/A | NT | 2.313 | NT | N/A |
| Ipilimumab | N/A | N/A | N/A | 0.010 | NT | NT | N/A |
| Bevacizumab | N/A | N/A | N/A | N/A | N/A | N/A | 0.020 |
| HC010-F6 | NT | NT | NT | NT | NT | NT | NT |
| HC010-F7 | 0.085 | NT | NT | 0.059 | NT | NT | 0.081 |
| HC010-F8 | 0.080 | 2.390 | 1.811 | 0.127 | 4.924 | 4.294 | 0.072 |
| HC010-F9 | 0.082 | 3.531 | NT | 0.031 | 2.803 | NT | 0.111 |
| HC010-F10 | 0.093 | 5.773 | 2.365 | 0.093 | 6.187 | 4.862 | 0.121 |
| HC010-F11 | 0.114 | NT | NT | 0.044 | NT | NT | 0.125 |
| HC010-F12 | NT | NT | NT | NT | NT | NT | NT |
| HC010-F13 | 0.307 | NT | NT | 0.438 | NT | NT | 0.098 |
| HC010-F14 | 0.472 | NT | NT | 0.734 | NT | NT | 0.096 |
| HC010-F15 | 0.712 | NT | NT | 0.527 | NT | NT | 0.087 |
| HC010-F16 | NT | NT | NT | NT | NT | NT | NT |
| HC010-F17 | 0.033 | NT | NT | 0.077 | NT | NT | 0.072 |
| HC010-F18 | 0.040 | NT | NT | 0.029 | NT | NT | 0.120 |
| HC010-F19 | NT | NT | NT | NT | NT | NT | NT |
| HC010-F20 | NT | NT | NT | NT | NT | NT | NT |
| HC010-F21 | NT | NT | NT | NT | NT | NT | NT |
| HC010-F22 | NT | NT | 3.445 | NT | 8.920 | 8.920 | NT |
| HC010-F23 | NT | NT | 2.126 | NT | 5.429 | 5.429 | NT |
| HC010-F24 | NT | NT | 7.910 | NT | 6.499 | 6.499 | NT |
| HIgG4 | N/A | N/A | N/A | N/A | N/A | N/A | N/A |

| | | | | | | | |
|---|---|---|---|---|---|---|---|
| N/A not applicable, NT not tested; FACS #1-#4 refer to different experimental batches, hIgG4 (Taizhou Baiying Biotechnology Co., Ltd., Catalog No.: B107804) is a negative control unrelated to the antibody of this application . | | | | | | | |

### 2.2 Binding activity of trispecific antibodies to CTLA-4

The binding activity of the trispecific antibodies to CTLA-4 protein and cell surface CTLA-4 antigen was detected by ELISA and FACS methods as disclosed in Section 2.1, respectively.

The human CTLA-4 protein used was purchased from Acro, Catalog No.: CT4-H52H9. The results are shown in Table 5.

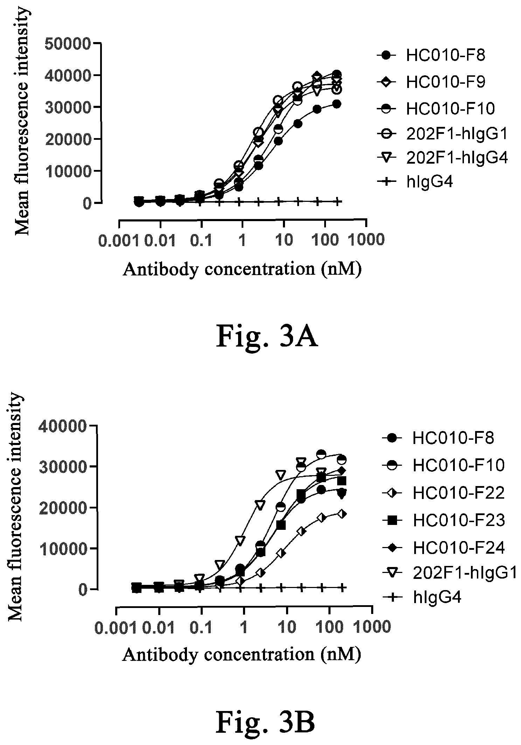

In the FACS assay, CHOK1 cells overexpressing human CTLA-4 (CHO-hCTLA-4, Genomeditech, GM-C18989) were used and added to a 96-well U-bottom plate at approximately 2x10⁵ cells/well. Antibodies were serially diluted 3-fold in FACS buffer, starting at 200 nM. The results are shown in Table 5 and Figure 3.

### 2.3 Binding activity of trispecific antibodies to VEGFA

The binding activity of the trispecific antibodies to VEGFA protein was detected by ELISA method as described in Section 2.1. The used human VEGF165-his protein was purchased from Acro, Catalog No.: VE5-H5248. The positive control antibody Bevacizumab was purchased from Roche, Catalog No.: 0210008H1545. The results are shown in Table 5 .

### 2.4 Binding activity of trispecific antibodies to activated T cells

The expression of PD-1 and CTLA-4 is upregulated on the surface of activated T cells. Human peripheral blood T cells were activated with anti-CD3 and anti-CD28 antibodies to detect the binding activity of the trispecific antibodies with activated T cells. The anti-PD-1 antibodies pembrolizumab (Merck, Catalog No.: S028905), nivolumab (Biointron, Catalog No.: B6924), sintilimab (Biointron, Catalog No.: B682101) and camrelizumab (Biointron, Catalog No.: B852001) and the anti-CTLA-4 antibody ipilimumab were used as positive controls.

Human peripheral blood mononuclear cells (PBMCs, Miaoshun (Shanghai) Biotechnology Co., Ltd., Catalog No.: A19K154025) were rapidly thawed in a 37°C water bath, resuspended in T cell isolating solution, and counted. After centrifugation at 400g for 10 minutes, the supernatant was removed and the cells were resuspended to 5x10⁷ cells/mL with T cell separation buffer. T cells were isolated according to the procedure of a T cell isolation kit (Stemcell, Catalog No.: 19051), and the obtained T cells were resuspended in culture medium. The cells were counted using NucleoCounter NC-200 and the T cell density was adjusted to approximately 1x10 ⁶ cells/mL. Dynabeads containing anti-CD3 and anti-CD28 antibodies (Invitrogen^{™}, Catalog No.: 11132D) and 10 ng/mL hIL-2 (Peprotech, Catalog No.: 200-02) were added and cultured in a 37°C, 5% CO₂ incubator for 3 days. After removing Dynabeads and supernatant, the activated T cells were resuspended in FACS buffer. The cell density was adjusted to about 1x10⁶ cells/mL, and 100 µL of cell solution was added into a 96-well U-bottom plate, 1x10⁵ cells/well. After centrifugation at 500 g for 5 minutes, the supernatant was discarded and 100 µL of the trispecific antibody solution of the present application serially diluted 3-fold with FACS buffer was added in triplicate, with a starting concentration of 200 nM. After mixing, incubated at 4°C for 60 minutes. After centrifugation and washing three times with FACS buffer, 100 µL/well of PE anti-human IgG Fc (Biolegend, Catalog No.: 366903) was added and incubated at 4°C for 50 minutes. After washing the plate three times with FACS buffer, 100 µL/well of BV421 anti-human CD3 antibody (OKT3) (Biolegend, Catalog No.: 317344) was added and incubated at 4°C for 50 minutes. After washing the plate three times with FACS buffer, 70 µL/well of PBS solution was added to resuspend the cells, and the fluorescent signal on CD3 T cells was detected on a flow cytometer (BD Cellesta). The binding activity was fitted using a four-parameter model, and the EC₅₀ value of each antibody binding to T cells was calculated.

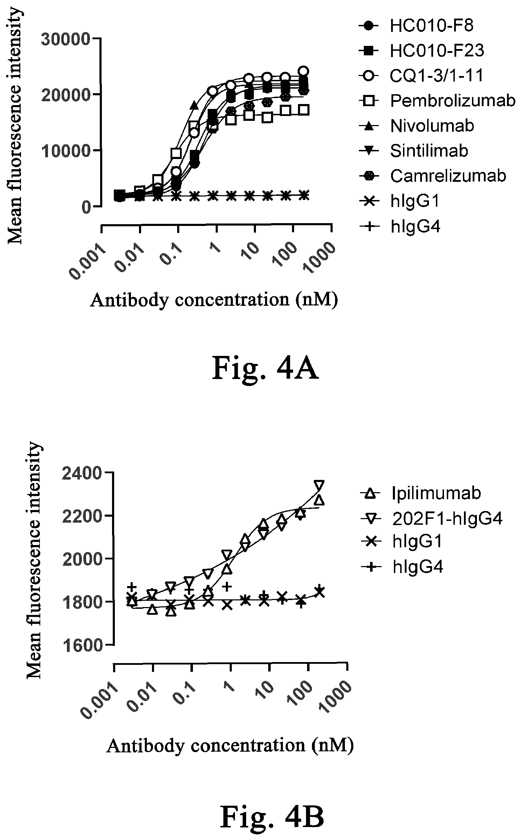

As shown in Figure 4 and Table 6, the commercial anti-PD-1 antibodies pembrolizumab, nivolumab, sintilimab and carrelizumab can bind to the activated T cells in a dose-dependent manner. In addition, the commercial anti-CTLA-4 antibody ipilimumab also binds to the activated T cells in a dose-dependent manner, indicating that the T cells activated by the method of this example express PD-1 and CTLA-4 on their surface. Since the mean fluorescence intensity (MFI) of ipilimumab binding to cell surface CTLA-4 is lower than the MFI of anti-PD-1 antibody binding to cell surface PD-1, this may be related to the low expression of CTLA-4 on the surface of activated T cells.

As shown in Figure 4 and Table 6, both the trispecific antibody HC010-F8 and HC010-F23 prepared in the present application can bind to the activated T cells in a dose-dependent manner, and their binding ability is better than that of the anti-CTLA-4 antibodies ipilimumab and 202F1.

**Table 6 Binding activity of specific antibodies to activated T cells**

| Antibody Name | EC₅₀, nM |
|---|---|
| HC010-F8 | 0.498 |
| HC010-F23 | 0.375 |
| CQ1-3/1-11 | 0.244 |
| Pembrolizumab | 0.082 |
| Nivolumab | 0.122 |
| Sintilimab | 0.231 |
| Camrelizumab | 0.473 |
| Ipilimumab | 1.274 |
| 202F1-hIgG4 | 1.463 |
| hIgG1 | N/A |
| hIgG4 | N/A |

| | |
|---|---|
| N/A: Not applicable. hIgG1 and hIgG4 are negative controls not related to the antibodies of this application. | |

### 2.5 Binding activity of trispecific antibodies to PD-1, CTLA-4, and VEGFA simultaneously

### 2.5.1 Binding of trispecific antibodies to CTLA-4 or VEGFA after binding to PD-1 protein

1 µg/mL human PD1-his (Acro, Catalog No. PD1-H5221) was used to coat a high-adsorption 96-well ELISA plate and incubated at 4°C overnight. The plate was washed with PBST solution and then blocked with 300 µL/well of 1% BSA-PBS at 37°C for 2 hours. At the same time, 1% BSA-PBS solution was used to prepare 3-fold serial dilutions of the antibody, with a starting concentration of 20 nM. After washing the plate, 100 µL of antibody solution at each gradient concentration was added to each well, in triplicate, and incubated at 37°C for 1 hour. The ELISA plate was then machine-washed three times with 0.05% PBST solution. One set of wells was incubated with 100 µL/well of 1 µg/mL Biotinylated-hCTLA4 (Acro, Catalog No.: CT4-H82E3) to assess the binding activity of the trispecific antibodies to CTLA-4 after binding to the PD-1 protein. Another set was incubated with 100 µL/well of 1 µg/mL Biotinylated-hVEGF165 (Acro, Catalog No.: VE5-H82Q0) to assess the binding activity of the trispecific antibodies to VEGF after binding to the PD-1 protein. The last set was incubated with hIgG4 as a negative control. After incubation at 37°C for 1 hour, the ELISA plate was machine-washed three times with PBST solution, and 100 µL/well of Streptavidin-HRP (BD pharmingen, Catalog No. 554066) diluted 1:5000 was added to the plate and incubated at 37°C for 45 minutes. The ELISA plate was machine-washed three times with PBST, and TMB was used for color development. The absorbance of the 96-well ELISA plate at 450 nm was detected by an ELISA reader, and the EC₅₀ value of the antibodies was calculated by fitting using a four-parameter model.

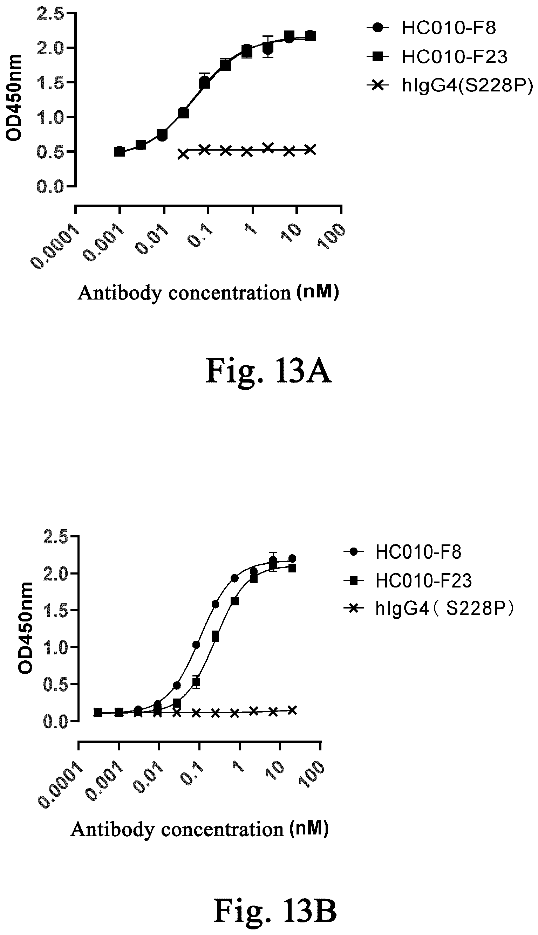

The results are shown in Figure 13. After binding to human PD1, the trispecific antibodies HC010-F8 and HC010-F23 can still bind to human VEGF (Figure 13A, EC₅₀ values were 0.049 and 0.054 nM, respectively) or human CTLA-4 (Figure 13B, EC₅₀ values were 0.105 and 0.250 nM, respectively), and the simultaneously binding activity is dose-dependent.

### 2.5.2 Trispecific antibodies simultaneously binding to CTLA-4 and VEGFA after binding to cell surface PD-1 protein

293T-hPD-1 cells were obtained by trypsin digestion, washed twice with PBS containing 2% FBS (FACS buffer), and the cell density was adjusted to about 6x10⁵ cells/ml. 200 µL/well of the cells were added to a 96-well U-bottom plate. After centrifugation at 300 g for 5 minutes, the supernatant was discarded and 100 µL of antibody solution serially diluted with FACS buffer was added to each well in triplicate (with starting concentration of 200 nM, 3-fold serial dilution, and the same volume of FACS buffer was used as a negative control). Mixed well and incubated at 4°C for 60 min. After washing the plate three times with FACS buffer, a mixture consisting of 1 µg/mL Biotin-hVEGF165 and 1 µg/mL hCTLA4-mFc (DIMA BIOTECH, Catalog No.: PME100017) in a 1:1 volume ratio was added to each well at 100 µL/well to detect the simultaneously binding activity of the trispecific antibodies to CTLA-4 and VEGF after binding to cell surface PD-1. After incubation at 4°C for 60 minutes, the cells were washed three times with FACS buffer, and a mixture of AF647 donkey anti-mIgG (H+L) (1:800 dilution, Invitrogen, Catalog No.: A31571) and PE Streptavidin (1:800 dilution, BD, Catalog No.: 554061) was added to each well at 100 µL/well and incubated at 4°C for 50 minutes. After washing the plate three times with FACS buffer, 100 µL/well of PBS solution was added to resuspend the cells, and the fluorescence signal was detected on a flow cytometer (BECKMAN COULTER cytoFLEX). The binding activity was fitted using a four-parameter model, and the EC₅₀ value of each antibody was calculated.

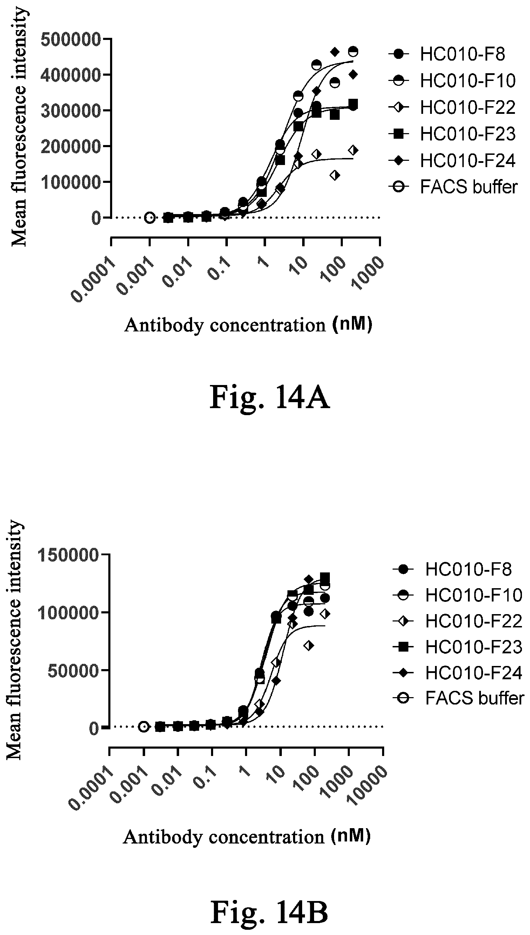

The results are shown in Figure 14. After binding to human PD-1 on the cell surface, the trispecific antibodies HC010-F8, HC010-F10, HC010-F22, HC010-F23 and HC010-F24 can still simultaneously bind to human VEGF (Figure 14A, EC₅₀ values were 1.413, 2.835, 2.288, 2.223 and 9.151 nM, respectively) and to human CTLA-4 (Figure 14AB, EC₅₀ values were 2.744, 3.323, 5.196, 3.837 and 12.000 nM, respectively), and the simultaneously binding activity is dose-dependent.

### 2.5.3 Trispecific antibodies simultaneously binding to PD-1 and VEGFA after binding to cell surface CTLA-4 protein

CHOK1-hCTLA4 cells were obtained by trypsin digestion, washed twice with PBS containing 2% FBS (FACS buffer), and the cell density was adjusted to about 8x10⁵ cells/ml. 200 µL/well of the cells were added to a 96-well U-bottom plate. After centrifugation at 300 g for 5 minutes, the supernatant was discarded and 100 µL of antibody solution serially diluted with FACS buffer was added in triplicate (with starting concentration of 200 nM, 3-fold serial dilution, and the same volume of FACS buffer was used as a negative control). Mixed well and incubated at 4°C for 60 min. After washing the plate three times with FACS buffer, a mixture consisting of 1 µg/mL hPD1-mFc (Acro, Catalog No.: PD1-H5255) and 1 µg/mL Biotin-hVEGF165 in a 1:1 volume ratio was added to each well at 100 µL/well to detect the simultaneously binding activity of the trispecific antibodies to VEGF and PD-1 after binding to cell surface CTLA-4. After incubation at 4°C for 60 minutes, the cells were washed three times with FACS buffer, and a mixture of AF647 donkey anti-mIgG (H+L) (1:800 dilution) and PE Streptavidin (1:800 dilution) dilution in a 1:1 volume ratio was added to each well at 100 µL/well and incubated at 4°C for 50 minutes. After washing the plate three times with FACS buffer, 100 µL/well of PBS solution was added to resuspend the cells, and the fluorescence signal was detected on a flow cytometer. The binding activity was fitted using a four-parameter model, and the EC₅₀ value of each antibody was calculated.

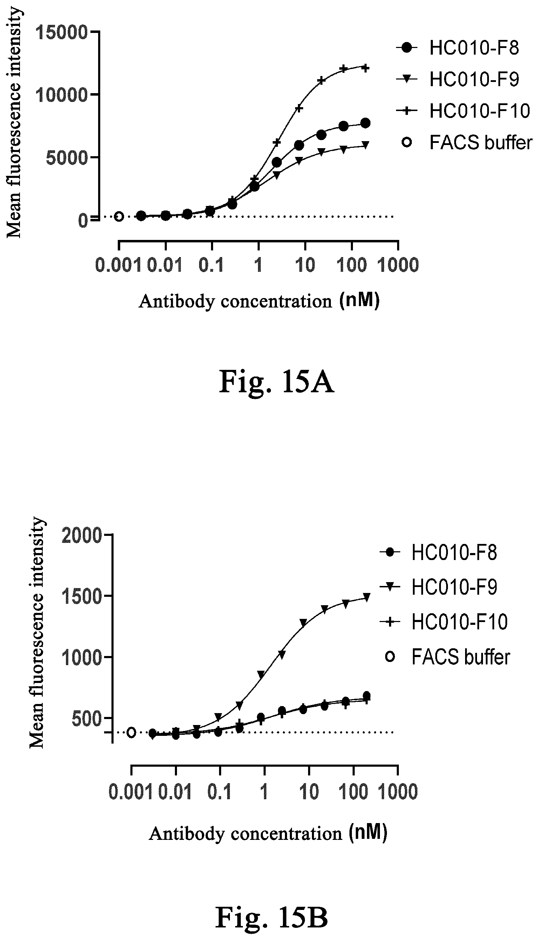

The results are shown in Figure 15. After binding to human CTLA-4 on the cell surface, the trispecific antibodies HC010-F8, HC010-F9 and HC010-F10 can still simultaneously bind to human PD-1 (Figure 15A, EC₅₀ values were 1.919, 1.533 and 2.734 nM, respectively) and to human VEGF (Figure 15B, EC₅₀ values were 1.435, 1.376 and 1.370 nM, respectively), and the simultaneously binding activity is dose-dependent.

### 2.6 Binding activity of trispecific antibodies to free VEGFA after binding to cells co-expressing PD-1 and CTLA-4

In the tumor microenvironment, trispecific antibodies can block immunosuppression and angiogenesis simultaneously by binding to PD-1 and CTLA-4 on immune cells and binding to VEGFA, thereby regulating the immune response and angiogenesis in the tumor microenvironment . In this example, the activity of the trispecific antibodies binding to free VEGFA after binding to cells co-expressing PD-1 and CTLA-4 was detected separately by using 293T cells overexpressing PD-1 and CTLA-4 and activated T cells.

### 2.6.1 Binding of trispecific antibodies to VEGFA after binding to cells co-expressing PD-1 and CTLA-4

293T cells overexpressing human PD-1 and human CTLA-4 (293T-hPD-1-hCTLA-4, Genomeditech, GM-C19526) were used to detect the binding activity of the trispecific antibodies to VEGFA after binding to cells co-expressing PD-1 and CTLA-4. The expression level of PD-1 on 293T-hPD-1-hCTLA-4 cells was higher than that of CTLA-4. After binding to 293T-hPD-1-hCTLA-4 cells through the arms against PD-1 and CTLA-4, the trispecific antibody bound to 293T-hPD-1-hCTLA-4 cells was detected using biotin-labeled VEGFA. 293T-hPD-1-hCTLA-4 cells were digested with trypsin, the separated cells were resuspended in FACS buffer, the cell density to was adjusted to about 1x10⁶ cells/mL, and then 200 µL/well of the cells were added into a 96-well U-bottom plate at a cell density of about 2x10⁵ cells/well. After centrifugation at 300 g for 5 minutes, the supernatant was discarded, and 100 µL of trispecific antibody solution serially diluted 3-fold with FACS buffer was added, with a starting concentration of 200 nM, and the same volume of FACS buffer was added as a negative control. After mixing, incubated at 4°C for 60 minutes. After centrifugation and washing three times with FACS buffer, 2 µg/mL Biotin-hVEGF165 was added at 100 µL/well and incubated at 4°C for 60 minutes. After centrifugation and washing three times with FACS buffer, 100 µL/well of 1:800 diluted PE Streptavidin (BD, Catalog No.: 554061) was added and incubated at 4°C for 50 minutes. After centrifugation and washing three times with FACS buffer, 100 µL/well of FACS buffer was added to resuspend the cells, and the fluorescence signal was detected on a flow cytometer (BECKMAN COULTER cytoFLEX). The binding activity was fitted using a four-parameter model, and the EC₅₀ value of each antibody binding was calculated.

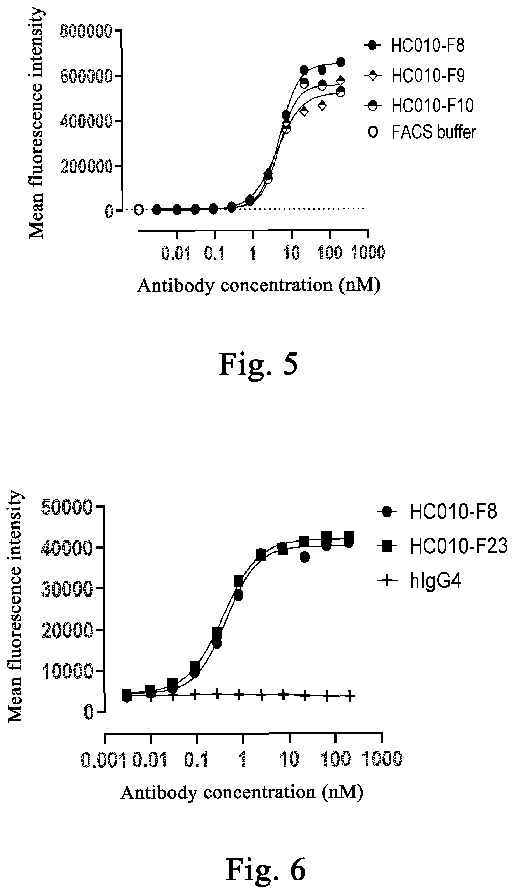

As shown in Figure 5, the trispecific antibodies HC010-F8, HC010-F9, and HC010-F10 still can bind to free VEGFA after binding to cells co-expressing PD-1 and CTLA-4. The EC₅₀ values of the binding activity of HC010-F8, HC010-F9 and HC010-F10 on cell surface to VEGFA were 5.057, 4.204 and 4.904 nM, respectively, indicating that the trispecific antibodies still have high binding activity to VEGFA after binding to cells co-expressing PD-1 and CTLA-4.

### 2.6.2 Binding activity of trispecific antibodies to free VEGFA after binding to activated T cells

The activated T cells in Section 2.4 were used, and the cell density was adjusted to 1x10 ⁶ cells/mL. 100 µL of the cells were added into a 96-well U-bottom plate, 1x10 ⁵ cells/well. After centrifugation at 500 g for 5 minutes, the supernatant was discarded and 100 µL of antibody solution serially diluted 3-fold in FACS buffer was added in triplicate, with a starting concentration of 200 nM. After mixing, incubated at 4°C for 60 minutes. After centrifugation and washing three times with FACS buffer, 2 µg/mL Biotinylated Human VEGF165 (Acro, Catalog No.:VE5-H82Q0) was added at 100 µL/well and incubated at 4°C for 50 minutes. After washing three times with FACS buffer, a mixture of BV421 anti-human CD3 antibody (OKT3) (Biolegend, Catalog No.: 317344) and PE Streptavidin was added at 100 µL/well and incubated at 4°C for 50 minutes. After washing three times with FACS buffer, 70 µL/well of PBS was added to resuspend the cells, and the fluorescent signal on CD3 T cells was detected on a flow cytometer (BDCellesta). The binding activity was fitted using a four-parameter model, and the EC₅₀ value of each antibody was calculated.

As shown in Figure 6, the trispecific antibodies HC010-F8 and HC010-F23 can still bind to free VEGFA after binding to activated T cells. The EC₅₀ values of the binding activity of HC010-F8 and HC010-F23 on T cell surface to VEGFA were 0.440 and 0.377 nM, respectively, indicating that the trispecific antibodies still have high binding activity to VEGFA after binding to T cells co-expressing PD-1 and CTLA-4, and can simultaneously block immune checkpoints and angiogenesis in the tumor microenvironment.

### 2.7 Determination of affinty of trispecific antibodies to human PD-1, CTLA-4 and VEGFA

Octet^{®} RED96e (ForteBio) was used to detect the affinity of trispecific antibodies to human PD-1, CTLA-4 and VEGFA. The specific method was as follows: the AHC Sensor was immersed in PBST (PBS containing 0.02% Tween) buffer for 10 minutes to activate the Sensor. The ligand (antibody to be tested) was diluted to 5 µg/mL with PBST buffer. The analytes: human PD1 (ACRO, Catalog No.: PD1-H5221), human VEGF (ACRO, Catalog No.: VE5-H4210) and human CTLA4 (ACRO, Catalog No.: CT4-H52H9) were diluted to a concentration gradient of 100, 50, 25, 12.5 and 6.25 nM with PBST buffer, the intermediate concentration was set as the quality control point, and 0 nM was set as the blank control point. The diluted ligand and analyte were added to the corresponding wells at 200 µL/well in triplicate. Program parameter settings: loading: 180 s, association: 180 s, dissociation: 600 s, regeneration: Gly-HCl pH 1.5, 30s, regeneration 3 times. Data Analysis 11.1r software was used to fit the curve using a 1:1 model after deducting the blank (concentration point of 0) and to analyze and calculate the experimental results. Curve fit acceptance criteria: Full R^2 ≥ 0.95.

The results are shown in Table 7. HC010-F8 and HC010-F23 have high affinity to human PD-1, human VEGF and human CTLA-4, respectively, reaching the order of 10⁻⁹.

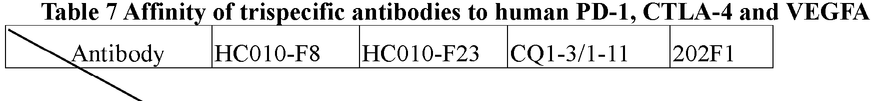

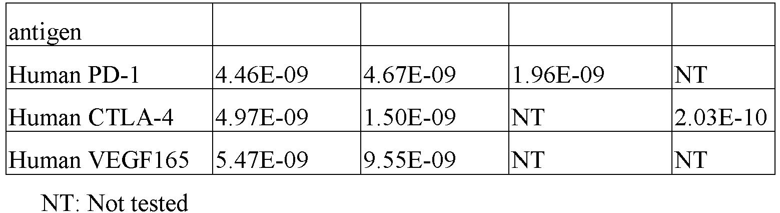

### Example 3. Blocking activity of anti-PD-1/CTLA-4/VEGF trispecific antibodies

### 3.1 Activity of trispecific antibodies to block the binding of PD-1 and PD-L1

Competitive ELISA and FACS were used to detect the activity of trispecific antibodies to block the binding of PD-1 and PD-L1.

The activity of trispecific antibodies to block the binding of PD-1 and PD-L1 was detected by ELISA: A high-adsorption 96-well ELISA plate was coated with 1 µg/mL human PD-1 protein (Acro, PD1-H5257) and incubated at 4°C overnight. The next day, the plate was washed three times with PBS solution and blocked with 1% BSA-PBS solution at 37°C for 1 hour. The antibody serial dilutions (starting concentration of 200 nM, 3-fold serial dilution) prepared in 1% BSA-PBST diluting solution and 1 µg/mL biotin-labeled PD-L1 protein (Acro, PD1-H82F3) were mixed in a volume of 1:1 to obtain an antibody-protein mixture. 100 µL of the antibody-protein mixture was added to each well of the blocked 96-well ELISA plate and incubated at 37°C for 1 hour. After washing four times with PBST, 100 µL of Strep-HRP (BD, Catalog No. 554066) diluted 1:5000 was added to each well of the ELISA plate and incubated at 37°C for 1 hour. After washing 4 times with PBST, TMB was used for color development. The absorbance at 450 nm and 630 nm was detected by a microplate reader, and OD450-OD630 was calculated. The fluorescence intensity was fitted using a four-parameter model, and the IC₅₀ value of the blocking activity of each antibody was calculated. The results are shown in Table 8.

In order to compare the performance of the self-developed antibody CQ1-3/1-11 with pembrolizumab, the applicant used the same method to detect the activity of the control self-developed antibody CQ1-3/1-11-hIgG4 to block the binding of the cell surface antigen PD-1 to PD-L1. The results show that the self-developed antibody CQ1-3/1-11 and pembrolizumab have comparable blocking abilities, with IC₅₀ values of 3.190 nM and 3.569 nM, respectively.

The activity of trispecific antibodies to block the binding of PD-1 and PD-L1 was detected by FACS: 293T cells overexpressing human PD-1 (293T-hPD-1, Kangyuan Bochuang, KC-0204) were used to detect the ability of trispecific antibodies to block the binding of human PD-1 and human PD-L1. 293T-hPD-1 cells were obtained by digeating with trypsin, washed twice with PBS containing 2% FBS (FACS buffer), and resuspended in FACS buffer. 1 µg/mL biotin-human PDL1 (Acro, Catalog No.: PD1-H82F3) was added to the suspended cell solution, and 50 µL/well of the cells were added to a 96-well U-bottom plate, approximately 1x10⁵ cells/well. 50 µL of antibody solution serially diluted 3-fold in FACS buffer was added in triplicate, with a starting concentration of 200 nM. After mixing, incubated at 4°C for 60 minutes. After washing three times with FACS buffer, 100 µL/well of 1:800 diluted PE Streptavidin was added and incubated at 4°C for 50 min. After washing three times with FACS buffer, 150 µL/well of PBS was added to resuspend the cells, and the fluorescence signal was detected on a flow cytometer (BECKMAN COULTER cytoFLEX). The four-parameter model was used to fit the binding activity and the IC₅₀ value of the blocking activity of each antibody was calculated. The results are shown in Table 8. The trispecific antibodies can effectively block the binding of PD-L1 to PD-1 on the surface of 293T cells.

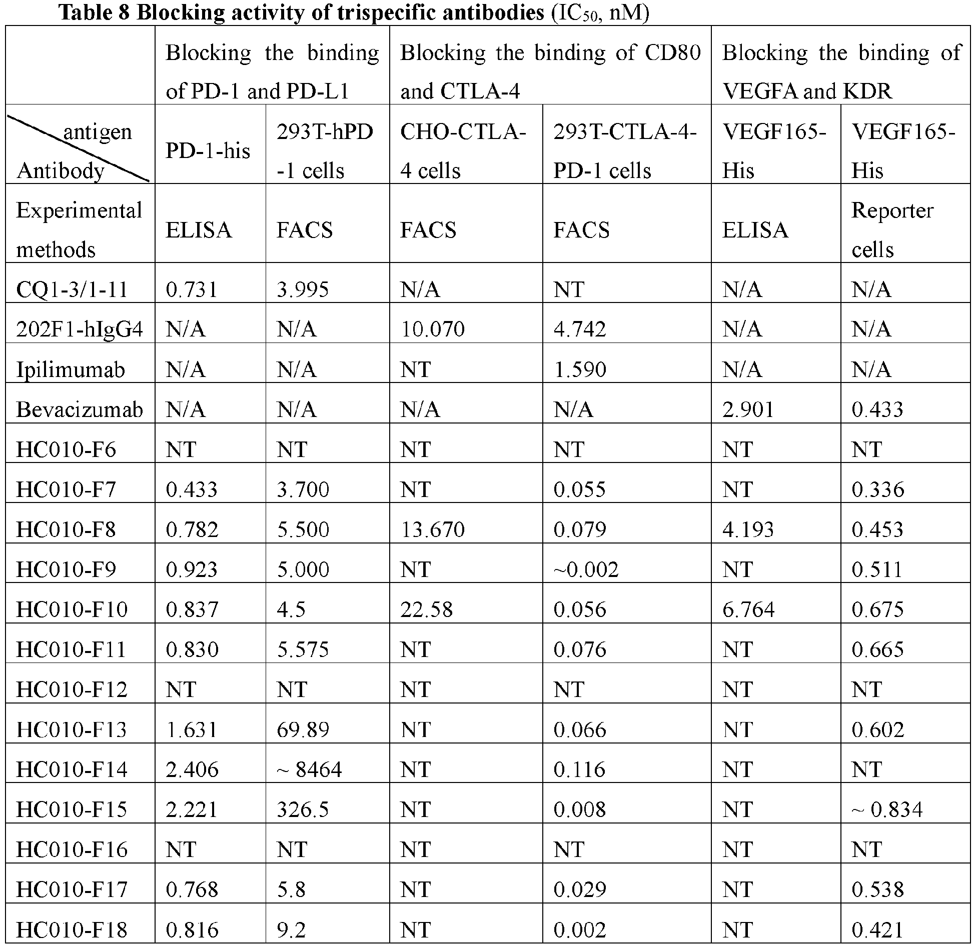

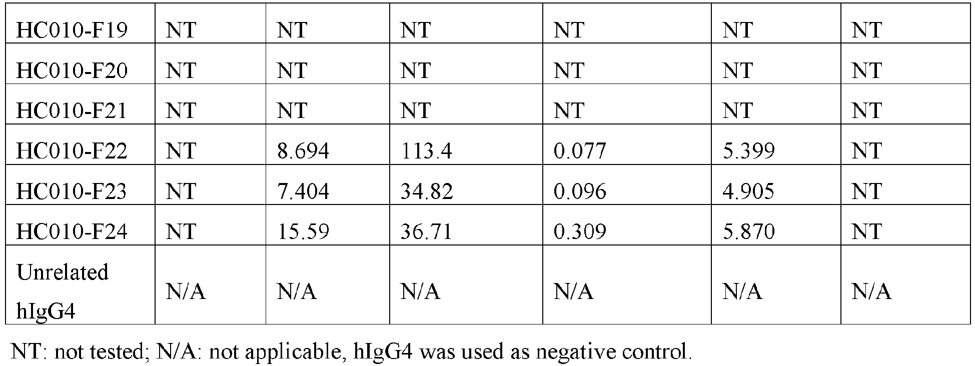

### 3.2 Activity of trispecific antibodies to block the binding of CD80 and CTLA-4

### 3.2.1 Activity of trispecific antibodies to block the binding of CD80 and CTLA-4

CHO-hCTLA-4 cells were used to detect the ability of the trispecific antibodies to block the binding of human CTLA-4 and human CD80. CHO-hCTLA-4 cells were obtained by trypsin digestion, washed twice with PBS containing 2% FBS (FACS buffer), and resuspended in FACS buffer. 200 µL of the cells were added to a 96-well U-bottom plate, approximately 2x10⁵ cells/well. After centrifugation at 300 g for 5 minutes, the supernatant was discarded. A series of antibody concentration gradients dilution (starting concentration was 200 nM, 3-fold serial dilutions) and 0.6 µg/mL biotin-labeled human CD80 protein (Acro, Catalog No.: B71-H82F2) were prepared using FACS buffer. 50 µL of antibody and 50 µL of biotin-human CD80 were added to each well of a 96-well plate, mixed well, and incubate at 4°C for 60 minutes. After washing three times with FACS buffer, 100 µL/well of 1:800 diluted PE Streptavidin was added and incubated at 4°C for 50 min. After washing three times with FACS buffer, 150 µL/well of PBS was added to resuspend the cells, and the fluorescence signal was detected on a flow cytometer (BECKMAN COULTER cytoFLEX). The four-parameter model was used to fit the binding activity and the IC₅₀ value of the blocking activity of each antibody was calculated. The results are shown in Table 8. The trispecific antibodies can effectively block the binding of human CTLA-4 and human CD80.

### 3.2.2 Activity of trispecific antibodies to block the binding of CD80 to CTLA-4 on cells co-expressing PD-1 and CTLA-4

PD-1 and CTLA-4 are co-expressed on functionally exhausted T cells in the tumor microenvironment, and the expression level of PD-1 is higher than that of CTLA-4. Bispecific antibodies that simultaneously target PD-1 and CTLA-4 can enhance the blocking function of anti-CTLA-4 antibodies on cells co-expressing PD-1 and CTLA-4. 293T cells that simultaneously overexpress human PD-1 and human CTLA-4 (293T-hPD-1-hCTLA-4, Genomeditech, GM-C19526, also known as 293T-CTLA-4-PD-1 cells) were used to detect the ability of the trispecific antibodies to block the binding of human CTLA-4 and human CD80. 293T-hPD-1-hCTLA-4 cells were obtained by trypsin digestion, washed twice with PBS containing 2% FBS (FACS buffer), and then resuspended in FACS buffer. 200 µL of the cells were added to a 96-well U-bottom plate, approximately 1.4x10⁵ cells/well. After centrifugation at 300 g for 5 minutes, the supernatant was discarded. A series of antibody concentration gradients dilution (starting concentration was 400 nM, 3-fold serial dilutions) and 2 µg/mL biotin-labeled human CD80 protein were prepared using FACS buffer. 50 µL of antibody and 50 µL of biotin-human CD80 were added to each well of a 96-well plate, mixed well, and incubate at 4°C for 60 minutes. After washing three times with FACS buffer, 100 µL/well of 1:800 diluted PE Streptavidin was added and incubated at 4°C for 50 min. After washing three times with FACS buffer, 150 µL/well of PBS was added to resuspend the cells, and the fluorescence signal was detected on a flow cytometer (BECKMAN COULTER cytoFLEX). The binding activity was fitted using a four-parameter model, and the IC₅₀ value of the blocking activity of each antibody was calculated.

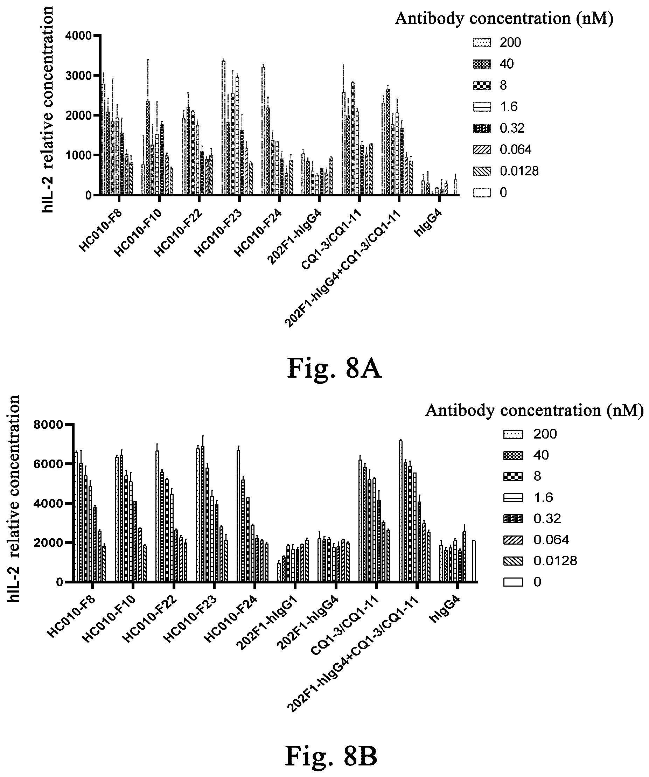

The results are shown in Figure 8. Compared with the parent antibody, the trispecific antibodies significantly enhanced the blocking effect on the binding of CD80 to CTLA-4 on 293T-hPD-1-hCTLA-4 cells.

### 3.3 Activity of trispecific antibodies to block the binding of VEGFA and KDR (VEGFR2)

### 3.3.1 Activity of trispecific antibodies to block the binding of VEGFA and KDR (VEGFR2) was detected by competitive ELISA

A high-adsorption 96-well ELISA plate was coated with 1 µg/mL human VEGF165-His (Acro, Catalog No.: VE5-H5248) and incubated at 37°C for 2 hours. After washing, each well was blocked with 300 µL 1% BSA-PBS at 37°C for 1.5 hours. 50 µL of serially diluted antibody solution (starting concentration was 400 nM, 3-fold serial dilution) and 50 µL of 1.72 µg/mL biotin-human KDR (Acro, Catalog No. KDR-H82E5) were added to each well of the ELISA plate and incubated at 37°C for 1 hour. After washing three times with PBST solution, 100 µL/well of 1:5000 diluted Streptavidin-HRP (BD pharmingen, Catalog No. 554066) was added to the plate and incubated at 37°C for 45 minutes. After washing three times with PBST, TMB was used for color development, the absorbance at 450 nm was detected by an enzyme reader and fitted by a four-parameter model. The IC₅₀ value for blocking activity of the antibody was calculated. The results are shown in Table 8. The activity of the trispecific antibodies to block the binding of VEGFA and KDR was comparable to that of bevacizumab.

### 3.3.2 Detection of blocking activity of trispecific antibodies on the binding of VEGF to KDR by Reporter gene assay

The human VEGFR2-293 reporter cell line (Genomeditech, GM-C09057) was used to detect the blocking ability of the trispecific antibodies on the binding of human VEGFA and human KDR. The human VEGFR2-293 reporter cell line is a luciferase reporter gene cell line constructed based on the NFAT signaling pathway. After binding to the KDR receptor, VEGF activates the NFAT signaling pathway, thereby activating the expression of luciferase.

VEGFR2-293 reporter cells were obtained by trypsin digestion and centrifuged at 300 g for 5 minutes, and the supernatant was discarded. After being resuspended in DMEM medium containing 1% FBS (test medium), 0.6 µg/mL hVEGF165-His was added to the cell suspension, which was then added to a 96-well plate with a transparent bottom at approximately 1x10⁴ cells/well. A 3-fold serial dilutions of antibody were prepared in assay medium (starting at a concentration of 22.2 nM), added to 96-well plate, mixed well and incubated at 37°C for 6 hours. Luciferase buffer (Promega, Catalog No.: G7940) was added and incubated for 5 minutes in the dark, and the fluorescence signal was detected on a Microplate Reader (CLARIOstar Plus).

The results are shown in Table 8. The trispecific antibodies can effectively block the binding of human VEGF to human KDR on the cell surface, and its blocking efficiency is comparable to that of the parent antibody bevacizumab.

### Example 4 The function of trispecific antibodies in activating immune response and inhibiting VEGF-induced cell proliferation in vitro

### 4.1 Trispecific antibodies enhance T cell function

### 4.1.1 Trispecific antibodies enhance IL-2 secretion by T cells under SEB-stimulated PBMCs

Staphylococcal enterotoxin B (SEB) is a superantigen that can activate a large number of T cells at low concentrations and produce a strong immune response. It can directly bind to T cell receptors and MHC molecules without being processed into antigenic peptides to activate T cells. Anti-PD-1 and anti-CTLA-4 antibodies can promote SEB-stimulated T cells to express and secrete IL-2.

This example tested the effect of the trispecific antibodies on IL-2 secretion by SEB-stimulated T cells, with irrelevant human anti-HEL hIgG4 (Taizhou Baiying Biotechnology Co., Ltd., Catalog No.: B107804) as a negative control. PBMCs were rapidly thawed in a 37°C water bath and resuspended in RPMI 1640 medium (Gibco, Catalog No.: A10491-01) containing 10% FBS (fetal bovine serum, Gibco, Catalog No.: 10091-148) and 1% P/S (Pen-Strep, Gibco, Catalog No.: 15140122). The cell density of PBMCs was adjusted to about 1x10⁶ cells/mL, and SEB (Toxin technology, Catalog No.: 92815B) was added to the cell suspension to a final concentration of 200 ng/mL. 100 µL/well of the cell suspension mixed with SEB was added to a flat-bottom 96-well plate. Antibodies and control antibodies were diluted 3-fold with culture medium, with a starting concentration of 400 nM, added at 100 µL/well to a 96-well plate in triplicate, mixed with cells. Cultured in a 37°C, 5% CO₂ incubator for 3 days. After 3 days, the supernatant was collected and the IL-2 secretion was detected using a human IL-2 detection kit (CisBio, Catalog No.: 62HIL02PEH) according to the instructions. The value was read on CLARIOstar Plus.

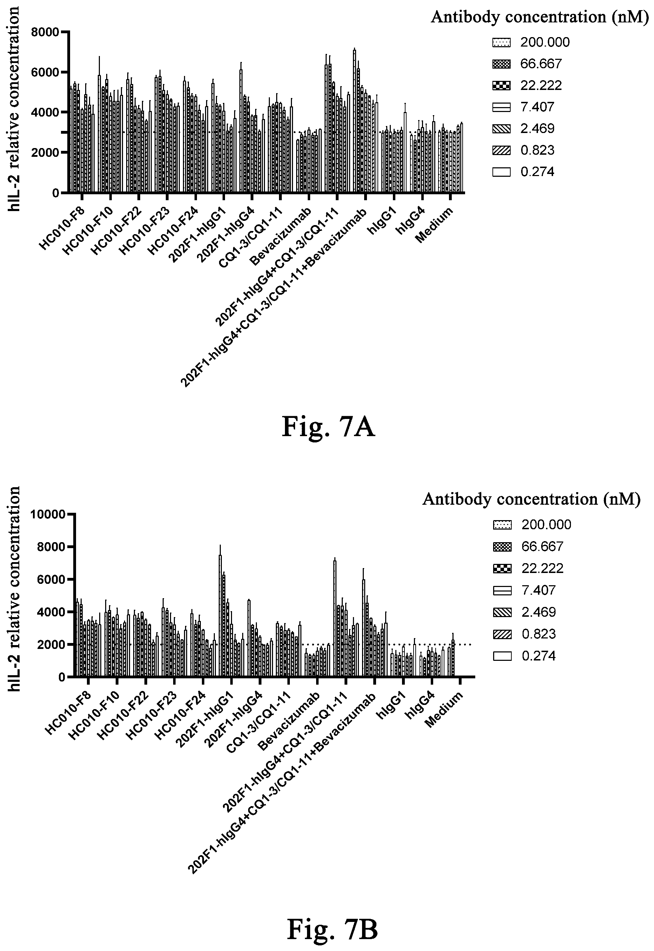

The results are shown in Figure 7, and the trispecific antibodies HC010-F8, HC010-F10, HC010-F22, HC010-F23 and HC010-F24 can all enhance the secretion of IL-2 by SEB-stimulated PBMCs.

### 4.1.2 Trispecific antibodies enhance IL-2 secretion by T cells in mixed lymphocyte reaction (MLR)

When mature dendritic cells (DCs) from different donors are incubated with PBMCs, DCs promote the expression and secretion of IL-2 by activating PBMCs (mainly T cells). PD-1 and CTLA-4 on T cells will bind to PD-L1/PD-L2 and CD80/CD86, which are highly expressed on mature DCs, respectively, resulting in the reduced expression of IL-2 cytokines. Immune checkpoint inhibitors such as anti-PD-1 antibody can promote the secretion of cytokines in MLR reaction.

This study tested the effect of the trispecific antibodies on IL-2 secretion in the MLR. DCs (Allcells) and PBMCs (Allcells) were rapidly thawed in a 37°C water bath and resuspended in X-VIVO15 (Lonza, Catalog No.: 04-418Q) medium. The cell densities of DCs and PBMCs were adjusted to 1x10⁵ cells/mL and 2x10⁶ cells/mL, respectively. DCs and PBMCs were mixed in a 1:1 volume ratio. After mixing, 200 µL of the cell mixture was added to a round-bottom 96-well plate. The trispecific antibodies and control antibodies were diluted 5-fold with X-VIVO15 solution, with a starting concentration of 1000 nM, and added at 50 µL/well to a 96-well plate in triplicate, mixed with the cells. Cultured in a 37°C, 5% CO₂ incubator for 4 days. After 4 days, the supernatant was collected and the IL-2 secretion was detected using a human IL-2 detection kit according to the instructions. The value was read on CLARIOstar Plus.

The results are shown in Figure 8. The trispecific antibodies HC010-F8, HC010-F10, HC010-F22, HC010-F23 and HC010-F24 can all enhance the secretion and expression of IL-2 in MLR. The activity is better than that of the parent anti-CTLA-4 antibody 202F1-hIgG4, and is comparable to the activity of the anti-PD-1 antibody CQ1-3/1-11, and the combination of 202F1-hIgG4 and CQ1-3/1-11.

The same approach was used to compare the effects of antibodies CQ1-3/1-11 and pembrolizumab on IL-2 secretion in MLR and the results showed that CQ1-3/1-11 and pembrolizumab have comparable activity.

### 4.1.3 Trispecific antibodies enhance the activity of T cells to secrete IL-2 in mixed lymphocyte reactions (MLRs) containing Tregs

Blocking PD-1 activity on effector T cells can promote anti-tumor immunity. However, since Treg cells also express high levels of PD-1, blocking PD-1 activity on Tregs can increase the inhibitory function of Tregs, thereby inhibiting anti-tumor immunity. This may be related to the poor response of some cancer patients to anti-PD-1 treatment. Studies have found that the combined use of anti-PD-1 antibody and anti-CTLA-4 antibody or bispecific antibody against PD-1 and CTLA-4 can increase the activity of T cells in the presence of Tregs. Therefore, in this example, MLRs containing different ratios of Tregs were used to detect the performance of trispecific antibodies on activating immune cells in the presence of Tregs.

The frozen PBMCs (Allcells), mature DCs (Allcells) and activated Tregs (Allcells) from three different healthy donors were thawed. PBMCs and DCs were adjusted with RPMI 1640 complete medium (Gibco, Catalog No.: 61870-036) to the cell density of 2×10⁶ cells/mL and 0.1×10⁶ cells/mL, respectively, and were placed 50 µL per well in a U-bottom 96-well plate. Treg cells were adjusted to the cell density of 0.5×10⁶ cells/mL with RPMI 1640 complete medium_{'} and then serially diluted 2-fold to 0.25×10⁶, 0.125×10⁶ and 0.0625×10⁶ cells/mL, and 50 µL of the Treg cells with different densities were placed in each well of the above 96-well plate. Antibodies HC010-F8, HC010-F23, CQ1-3/1-11, 202F1, bevacizumab, the combination of CQ1-3/1-11 and 202F1-hIgG4, the combination of CQ1-3/1-11 and 202F1-hIgG4 and bevacizumab, and irrelevant isotype antibody were serially diluted 4-fold with RPMI 1640 complete medium, with a starting concentration of 2000 nM; the serially diluted solution samples were then diluted 1:10 with culture medium, and were placed 50 µL per well in the above-mentioned 96-well plate in triplicate. Incubated at 37°C, 5% CO₂ for 4 days. After 4 days, the supernatant was collected by centrifugation and the release of IL-2 was detected using hIL-2 HTRF (Cisbio, Catalog No.: 62HIL02PEH) according to the instructions.

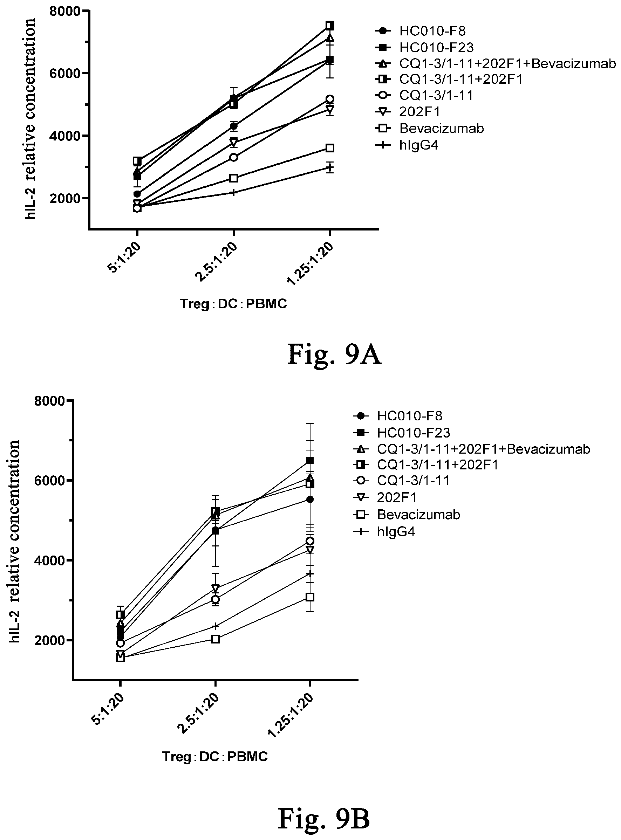

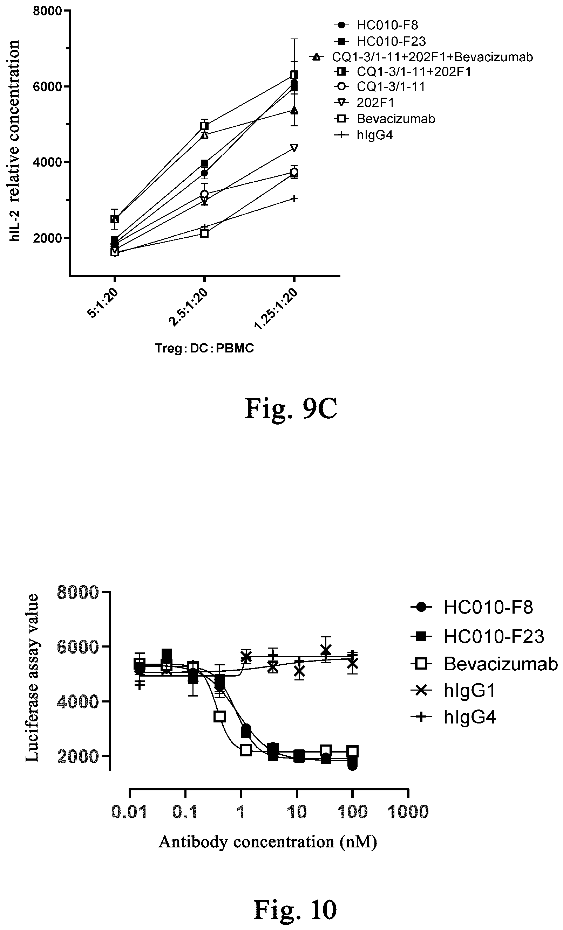

The results are shown in figure 9. When a higher proportion of Tregs were contained, the release of IL-2 in MLR was inhibited. The trispecific antibodies HC010-F8 and HC010-F23 can increase the release of IL-2, and the increasing effect is basically equivalent to that of the combined use of CQ1-3/1-11 and 202F1-hIgG4, and the combined use of bevacizumab, CQ1-3/1-11 and 202F1-hIgG4.

### 4.2 Trispecific antibodies inhibit VEGF-induced proliferation of human umbilical vein endothelial cells (HUVEC)

Human umbilical vein endothelial cells (HUVEC) express VEGF receptors and are therefore induced to proliferate by VEGF. They can be used to evaluate the inhibitory effects of anti-VEGF antibodies such as bevacizumab on the angiogenesis of VEGFA. In this example, HUVEC (ATCC, CRL-1730) was also used to detect the inhibition of VEGF function by the trispecific antibodies. HUVEC cells were obtained by trypsin digestion and resuspended in F-12K medium (Gibco, Cat. No.: 21127-022) containing 10% FBS. The cell density was adjusted to 1x10⁵ cells/mL. 50 µL of cell solution was added to a 96-well transparent bottom plate, about 5x10³ cells/well. Serially diluted antibodies solution (starting concentration was 200 nM, 3-fold serial dilution) and 0.4 µg/mL human VEGFA 165 protein were prepared with test medium, 25 µL antibody and 25 µL human VEGFA165 were added to each well of a 96-well plate, mixed well, and incubated at 37°C for five days. Five days later, 60 µL/well of CellTiter-Glo (Promega, Catalog No.: G7572) was added, and the fluorescence signal was detected on a Microplate Reader (CLARIOstar Plus). The IC₅₀ value of the antibody inhibitory activity was calculated using a four-parameter model.

As shown in Figure 10, the trispecific antibodies HC010-F8 and HC010-F23 can effectively block VEGFA-induced HUVEC cell proliferation, and the blocking activity IC₅₀ were 0.860 and 0.828 nM, respectively. The IC₅₀ of the blocking activity of the parent antibody bevacizumab was 0.367 nM. hIgG1 and hIgG4 were negative control antibodies unrelated to the antibodies of the present application.

### Example 5 In vivo efficacy of trispecific antibodies

### 5.1 Trispecific antibody HC010-F8 induces long-term anti-tumor effects

Human melanoma A375 cells were cultured in DMEM supplemented with 10% fetal bovine serum (FBS). When the growth density of A375 cells reached 60-80%, PBMCs (Allcells, Catalog No.: FPB004F-C) were thawed, and then resuspended in RPMI 1640 medium. The PBMCs was adjusted to cell density of 3×10⁶ cells/mL and co-cultured with A375 cells treated with Mitomycin C. After PBMCs were co-cultured with A375 for 5 days, the freshly digested PBMCs and A375 cells were collected. PBMCs (5×10⁵ cells/mouse) and A375 cells (4×10⁶ cells/mouse) were inoculated subcutaneously on the right side of NCG female mice (purchased from GemPharmatech Co., Ltd.) with an inoculation volume of 0.2 mL/mouse, containing 50% Matrigel (BD, Catalog No.: 354234). On the day of cell inoculation, the mice were randomly divided into 4 groups (5 mice in each group) and subcutaneously injected with trispecific antibody HC010-F8 (1 and 5 mg/kg), combination of three monoclonal antibodies CQ1-3/1-11 (0.65 mg/kg), ipilimumab (0.35 mg/kg) and bevacizumab (0.65 mg/kg) or negative control (PBS) twice a week for 3 weeks. Tumor volume was measured twice a week using a vernier caliper and the tumor volume was calculated using the formula V = 0.5 × a × b², where a and b represent the long and wide diameters of the tumor, respectively.

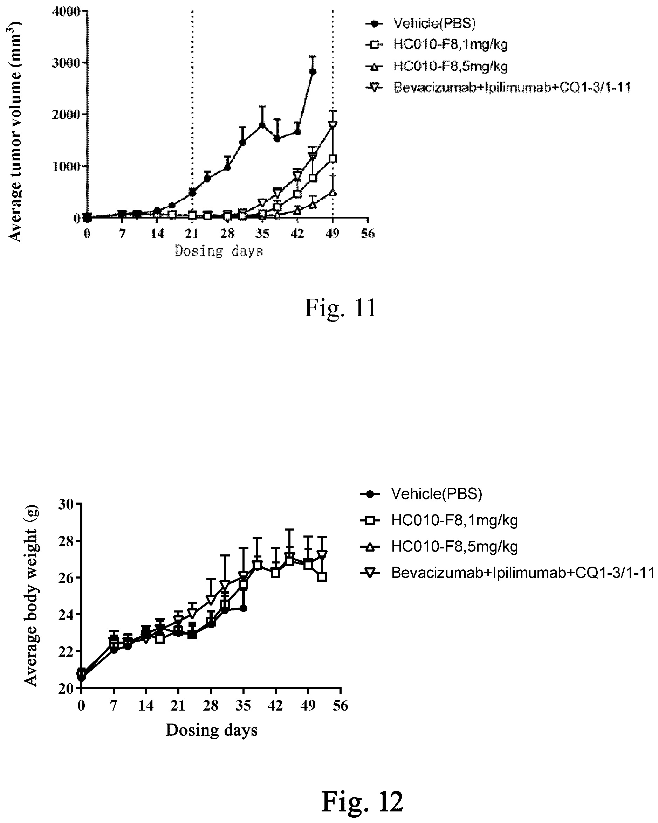

As shown in figure11, the antibody HC010-F8 effectively inhibited tumor growth at both 1 mg/kg and 5 mg/kg. After drug administration was terminated, the two treatment groups (1 and 5 mg/kg) of antibody HC010-F8 and the combination group all delayed tumor recurrence compared with the negative control group, and the tumor inhibition effect of HC010-F8 (1 and 5 mg/kg) was better than that of the combination group of CQ1-3/1-11, ipilimumab and bevacizumab.

In this example, mice in all administration groups showed no abnormal behavior or weight loss (figure12), indicating that tumor-bearing mice had good tolerance to the drug at the tested dose.

### 5.2 Anti-tumor effects induced by trispecific antibodies HC010-F8 and HC010-F23

Human melanoma A375 cells were cultured in DMEM supplemented with 10% fetal bovine serum (FBS). When the growth density of A375 cells reached 60-80%, PBMCs were thawed, and then resuspended in RPMI 1640 medium. The PBMCs was adjusted to cell density of 3×10⁶ cells/mL and co-cultured with A375 cells treated with Mitomycin C. After PBMCs were co-cultured with A375 for 5 days, the freshly digested PBMCs and A375 cells were collected. PBMCs (5×10⁵ cells/mouse) and A375 cells (4×10⁶ cells/mouse) were inoculated subcutaneously on the right side of NCG female mice with an inoculation volume of 0.2 mL/mouse, containing 50% Matrigel. On day 10 after tumor inoculation, when the average tumor volume was approximately 80 mm³, the mice were randomly divided into 4 groups (6 mice in each group) and subcutaneously injected with the combination group of three monoclonal antibodies (0.35 mg/kg of 202F1-hIgG4, 0.65 mg/kg of CQ1-3/1-11 and 0.65 mg/kg of bevacizumab), HC-010 F8 (1 mg/kg) group, HC010-F23 (1 mg/kg) group and negative control (PBS) group, twice a week for 3 weeks. Tumor volume was measured twice a week using a vernier caliper, and the tumor volume was calculated according to the above formula.

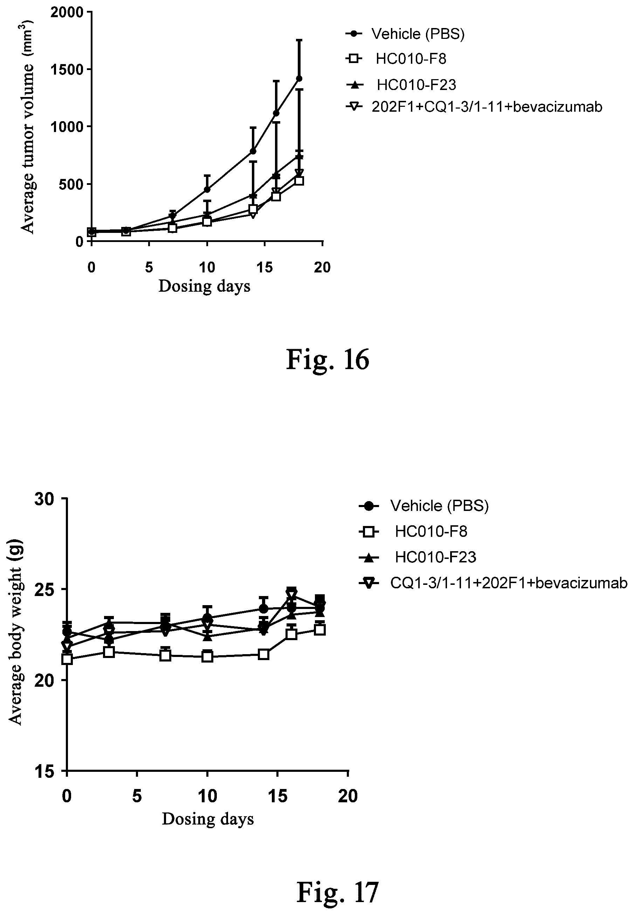

The results are shown in Figure 16. Compared with the negative control group, antibodies HC010-F8 (1 mg/kg) and HC010-F23 (1 mg/kg) and the combination group effectively inhibited tumor growth.

In this example, mice in all administration groups showed no abnormal behavior or weight loss (Figure17), indicating that tumor-bearing mice had good tolerance to the drug at the tested dose.

Both during the dosing period and during the withdrawal period, the excellent tumor inhibition of the trispecific antibodies of the present application make them have broad application prospects in the field of tumor treatment. They can not only effectively inhibit tumors, but also prolong the tumor inhibition period.

### 5.3 Trispecific antibodies inhibit the growth of non-small cell lung cancer A549 in human CD34⁺ HSC humanized mice

Human CD34⁺ HSC humanized mice (GemPharmatech) were obtained by transplanting human hematopoietic cells hCD34⁺ HSC into irradiated NCG mice for immune reconstruction. Human non-small cell lung cancer A549 cells were inoculated subcutaneously on the right side of human CD34⁺ HSC humanized mice. The inoculation volume was 0.2 mL/mouse, containing 30% Matrigel. When the average tumor volume was approximately 100 mm³, the mice were randomly divided into groups and subcutaneously injected with trispecific antibodies HC010-F8 (5 mg/kg), HC010-F23 (5 mg/kg), control antibodies pembrolizumab (3.25 mg/kg) and bevacizumab (3.25 mg/kg) and negative control (PBS), twice a week for 4 weeks. Tumor volume was measured twice a week using a vernier caliper, and the tumor volume was calculated according to the above formula. Tumor samples were harvested at the end of the experiment, minced with dissecting laboratory scissors, and enzymatically dissociated using a human tumor dissociation kit (Miltenyi Biotech) combined with a gentleMACS dissociator (Miltenyi Biotec) according to the manufacturer's instructions. The cell suspension was filtered through a 70 µm MACS smart filter to obtain a single-cell suspension. Immune cell subsets and functional biomarkers were analyzed by flow cytometry.

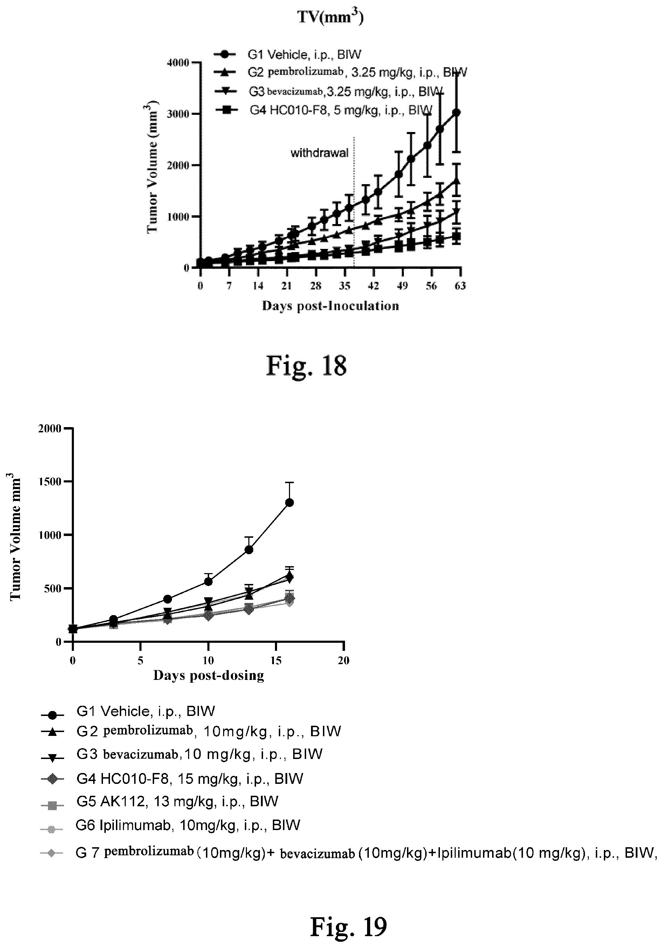

HC010-F8 and HC010-F23 significantly inhibited tumor growth compared with the negative control group, and the tumor inhibition rate was comparable to or even better than that of the pembrolizumab or bevacizumab administration group. During the drug withdrawal period, the tumor inhibition rate of the antibodies of the present invention was superior to that of the pembrolizumab or bevacizumab administration group (Figure 18).

### 5.4 Trispecific antibodies inhibit growth of human non-small cell lung cancer H1299 in human PBMC humanized mouse model

Human non-small cell lung cancer H1299 cells were inoculated subcutaneously on the right side of human PBMC humanized mice (GemPharmatech), with an inoculation volume of 0.2 mL/mouse, containing 30% Matrigel. When the average tumor volume was approximately 100 mm³, the mice were randomly divided into groups and subcutaneously injected with trispecific antibodies HC010-F8 (1-10 mg/kg), HC010-F23 (1-10 mg/kg), control antibody (1-10mg/kg) and negative control (PBS), twice a week for 4 weeks. Tumor volume was measured twice a week using a vernier caliper, and the tumor volume was calculated according to the above formula.

HC010-F8 and HC010-F23 effectively inhibited tumor growth relative to the negative control group.

The inventors further inoculate human non-small cell lung cancer H1299 cells into the right side of human PBMC humanized mice (Biocytogen). Seven days after inoculation, each animal was injected with 4.5×10⁶ cells/0.1 mL of PBMCs (human peripheral blood mononuclear cells). When the average tumor volume reached 120 mm³, appropriate mice were selected according to the tumor volume and body weight, with 9 mice in each group, for a total of 7 groups, namely: G1: PBS, G2: Pembrolizumab (10 mg/kg), G3: Bevacizumab (10 mg/kg), G4: HC010-F8 (15 mg/kg), G5: anti-PD-1/VEGFA bispecific antibody AK112 (13 mg/kg), G6: Ipilimumab (10 mg/kg) and G7: Pembrolizumab (10 mg/kg) + Bevacizumab (10 mg/kg) + Ipilimumab (10 mg/kg). The administration was initiated on the day of grouping for all groups, twice a week, and each group was administered 6 times in total. During the administration and observation period, the body weight and tumor volume of mice were measured twice a week, and the tumor growth inhibition rate (TGI_{TV}) was calculated.

At the end of the experiment, the mean tumor volume of the G1 vehicle control group (PBS) was 1303±188 mm³, and the mean tumor volumes of the G2-G7 treatment groups were 640±59 mm³, 582±96 mm³, 407±72 mm³, 406±44 mm³, 603±92 mm³ and 363±55 mm³, respectively. The corresponding TGI_{TV} of each treatment group was 57.40%, 60.97%, 75.70%, 75.72%, 59.06% and 79.60%, respectively. Compared with the vehicle control group, the G2-G7 groups had significant inhibitory effects on tumors (P <0.001). Therefore, the tumor inhibition rate of HC010-F8 alone was significantly better than that of pembrolizumab, bevacizumab or ipilimumab alone group, respectively; and was comparable to the efficacy of AK112 and the combination of pembrolizumab + bevacizumab + ipilimumab (Figure 19).

### 5.5 Trispecific antibodies inhibit growth of human non-small cell lung cancer NCI-H292 in human PBMC humanized mouse model

Human non-small cell lung cancer NCI-H292 cells were inoculated subcutaneously on the right side of human PBMC humanized mice (GemPharmatech), with an inoculation volume of 0.2 mL/mouse, containing 30% Matrigel. When the average tumor volume was approximately 100 mm³, the mice were randomly divided into groups and subcutaneously injected with trispecific antibodies HC010-F8 (1-10 mg/kg), HC010-F23 (1-10 mg/kg), control antibody (1-10mg/kg) and negative control (PBS), twice a week for 4 weeks. Tumor volume was measured twice a week using a vernier caliper, and the tumor volume was calculated according to the above formula.

HC010-F8 and HC010-F23 effectively inhibited tumor growth relative to the negative control group.

### 5.6 Trispecific antibodies inhibit growth of human colorectal cancer HT29 in human PBMC humanized mouse model

Human rectal cancer HT29 cells were inoculated subcutaneously on the right side of human PBMC humanized mice (Shanghai Model Organisms Center, Inc.) with an inoculation volume of 0.1 mL/mouse containing 30% Matrigel. When the average tumor volume was approximately 100 mm³, the mice were randomly divided into groups and subcutaneously injected with trispecific antibodies HC010-F8 (1-10 mg/kg), HC010-F23 (1-10 mg/kg), control antibody (1-10mg/kg) and negative control (PBS), twice a week for 4 weeks. Tumor volume was measured twice a week using a vernier caliper, and the tumor volume was calculated according to the above formula.

HC010-F8 and HC010-F23 effectively inhibited tumor growth relative to the negative control group.

Afterwards, the inventors further detect the activity and dose dependency of the trispecific antibodies of the present invention.

Human rectal cancer HT29 cells were inoculated subcutaneously on the right side of human PBMC humanized M-NSG mice (Shanghai Model Organisms Center, Inc.) with an inoculation volume of 0.1 mL/mouse containing 30% Matrigel. When the average tumor volume was approximately 100 mm³, the mice were randomly divided into groups, including Group 1: PBS, i.p., BIW *7 times group (hereinafter referred to as G1), Group 2: Pembrolizumab, 3.25 mg/kg, i.p., BIW *7 times group (hereinafter referred to as G2), Group 3: Bevacizumab, 3.25 mg/kg, i.p., BIW *7 times group (hereinafter referred to as G3), Group 4: anti-CTLA-4 antibody Ipilimumab, 3.25 mg/kg, i.p., BIW *7 times group (hereinafter referred to as G4), Group 5: HC010-F8, 1 mg/kg, i.p., BIW *7 times Group (hereinafter referred to as G5), Group 6: HC010-F8, 5 mg/kg, i.p., BIW *7 times Group (hereinafter referred to as G6), Group 7: HC010-F8, 20 mg/kg, i.p., BIW *7 times Group (hereinafter referred to as G7), Group 8: Pembrolizumab + bevacizumab 3.25 mg/kg + 3.25 mg/kg, i.p., BIW *7 times (hereinafter referred to as G8), Group 9: Pembrolizumab + Ipilimumab, 3.25 mg/kg + 3.25 mg/kg, i.p., BIW *7 times group (hereinafter referred to as G9) and Group 10: Pembrolizumab + Ipilimumab + Bevacizumab, 3.25 mg/kg + 3.25 mg/kg + 3.25 mg/kg, i.p., BIW *7 times group (hereinafter referred to as G10).

When the experiment was terminated on day 17 after administration, the average tumor volume of the control group G1 was 644.77±61.57 mm³. For G2, G4 and G9, the average tumor volumes were 498.68±48.30 mm³, 638.72±70.69 mm³ and 489.19±47.40 mm³, respectively; and the tumor volume inhibition rates (TGI) were 26.72%, 1.03% and 28.42%, respectively. There was no statistical difference compared with the control group (P > 0.05). For Group G3, the average tumor volume was 400.50±62.73 mm³, and the tumor volume inhibition rate (TGI) was 44.68%, which were statistically different from those of the control group (P <0.05). For G5, G6, G7, G8 and G10, the average tumor volumes were 403.54±45.88 mm³, 312.48±38.02 mm³, 270.45±33.25 mm³, 293.13±36.08 mm³ and 313.65±25.23 mm³, respectively; and the tumor volume inhibition rates (TGI) were 44.26%, 60.91%, 68.59%, 64.42% and 60.63%, respectively, which were significantly different from those of the control group (P < 0.01). The results refer to Table 9.

**Table 9**

| Group | Test agent | Tumor volume Day 17 after administration (mm³) | TGI (%) | *P* value |
|---|---|---|---|---|
| G1 | PBS | 644.77±61.57 | -- | -- |
| G2 | Pembrolizumab | 498.69±48.30 | 26.72 | ns |
| G3 | Bevacizumab | 400.50±62.73 | 44.68 | * |
| G4 | Ipilimumab | 638.72±70.69 | 1.03 | ns |
| G5 | HC010-F8_1mg/kg | 403.54±45.88 | 44.26 | ** |
| G6 | HC010-F8_5mg/kg | 312.48±38.02 | 60.91 | *** |
| G7 | HC010-F8_20mg/kg | 270.45±33.25 | 68.59 | *** |
| G8 | Pembrolizumab++bevacizumab | 293.13±36.08 | 64.42 | *** |
| G9 | Pembrolizumab + Ipilimumab | 489.19±47.40 | 28.42 | ns |
| G10 | Pembrolizumab + Ipilimumab + Bevacizumab | 313.65±25.23 | 60.63 | *** |

| | | | | |
|---|---|---|---|---|
| ns= not significant; *p<0.05, **p<0.01, ***p<0.001 | | | | |

Conclusion: Under the conditions of this example, the test agent HC010-F8 at 1, 5 and 20 mg/kg had significant antitumor effects in the colorectal cancer HT-29 model of PBMC humanized M-NSG mice in a dose-dependent manner. Compared with the vehicle control group (PBS), HC010-F8 showed significant efficacy at doses of equal to or more than 1 mg/kg. The tumor inhibition rate of HC010-F8 at 5 mg/kg (60.91%) was better than that of pembrolizumab (26.72%), bevacizumab (44.68%), or ipilimumab (1.03%). The anti-tumor effect of HC010-F8 at a dose of 5 mg/kg is better than the dual immune combination of pembrolizumab + ipilimumab.

### 5.7 Trispecific antibodies inhibit the growth of human liver cancer HepG2 in human PBMC humanized mouse model

Human liver cancer HepG2 cells were inoculated subcutaneously on the right side of human PBMC humanized mice (Shanghai Model Organisms Center, Inc.) with an inoculation volume of 0.2 mL/mouse containing 30% Matrigel. When the average tumor volume was approximately 150-200 mm³, the mice were randomly divided into groups and subcutaneously injected with trispecific antibodies HC010-F8 (1-10 mg/kg), HC010-F23 (1-10 mg/kg), control antibody (1-10mg/kg) and negative control (PBS), twice a week for 4 weeks. Tumor volume was measured twice a week using a vernier caliper, and the tumor volume was calculated according to the above formula.

HC010-F8 and HC010-F23 effectively inhibited tumor growth relative to the negative control group.

### 5.8 Trispecific antibodies inhibit the growth of human liver cancer Huh7 in human PBMC humanized mouse model

Human liver cancer Huh7 cells were inoculated subcutaneously into human PBMC humanized NCG mice (GemPharmatech) at a dose of 5×10^6 cells/100 µL/mouse (with Corning Matrigel added at a ratio of 1:1). Mice were enrolled when the average tumor volume reached 77.20 mm³. Twenty-four mice were randomly divided into three groups according to the tumor volume, with 8 mice in each group. Mice were administered with pembrolizumab (3.25 mg/kg), HC010-F8 (5 mg/kg) and vehicle PBS control group *(i.p.),* twice a week for a total of 6 times and observed for 20 days. The drug efficacy was evaluated based on observation indicators such as tumor inhibition rate and body weight.

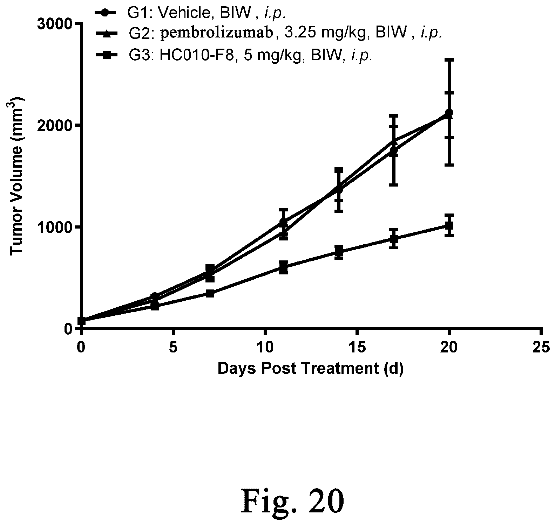

This study reached the endpoint on day 20 after the start of drug administration. The average tumor volumes of the PBS group, pembrolizumab group (3.25 mg/kg) and HC010-F8 (5 mg/kg) were 2124.08 mm³, 2099.87 mm³, and 1014.72 mm³, respectively. Compared with the PBS control group, HC010-F8 had a significant difference in tumor growth inhibition (P <0.0001), while pembrolizumab had almost no tumor inhibitory effect. Compared with pembrolizumab, HC010-F8 had a significant anti-tumor effect (P < 0.0001) (see Figure 20). During the experiment, no animal deaths related to the toxicity of the test agents occurred. Conclusion: In the mouse tumor model with human liver cancer Huh7, HC010-F8 had a significant anti-tumor effect, while pembrolizumab showed no obvious tumor inhibitory effect. HC010-F8 was well tolerated by animals at a dose of 5 mg/kg.

### 5.9 Efficacy of surrogate antibody to the trispecific antibodies in human PD-1 and human CTLA-4 dual targets humanized mouse tumor model

The trispecific antibodies do not recognize mouse PD-1, CTLA-4, and VEGFA. The anti-VEGF sequence in HC010-F8 and HC010-F23 was replaced with anti-VEGFA sequence (B20.4.1) as reported in US20110159009A1 and Wei-Ching Liang et al. Cross-species vascular endothelial growth factor (VEGF)-blocking antibodies completely inhibit the growth of human tumor xenografts and measure the contribution of stromal VEGF. J Biol Chem. 2006 Jan 13;281(2):951-61. doi: 10.1074/jbc.M508199200. Epub 2005 Nov 7. Thus a surrogate antibody capable of recognizing mouse VEGF was obtained. B20.4.1 has similar binding and blocking activities to bevacizumab, which has been used in HC010-F8 and HC010-F23.

The anti-tumor activity of the surrogate antibody to the trispecific antibodies was evaluated in the colorectal cancer MC38 tumor model and the liver cancer Hepa1-6 tumor model of humanized mice with dual targets of human PD-1 and human CTLA-4 (B-hPD-1/hCTLA4, Biocytogen). When the average tumor volume was approximately 100 mm³, the mice were randomly divided into groups and subcutaneously injected with the surrogate antibodies of trispecific antibodies HC010-F8 (1-10 mg/kg), the surrogate antibodies of HC010-F23 (1-10 mg/kg), control antibody (1-10mg/kg) and negative control (PBS), twice a week for 4 weeks.

The results showed that the surrogate antibody of HC010-F8 and the surrogate antibody of HC010-F23 had good anti-tumor activity in the colorectal cancer MC38 tumor model and the liver cancer Hepa1-6 tumor model of humanized mice with dual targets of human PD-1 and human CTLA-4 (B-hPD-1/hCTLA4, Biocytogen).

### Example 6 Stability study of trispecific antibodies

In the field of antibody preparation, stability issues are often faced for various antibody derivatives (such as multispecific antibodies) obtained based on the modification of natural antibodies. It is of great significance in the preparation, transportation, storage, etc. of antibodies to provide antibody derivatives with excellent stability. The stability of the trispecific antibodies obtained in the present application was detected in the example, and the thermal stability of the trispecific antibodies was studied under accelerated conditions of elevated temperature (40° C). Specifically, each of the trispecific antibodies was exchanged in formulation buffer comprising 20 mM His-HCl, pH 5.5, 230 mM trehalose and 0.01% tween 80. The samples were then incubated at 40°C for 0, 1, 2, and 4 weeks and analyzed results of size exclusion chromatography (SEC) and non-reducing capillary gel electrophoresis (NR-CE-SDS) by high performance liquid chromatography (Dionex, Ultimate 3000). The results are shown in Table 9. After incubation at 40°C for 4 weeks, the SEC purity of the trispecific antibodies HC010-F8 and HC010-F22 changed slightly, with the main peak (MP%) decreasing by less than 5%, and the main peak of HC010-F10 and HC010-F23 decreasing by about 10%. After incubation at 40°C for 4 weeks, the main peak of the trispecific antibodies in NR-CE-SDS decreased by 6.6% to 13.2%, indicating that the trispecific antibodies constructed and expressed in the present application have good stability.

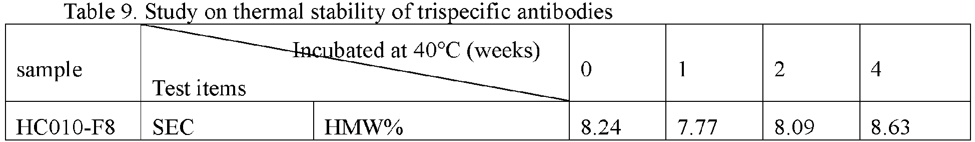

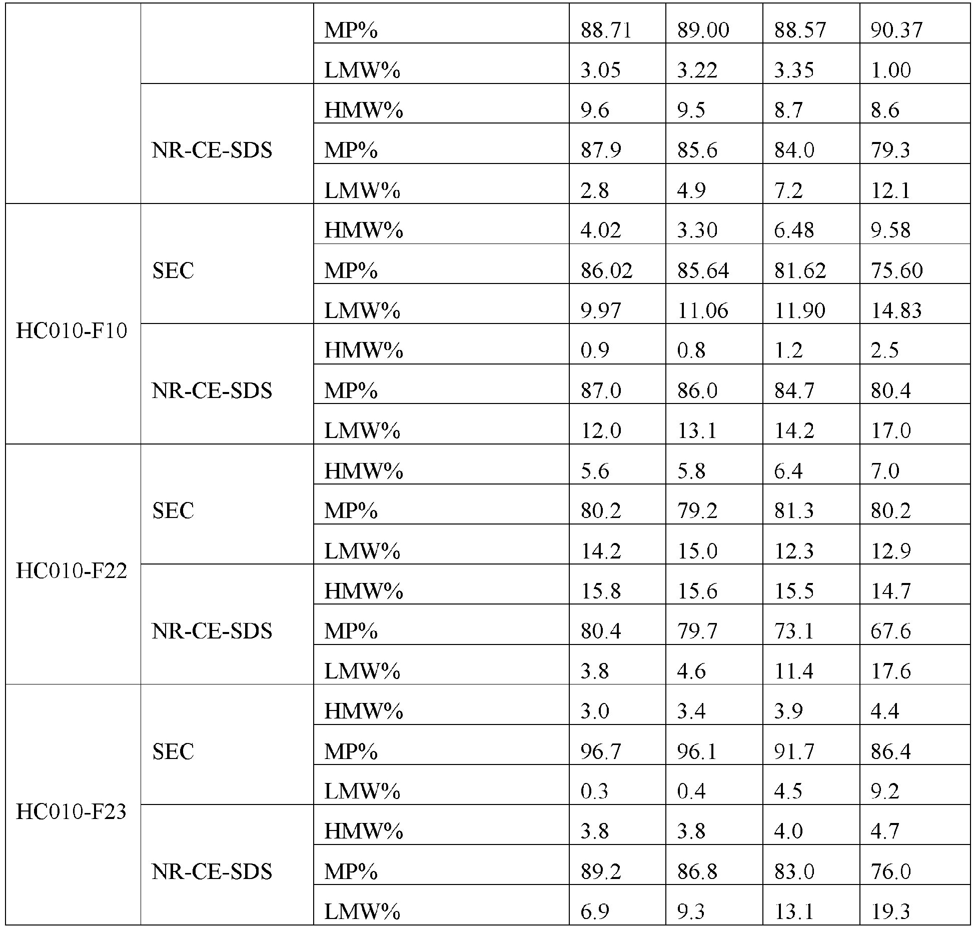

## Claims

1. A trispecific antibody comprising a first, a second, and a third antigen binding site, wherein the first, the second, and the third antigen binding site bind to a first, a second, and a third antigen that are different from each other and independently selected from PD-1, CTLA-4 and VEGF.

2. The trispecific antibody of claim 1, comprising a first, a second, and a third antigen binding site, and consisting of two identical heavy chains and two identical light chains, wherein the heavy chain and the light chain have a structure selected from the following:
1) the heavy chain having the structure of VH-CH1-Fc-VHH-ScFv from N-terminus to C-terminus,
the light chain having the structure of VL-CL from N-terminus to C-terminus; or
2) the heavy chain having the structure of ScFv-VH-CH1-Fc-VHH from N-terminus to C-terminus,
the light chain having the structure of VL-CL from N-terminus to C-terminus; or
3) the heavy chain having the structure of VHH-VH-CH1-Fc-ScFv from N-terminus to C-terminus,
the light chain having the structure of VL-CL from N-terminus to C-terminus; or
4) the heavy chain having the structure of VH-CH1-Fc-ScFv-VHH from N-terminus to C-terminus,
the light chain having the structure of VL-CL from N-terminus to C-terminus; or
5) the heavy chain having the structure of VH-CH1-Fc-ScFv from N-terminus to C-terminus, the light chain having the structure of VL-CL-VHH from N-terminus to C-terminus; or
6) the heavy chain having the structure of ScFv-VH-CH1-Fc-ScFv from N-terminus to C-terminus,
the light chain having the structure of VL-CL from N-terminus to C-terminus; or
7) the heavy chain having the structure of VH-CH1-Fc-ScFv from N-terminus to C-terminus, the light chain having the structure of VHH-VL-CL from N-terminus to C-terminus; or
8) the heavy chain having the structure of ScFv-VHH-VH-CH1-Fc from N-terminus to C-terminus,
the light chain having the structure of VL-CL from N-terminus to C-terminus;
wherein Fc represents the Fc region of the immunoglobulin heavy chain, and the two heavy chains comprising the Fc region homodimerize through the Fc region,
wherein CH1 represents immunoglobulin heavy chain CH1 domain, and CL represents immunoglobulin light chain CL domain,
wherein VH-CH1 and VL-CL pair with each other to form Fab,
wherein the first, second and third antigen binding sites are in the form of Fab, VHH or ScFv, and each binds to different antigen independently selected from PD-1, CTLA-4 and VEGF.

3. The trispecific antibody of claim 1, comprising a first, second, and third antigen binding site, and consisting of three chains having the following structure:
1) a first heavy chain comprising the structure of VH-CH1-Fc-ScFv from N-terminus to C-terminus,
2) a second heavy chain comprising the structure of VHH-Fc-ScFv from N-terminus to C-terminus, and
3) a light chain comprising the structure of VL-CL from N-terminus to C-terminus,
wherein Fc represents Fc region of immunoglobulin heavy chain, and the two heavy chains comprising the Fc region dimerize through the Fc region,
wherein CH1 represents immunoglobulin heavy chain CH1 domain, and CL represents immunoglobulin light chain CL domain,
wherein VH-CH1 and VL-CL pair with each other to form Fab,
wherein the first, second and third antigen binding sites are in the form of Fab, VHH and/or ScFv, and each binds to different antigen independently selected from PD-1, CTLA-4 and VEGF.

4. The trispecific antibody of claim 1, comprising a first, second, and third antigen binding site, and consisting of three chains having the following structure:
1) a first heavy chain comprising the structure of VH-CH1-Fc-ScFv2 from N-terminus to C-terminus,
2) a second heavy chain comprising the structure of ScFv1-Fc-ScFv2 from N-terminus to C-terminus,
3) a light chain comprising the structure of VL-CL from N-terminus to C-terminus,
wherein Fc represents Fc region of immunoglobulin heavy chain, and the two heavy chains comprising the Fc region dimerize through the Fc region,
wherein CH1 represents immunoglobulin heavy chain CH1 domain, and CL represents immunoglobulin light chain CL domain,
wherein VH-CH1 and VL-CL pair with each other to form Fab,
wherein the first, second and third antigen binding sites are in the form of Fab, ScFv1 or ScFv2, and each binds to different antigen independently selected from PD-1, CTLA-4 and VEGF.

5. The trispecific antibody of claim 1, comprising a first, second, and third antigen binding site, and consisting of two chains having the following structure:
1) a first heavy chain comprising the structure of ScFv1-Fc-ScFv2 from N-terminus to C-terminus,
2) a second heavy chain comprising the structure of VHH-Fc-ScFv2 from N-terminus to C-terminus,
wherein Fc represents Fc region of immunoglobulin heavy chain, and the two heavy chains comprising the Fc region dimerize through the Fc region,
wherein the first, second and third antigen binding sites are in the form of VHH, ScFv1 or ScFv2, and each binds to different antigen independently selected from PD-1, CTLA-4 and VEGF.

6. The trispecific antibody of claims 1-5, wherein the first, second, and third antigen binding sites comprise:
1) an antigen binding site that binds to PD-1, comprising HCDR1 comprising or consisting of the sequence as shown in SEQ ID NO: 1, HCDR2 comprising or consisting of the sequence as shown in SEQ ID NO:2, and HCDR3 comprising or consisting of the sequence as shown in SEQ ID NO:3; and LCDR1 comprising or consisting of the sequence as shown in SEQ ID NO:4, LCDR2 comprising or consisting of the sequence as shown in SEQ ID NO:5, and LCDR3 comprising or consisting of the sequence as shown in SEQ ID NO:6;
2) an antigen binding site that binds to CTLA-4, comprising
i) HCDR1 comprising or consisting of the sequence as shown in SEQ ID NO: 17, HCDR2 comprising or consisting of the sequence as shown in SEQ ID NO: 18, and HCDR3 comprising or consisting of the sequence as shown in SEQ ID NO: 19; and LCDR1 comprising or consisting of the sequence as shown in SEQ ID NO: 20, LCDR2 comprising or consisting of the sequence as shown in SEQ ID NO: 21, and LCDR3 comprising or consisting of the sequence as shown in SEQ ID NO: 22; or
ii) HCDR1 comprising or consisting of the sequence as shown in SEQ ID NO: 12, HCDR2 comprising or consisting of the sequence as shown in SEQ ID NO: 13, and HCDR3 comprising or consisting of the sequence as shown in SEQ ID NO: 14; and
3) an antigen binding site that binds to VEGF, comprising HCDR1 comprising or consisting of the sequence as shown in SEQ ID NO:26, HCDR2 comprising or consisting of the sequence as shown in SEQ ID NO:27, and HCDR3 comprising or consisting of the sequence as shown in SEQ ID NO:28; and LCDR1 comprising or consisting of the sequence as shown in SEQ ID NO:29, LCDR2 comprising or consisting of the sequence as shown in SEQ ID NO:30, and LCDR3 comprising or consisting of the sequence as shown in SEQ ID NO:31.

7. The trispecific antibody of claims 1-6, wherein the first, second and third antigen binding sites comprise substitution in the FR regions, preferably, the substitution is heavy chain variable region G44C, light chain variable region Q100C or G100C (according to Kabat numbering).

8. The trispecific antibody of claims 1-7, wherein the first, second, and third antigen binding sites comprise:
1) an antigen binding site that binds to PD-1, comprising a heavy chain variable region and a light chain variable region, wherein the heavy chain variable region comprises the sequence as shown in SEQ ID NO:7, or comprises an amino acid sequence having at least 90% identity to SEQ ID NO:7, or consists of the sequence as shown in SEQ ID NO:7, and the light chain variable region comprises the sequence as shown in SEQ ID NO:8, or comprises an amino acid sequence having at least 90% identity to SEQ ID NO:8, or consists of the sequence as shown in SEQ ID NO:8;
2) an antigen binding site that binds to CTLA-4, comprising
i) a heavy chain variable region and a light chain variable region, wherein the heavy chain variable region comprises the sequence as shown in SEQ ID NO:15, or comprises an amino acid sequence having at least 90% identity to SEQ ID NO:15, or consists of the sequence as shown in SEQ ID NO:15, and the light chain variable region comprises the sequence as shown in SEQ ID NO:16, or comprises an amino acid sequence having at least 90% identity to SEQ ID NO:16, or consists of the sequence as shown in SEQ ID NO:16;
ii) comprises the sequence as shown in SEQ ID NO: 11, or comprises an amino acid sequence having at least 90% identity to SEQ ID NO: 11, or consists of the sequence as shown in SEQ ID NO: 11; and
3) an antigen binding site that binds to VEGF, comprising a heavy chain variable region and a light chain variable region, wherein the heavy chain variable region comprises the sequence as shown in SEQ ID NO:24, or comprises an amino acid sequence having at least 90% identity to SEQ ID NO:24, or consists of the sequence as shown in SEQ ID NO:24, and the light chain variable region comprises the sequence as shown in SEQ ID NO:25, or comprises an amino acid sequence having at least 90% identity to SEQ ID NO:25, or consists of the sequence as shown in SEQ ID NO:25.

9. The trispecific antibody of claims 1-8, wherein adjacent antigen binding sites are connected via a linker, and the antigen binding site is connected to Fc via a linker/hinge region.

10. The trispecific antibody of claim 9, wherein the linker comprises an amino acid sequence (G₄S)ₙ, wherein n is an integer equal to or greater than 1, preferably, the linker consists of an amino acid sequence (G₄S)₃ or (G₄S)₄, preferably, the linker has the sequence as shown in SEQ ID NO: 9, and the hinge region is a hinge region from immunoglobulin, preferably a hinge region from IgG

11. The trispecific antibody of claims 1-8, wherein the Fc region is from the Fc region of immunoglobulin IgG1 or IgG4, preferably, the Fc region is derived from the heavy chain constant region sequence as shown in SEQ ID NO: 33, 34 or 35, preferably, the Fc region comprises modification, for example, the Fc region comprises knob-into-hole structure.

12. The trispecific antibody of claim 11, wherein the Fc region comprises substitution selected from the group consisting of S228P, S354C, T366W, T366S, L368A, Y394C, Y407V, H435R, Y436F and K447A (according to the EU numbering system).

13. The trispecific antibody of claim 1 or 2, comprising:
1) a heavy chain comprising SEQ ID NO: 37 or an amino acid sequence having at least 90% identity thereto, and a light chain comprising SEQ ID NO: 38 or an amino acid sequence having at least 90% identity thereto; or
2) a heavy chain comprising SEQ ID NO: 39 or an amino acid sequence having at least 90% identity thereto, and a light chain comprising SEQ ID NO: 40 or an amino acid sequence having at least 90% identity thereto; or
3) a heavy chain comprising SEQ ID NO: 41 or an amino acid sequence having at least 90% identity thereto, and a light chain comprising SEQ ID NO: 42 or an amino acid sequence having at least 90% identity thereto; or
4) a heavy chain comprising SEQ ID NO: 43 or an amino acid sequence having at least 90% identity thereto, and a light chain comprising SEQ ID NO: 44 or an amino acid sequence having at least 90% identity thereto; or
5) a heavy chain comprising SEQ ID NO: 45 or an amino acid sequence having at least 90% identity thereto, and a light chain comprising SEQ ID NO: 46 or an amino acid sequence having at least 90% identity thereto; or
6) a heavy chain comprising SEQ ID NO: 47 or an amino acid sequence having at least 90% identity thereto, and a light chain comprising SEQ ID NO: 48 or an amino acid sequence having at least 90% identity thereto; or
7) a heavy chain comprising SEQ ID NO: 49 or an amino acid sequence having at least 90% identity thereto, and a light chain comprising SEQ ID NO: 50 or an amino acid sequence having at least 90% identity thereto; or
8) a heavy chain comprising SEQ ID NO: 59 or an amino acid sequence having at least 90% identity thereto, and a light chain comprising SEQ ID NO: 60 or an amino acid sequence having at least 90% identity thereto; or
9) a heavy chain comprising SEQ ID NO: 61 or an amino acid sequence having at least 90% identity thereto, and a light chain comprising SEQ ID NO: 62 or an amino acid sequence having at least 90% identity thereto; or
10) a heavy chain comprising SEQ ID NO: 63 or an amino acid sequence having at least 90% identity thereto, and a light chain comprising SEQ ID NO: 64 or an amino acid sequence having at least 90% identity thereto; or
11) a heavy chain comprising SEQ ID NO: 65 or an amino acid sequence having at least 90% identity thereto, and a light chain comprising SEQ ID NO: 66 or an amino acid sequence having at least 90% identity thereto; or
12) a heavy chain comprising SEQ ID NO: 67 or an amino acid sequence having at least 90% identity thereto, and a light chain comprising SEQ ID NO: 68 or an amino acid sequence having at least 90% identity thereto; or
13) a heavy chain comprising SEQ ID NO: 69 or an amino acid sequence having at least 90% identity thereto, and a light chain comprising SEQ ID NO: 70 or an amino acid sequence having at least 90% identity thereto; or
14) a heavy chain comprising SEQ ID NO: 71 or an amino acid sequence having at least 90% identity thereto, and a light chain comprising SEQ ID NO: 72 or an amino acid sequence having at least 90% identity thereto; or
15) a heavy chain comprising SEQ ID NO: 73 or an amino acid sequence having at least 90% identity thereto, and a light chain comprising SEQ ID NO: 74 or an amino acid sequence having at least 90% identity thereto; or
16) a heavy chain comprising SEQ ID NO: 75 or an amino acid sequence having at least 90% identity thereto, and a light chain comprising SEQ ID NO: 76 or an amino acid sequence having at least 90% identity thereto.

14. The trispecific antibody of claim 3, comprising:
a first heavy chain comprising SEQ ID NO:51 or an amino acid sequence having at least 90% identity thereto,
a second heavy chain comprising SEQ ID NO:53 or an amino acid sequence having at least 90% identity thereto, and
a light chain comprising SEQ ID NO: 52 or an amino acid sequence having at least 90% identity thereto.

15. The trispecific antibody of claim 4, comprising:
a first heavy chain comprising SEQ ID NO:56 or an amino acid sequence having at least 90% identity thereto,
a second heavy chain comprising SEQ ID NO:58 or an amino acid sequence having at least 90% identity thereto, and
a light chain comprising SEQ ID NO: 57 or an amino acid sequence having at least 90% identity thereto.

16. The trispecific antibody of claim 5, comprising:
a first heavy chain comprising SEQ ID NO:54 or an amino acid sequence having at least 90% identity thereto, and
a second heavy chain comprising SEQ ID NO:55 or an amino acid sequence having at least 90% identity thereto.

17. The trispecific antibody of claim 1, 2 or 13, comprising:
1) a heavy chain comprising or consisting of SEQ ID NO: 37, and a light chain comprising or consisting of SEQ ID NO: 38; or
2) a heavy chain comprising or consisting of SEQ ID NO: 39, and a light chain comprising or consisting of SEQ ID NO: 40; or
3) a heavy chain comprising or consisting of SEQ ID NO: 41, and a light chain comprising or consisting of SEQ ID NO: 42; or
4) a heavy chain comprising or consisting of SEQ ID NO: 43, and a light chain comprising or consisting of SEQ ID NO: 44; or
5) a heavy chain comprising or consisting of SEQ ID NO: 45, and a light chain comprising or consisting of SEQ ID NO: 46; or
6) a heavy chain comprising or consisting of SEQ ID NO: 47, and a light chain comprising or consisting of SEQ ID NO: 48; or
7) a heavy chain comprising or consisting of SEQ ID NO: 49, and a light chain comprising or consisting of SEQ ID NO: 50; or
8) a heavy chain comprising or consisting of SEQ ID NO: 59, and a light chain comprising or consisting of SEQ ID NO: 60; or
9) a heavy chain comprising or consisting of SEQ ID NO: 61, and a light chain comprising or consisting of SEQ ID NO: 62; or
10) a heavy chain comprising or consisting of SEQ ID NO: 63, and a light chain comprising or consisting of SEQ ID NO: 64; or
11) a heavy chain comprising or consisting of SEQ ID NO: 65, and a light chain comprising or consisting of SEQ ID NO: 66; or
12) a heavy chain comprising or consisting of SEQ ID NO: 67, and a light chain comprising or consisting of SEQ ID NO: 68; or
13) a heavy chain comprising or consisting of SEQ ID NO: 69, and a light chain comprising or consisting of SEQ ID NO: 70; or
14) a heavy chain comprising or consisting of SEQ ID NO: 71, and a light chain comprising or consisting of SEQ ID NO: 72; or
15) a heavy chain comprising or consisting of SEQ ID NO: 73, and a light chain comprising or consisting of SEQ ID NO: 74; or
16) a heavy chain comprising or consisting of SEQ ID NO: 75, and a light chain comprising or consisting of SEQ ID NO: 76.

18. The trispecific antibody of claim 1, 3 or 14, comprising:
a first heavy chain comprising or consisting of SEQ ID NO:51,
a second heavy chain comprising or consisting of SEQ ID NO: 53, and
a light chain comprising or consisting of SEQ ID NO: 52.

19. The trispecific antibody of claim 1, 4 or 15, comprising:
a first heavy chain comprising or consisting of SEQ ID NO:56,
a second heavy chain comprising or consisting of SEQ ID NO: 58, and
a light chain comprising or consisting of SEQ ID NO: 57.

20. The trispecific antibody of claim 1, 5 or 16, comprising:
a first heavy chain comprising or consisting of SEQ ID NO:54, and
a second heavy chain comprising or consisting of SEQ ID NO: 55.

21. Polynucleotides encoding the trispecific antibody of any one of claims 1-20.

22. A vector comprising the polynucleotides of claim 21, preferably, the vector is an expression vector.

23. A host cell comprising the polynucleotide of claim 21 or the vector of claim 22, for example, the host cell is a mammalian cell.

24. A method for producing the trispecific antibody of any one of claims 1-20, comprising:
culturing the host cell comprising the polynucleotides encoding the polypeptide chains of the antibody under conditions suitable for expressing the polypeptide chains; and assembling the polypeptide chains to produce the antibody under conditions suitable for assembly of the polypeptide chains into the antibody.

25. A pharmaceutical composition comprising the trispecific antibody of any one of claims 1-20 and a pharmaceutically acceptable carrier.

26. Use of the multispecific antibody according to any one of claims 1 to 20 or the pharmaceutical composition according to claim 25 in the manufacture of a medicament for treating and/or preventing cancer, autoimmune disease, infectious disease or angiogenesis-related disease in an individual, or in the manufacture of a reagent for diagnosing cancer, autoimmune disease, infectious disease or angiogenesis-related disease.

27. The use of claim 26, wherein the cancer is selected from the group consisting of solid tumors such as lung cancer (e.g., small cell lung cancer, non-small cell lung cancer), breast cancer, liver cancer, bladder cancer, breast cancer, melanoma, colon cancer, rectal cancer, ovarian cancer, cervical cancer, prostate cancer, pancreatic adenocarcinoma, basal cell carcinoma, esophageal cancer, bile duct cancer, head and neck squamous cell carcinoma, thyroid cancer, brain cancer, gastric cancer, head and neck cancer, head and neck squamous cell carcinoma, kidney cancer, testicular cancer, multiple myeloma, glioblastoma, glioma, and hematologic tumor such as leukemia, lymphoma (e.g., Hodgkin's lymphoma, non-Hodgkin's lymphoma, diffuse large B-cell lymphoma, acute B-cell lymphoma, follicular lymphoma).

28. A method for treating, preventing and/or diagnosing cancer, autoimmune disease, infectious disease or angiogenesis-related disease, including administering an effective amount of the trispecific antibody of any one of claims 1 to 20, or the pharmaceutical composition of claim 25 to a patient in need thereof.

29. The method of claim 28, wherein the cancer is selected from the group consisting of solid tumor such as lung cancer (e.g., small cell lung cancer, non-small cell lung cancer), breast cancer, liver cancer, bladder cancer, breast cancer, melanoma, colon cancer, rectal cancer, ovarian cancer, cervical cancer, prostate cancer, pancreatic adenocarcinoma, basal cell carcinoma, esophageal cancer, bile duct cancer, head and neck squamous cell carcinoma, thyroid cancer, brain cancer, gastric cancer, head and neck cancer, head and neck squamous cell carcinoma, kidney cancer, testicular cancer, multiple myeloma, glioblastoma, glioma, and hematologic tumor such as leukemia, lymphoma (e.g., Hodgkin's lymphoma, non-Hodgkin's lymphoma, diffuse large B-cell lymphoma, acute B-cell lymphoma, follicular lymphoma).
